(19) Europäisches Patentamt
European Patent Office
Office européen des brevets

(11) EP 2 651 403 B1

(12) EUROPEAN PATENT SPECIFICATION

(45) Date of publication and mention of the grant of the patent:
**02.12.2020 Bulletin 2020/49**

(21) Application number: **11802907.3**

(22) Date of filing: **16.12.2011**

(51) Int Cl.:
***A61K 31/00*** *(2006.01)* ***A61K 31/135*** *(2006.01)*
***A61K 31/517*** *(2006.01)* ***A61K 31/7088*** *(2006.01)*
***A61P 27/02*** *(2006.01)*

(86) International application number:
**PCT/EP2011/073121**

(87) International publication number:
**WO 2012/080497 (21.06.2012 Gazette 2012/25)**

(54) **USE OF LP-PLA2 INHIBITORS IN THE TREATMENT AND PREVENTION OF EYE DISEASES**

VERWENDUNG VON LP-PLA2-INHIBITOREN ZUR BEHANDLUNG UND VORBEUGUNG VON AUGENERKRANKUNGEN

PROCÉDÉS DE TRAITEMENT ET DE PRÉVENTION DE MALADIES OCULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2010 PCT/CN2010/002076**
**20.10.2011 PCT/CN2011/001747**

(43) Date of publication of application:
**23.10.2013 Bulletin 2013/43**

(73) Proprietor: **Glaxo Group Limited**
**Middlesex TW8 9GS (GB)**

(72) Inventors:
- **ADAMSON, Peter**
  **Stevenage, Hertfordshire SG12NY (GB)**
- **LEE, Daniel**
  **Shanghai 201203 (CN)**

(74) Representative: **Jewson, Rie**
**GlaxoSmithKline**
**Global Patents (CN925.1)**
**980 Great West Road**
**Brentford, Middlesex TW8 9GS (GB)**

(56) References cited:
**WO-A1-2008/140449**
**WO-A1-2008/141176**
**US-A1- 2008 279 846**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 651 403 B1

## Description

### FIELD OF THE INVENTION

The present invention relates generally to methods for the treatment and/or prevention of eye diseases, and more particularly to treatment and/or prevention of eye diseases using agents that inhibit the expression and/or activity of Lp-$PLA_2$ protein.

### BACKGROUND OF THE INVENTION

The process of blood vessel deterioration that occurs in diabetic retinopathy is not fully understood, however the severity of the condition is directly related to the degree and duration of hyperglycemia. Visual impairment normally occurs in the later stages of diabetic retinopathy. The development of macula edema is a direct consequence of inner blood-retinal barrier (iBRB) breakdown while in the neovascular phase of the disease (proliferative diabetic retinopathy; PDR) there is an abnormal growth of new blood vessels that give rise to sight-threatening vitreous haemorrhage and tractional retinal detachment.

Macular edema occurs as a result of increasing inner retinal ischemia and hypoxia-driven secretion of cytokines and growth factors, the best known being vascular endothelial growth factor (VEGF). Macular edema may occur as a result of Retinal Vein Occlusion (RVO), inflammation, post-surgical, traction, and the like. Macular edema, which can be a result of diabetes, is a major cause of vision loss in diabetic patients. In addition, macular edema may occur at any stage of diabetic retinopathy, even before the clinical appearance of the disease (Antonetti et al., 2006; Curtis et al., 2008).

A large number of asymptomatic patients with preclinical macular edema fail to seek treatment until some degree of vision loss has already occurred. While sight-threatening diabetic retinopathy can be treated or contained to some extent by laser photocoagulation, corticosteroids, VEGF inhibitors and vitreoretinal surgery, these interventions are mainly focused on end stage disease, have significant sight-threatening side effects and do not address the early and potentially reversible vasodegenerative pathology (Chung et al., 2008). The net effect of sustained retinal ischemia or macula edema is neurodegeneration of the neuroretinal layer leading to vision loss. Therefore, an unmet need persists to treat macula edema when less burdensome treatment options would ideally provide superior outcomes, such as visual acuity.

Breakdown of the iBRB is one of the most important pathophysiological changes in the early stages of diabetic retinopathy as well as of other ischemic or inflammatory retinal diseases, such as central retinal vein occlusion and uveitis. Vasopermeability and overt iBRB dysfunction has been observed both in patients with diabetes and streptozotocin-(STZ)-induced diabetic animal models. The exact mechanisms underlying the breakdown of iBRB are largely unclear but it is known that part of the mechanism is related to VEGF (and other cytokine) levels leading to endothelial dysfunction including loss of tight junction integrity and cell-loss. The retinal vessels of the eye reside in the neural retina, which is an extension of the central nervous system, and consequently these are CNS-type vessels which are anatomically identical to CNS vessels in the brain. Thus the inner retinal (vascular) barrier is referred to as the anterior blood-retinal barrier whilst those vessels in the brain are referred to as the blood-brain barrier. Both the anterior blood-retinal and blood-brain barriers are unique when compared to all other vascular beds in that the barrier properties of the CNS barriers are significantly enhanced. This important anatomical feature is achieved through the development of endothelial tight junctions which provides an ionically tight barrier with very low permeability. This incredibly low permeability is important to CNS homeostasis and perturbations of this permeability can lead to serious events such as CNS edema and diabetic macular edema (DME).

Oxidative stress has generated much interest primarily due to its accepted role as a major contributor to the etiology of normal, senescence and chronic pathologies with serious public health implications such as diabetes and atherosclerosis. It follows; therefore, that increased oxidative stress appears to be an important contributor for diabetic cardiovascular disease (Pennathur et al., 2007) and likely other complications of diabetic vascular disease such as diabetic retinopathy, diabetic macular edema and diabetic nephropathy. Indeed, individuals with both diabetes mellitus and hypercholesterolemia have an increased risk of macrovascular atherosclerotic complications (Moreno et al., 2000). Activation of nicotinamide adenine dinucleotide phosphate (NADPH) oxidase has been implicated as the major source of reactive oxygen species (ROS) generation in the vasculature in response to high glucose and advanced glycation end-products (AGEs) (Dave et al., 2007). AGEs are strongly implicated in diabetic retinopathy and in fact AGEs accumulate within the various organs that are damaged in diabetes, with the accumulation rate of these AGEs accelerated by hyperglycemia. AGEs accumulate in most sites of diabetes complications, including the kidney, retina, and atherosclerotic plaques (Hammes et al., 1999; Bucala et al., 1995; Makita et al., 1994). AGEs have been localized to retinal blood vessels in patients with type-2 diabetes and found to correlate with the degree of retinopathy (Murata et al., 1997; Stitt 2001). When non-diabetic animals are infused with preformed AGE albumin, the adducts accumulate around and within the pericytes, co-localize with AGE-receptors, induce basement membrane thickening, and contribute to the

breakdown of the inner blood-retinal barrier (Stitt et al., 1997; Stitt et al., 2000). Furthermore, retinal vascular endothelial cells exposed to AGEs show abnormal endothelial nitric oxide synthase expression (Chakravarthy et al., 1998), which may account for some of the vasoregulatory abnormalities seen in the retinal microcirculation in diabetes. *In vitro* studies have also demonstrated the up-regulation of VEGF in retinal cells after exposure toAGEs (Lu et al., 1998), potentially promoting retinal neovascularization and increasing permeability to proteins across the retinal barrier.

AGE formation on proteins, lipids, and DNA can have serious consequences for macromolecular function (Paget et al., 1998; Giardino ert al; 1994; Addel-Wahab et al., 1996) and are constantly forming under physiological conditions. Complex receptor systems have evolved to remove senescent, glycation-modified molecules and/or degrade existing AGE-cross-links from tissues thereby limiting their deleterious effects. Such receptors play a critical role in AGE-related biology and the pathology associated with diabetes (Vlassara et al., 2001; Schmidt et al., 2000, Sano et al., 1999). Several AGE-binding molecules have been described and it is thought that many of the adverse effects caused by advanced glycation products are mediated via AGE-receptors. Receptor for Advanced Glycation Endproducts (RAGE) (Schmitd et al., 1994) is the best characterised of these receptors. Nevertheless it remains controversial if some or all AGE-receptors serve to promote or limit AGE-mediated cell and tissue dysfunction. The elucidation of AGE-receptor modulatory roles and signal transduction pathways are areas of intensive investigation and recent evidence suggests that AGE-receptor binding can initiate important signalling pathways involving activation of protein kinase C (Mene et al., 1999; Scivittaro et al., 2000), tyrosine phosphorylation of Janus kinase (JAK)/signal transducers and activators of transcription (STAT) (Huang et al., 1999) recruitment of phosphotidylinositol 3' kinase to Ras (Deora et al., 1998) transcriptional activation (Lander et al., 1997), and induction of oxidative stress cascades involving NFκB and AP-1 (Bierhaus et al., 2007).

A consequence of increased oxidative stress is the oxidation of lipids, including phospholipids contained within low density lipoprotein (LDL). Lipoprotein-Associated Phospholipase $A_2$ (Lp-PLA$_2$), also previously known in the art as Platelet Activating Factor Acetyl Hydrolase (PAF acetyl hydrolase) is a member of the super family of phospholipase $A_2$ enzymes that are involved in hydrolysis of lipoprotein lipids or phospholipids. It is secreted by several cells that play a major role in the systemic inflammatory response to injury, including macrophages, monocytes, lymphocytes, T lymphocytes, and mast cells and is found predominately associated with LDL in human circulation (Wilensky et al., 2009). The enzyme has also been linked to neurodegenerative disease, and inhibitors were proposed to reduce signs and symptoms of blood brain barrier permeability (WO 2008/140449 A1).

Lp-PLA2 uniquely cleaves oxidized, but not unmodified phospholipids, and has been shown to generate significant quantities of non-esterified free fatty acids (NEFAs) and lysophosphatidylcholine (lysoPC) both of which are pro-inflammatory lipids. LysoPC, for example, has been implicated in leukocyte activation, induction of apoptosis and mediation of endothelial dysfunction (Wilensky et al., 2009; Lavi et al., 2007). Interestingly, when plasma samples were collected simultaneously from the left main coronary artery and coronary sinus it was demonstrated the local net production of lysoPC was significantly correlated with coronary endothelial dysfunction. This clinical observation was consistent with *in vitro* studies showing that lysoPC causes down-regulation of endothelial nitric oxide synthase expression, activation of endothelial NADPH oxidase and inhibition of endothelial cell migration. Thus, Lp-PLA2 could contribute to the tissue damage associated with diabetes by producing lysoPC that could augment a continuous cycle of vascular inflammation and increased ROS production.

Given that Lp-PLA2 circulates with LDL (Wilensky et al., 2009) and specifically impacts the generation of pro-inflammatory lipids following its enzymatic activity on oxidised lipoproteins lipids, it is notable that treatment of db/db mice with lovastatin (a HMGCoA-reductase inhibitor, statin) which is demonstrated to lower LDL in humans, but also acts as an anti-inflammatory, is also effective in reducing retinal vascular leakage and aspects of retinal inflammation (Li et al., 2009). Lovastatin treatment significantly lowered serum cholesterol levels and as such may have contributed to the beneficial effects of lovastatin in the retina. The db/db mouse is a well defined genetic model of type2 diabetes which is also hyperlipidemic. A similar finding is also noted in uveitis-induced blood-retinal barrier breakdown in B10RIII mice (Gegg et al., 2005).

The concept that localized inflammatory processes play a role in the development of diabetic retinopathy is becoming established, and evidence that supports the hypothesis is accumulating rapidly including the findings that diabetic eye disease is associated with numerous inflammatory mediators which result in leukostasis (adherence of leukocytes to the luminal surface of retinal vessels) and increased vascular permeability. This new hypothesis offers new insight into the pathogenesis of diabetic retinopathy, and offers novel targets to inhibit the ocular disease.

## SUMMARY OF THE INVENTION

The present invention relates to methods for treatment and/or prevention of eye diseases and disorders by inhibition of Lp-PLA2, for example inhibition of expression and/or activity of Lp-PLA2 protein. In particular embodiments, eye diseases amenable to treatment and/or prevention by the methods of the present invention are associated with the breakdown of the inner blood-retinal barrier (iBRB). According to the present invention, the eye disease or disorder is

macular edema of any cause, e.g., due to RVO, inflammation, post-surgical, traction, and the like; age-related macular degeneration (AMD); uveitis; diabetic eye diseases and disorders; diabetic retinopathy, and the like.

In further embodiments, systemic inflammatory diseases such as, juvenile rheumatoid arthritis, inflammatory bowel disease, Kawasaki disease, multiple sclerosis, sarcoidosis, polyarteritis, psoriatic arthritis, reactive arthritis, systemic lupus erythematosus, Vogt-Koyanagi-Harada syndrome, Lyme disease, Bechet's disease, ankylosing sponsylitis, chronic granulomatous disease, enthesitis, can be the underlying cause of uveitis affecting the retina, and which can result in macula edema. The present invention relates to methods for treatment and/or prevention of uveitis by inhibition of Lp-PLA2, for example inhibition of expression and/or activity of Lp-PLA2 protein.

The present invention relates to methods for treatment and/or prevention of eye diseases and disorders by inhibition of Lp-PLA2, for example inhibition of expression and/or activity of Lp-PLA2 protein. In particular embodiments, eye diseases amenable to treatment and/or prevention by the methods of the present invention include, but are not limited to, central retinal vein occlusion, branched retinal vein occlusion, Irvine-Gass syndrome (post cataract and post-surgical), retinitis pigmentosa, pars planitis, birdshot retinochoroidopathy, epiretinal membrane, choroidal tumors, cystic macular edema, parafoveal telengiectasis, tractional maculopathies, vitreomacular traction syndromes, retinal detachment, neuroretinitis, idiopathic macular edema, and the like.

In one embodiment, the methods as disclosed herein comprise administering to a subject in need of treatment and/or prevention of an eye disease, a pharmaceutical composition comprising an agent which inhibits Lp-PLA2, for example an agent which inhibits the expression of Lp-$PLA_2$ and/or the activity of Lp-$PLA_2$ protein. It is not intended that the present invention to be limited to any particular stage of the disease (e.g., early or advanced).

In some embodiments, as disclosed herein, methods to prevent iBRB leakage are provided by inhibition of Lp-$PLA_2$, for example inhibition of expression of Lp-$PLA_2$ and/or inhibition of protein activity of Lp-$PLA_2$. Accordingly, some embodiments provide methods to inhibit Lp-$PLA_2$ by blocking enzyme activity and some embodiments provide methods to inhibit Lp-$PLA_2$ by reducing and/or down-regulating the expression of Lp-$PLA_2$ RNA. In some embodiments, prevention and/or reduction of iBRB leakage or iBRB permeability leads to prevention and/or reduction of symptoms associated with diabetic eye diseases.

In a further embodiment, the subject administered an agent that inhibits the activity or expression of the Lp-$PLA_2$ protein is a human.

In another embodiment, the present invention provides methods of treating and/or preventing a subject with or at risk of macular edema comprising administering to the subject a pharmaceutical composition comprising an agent which inhibits the activity and/or expression of Lp-$PLA_2$ protein, wherein inhibition of the Lp-$PLA_2$ protein reduces or stops a symptom of macular edema. In some embodiments, the macular edema is associated with diabetic eye disease, but not diabetic retinopathy. In another embodiment, the macular edema is associated with diabetic retinopathy. In other embodiments, the macular edema is associated with uveitis. In yet another embodiment, macular edema may be due to any other cause, e.g. due to RVO, inflammation, post-surgical, traction, and the like.

In another embodiment, the present invention provides methods of treating and/or preventing a disease or disorder associated with the breakdown of the inner blood-retinal barrier in a subject in need thereof, comprising administering to the subject a pharmaceutical composition comprising an agent which inhibits the expression and/or activity of the Lp-$PLA_2$ protein.

In some embodiments, the agent that inhibits Lp-$PLA_2$ can inhibit the expression of the Lp-$PLA_2$, for example inhibit the translation of Lp-$PLA_2$ RNA to produce the Lp-$PLA_2$ protein. In alternative embodiments, the agent that inhibits Lp-$PLA_2$ can inhibit Lp-$PLA_2$ protein activity. Any agent is encompassed for use in the methods as disclosed herein.

The agent that inhibits the protein activity of Lp-$PLA_2$ is a small molecule, as defined below. According to the invention, a small molecule inhibitor of Lp-$PLA_2$ is, N-[2-(diethylamino)ethyl]-2-[[(4-fluorophenyl)methyl]thio]-4,5,6,7-tetrahydro-4-oxo-N-[[4'-(trifluoromethyl)[1,1'-biphenyl]-4-yl]methyl]-1H-cyclopentapyrimidine-1-acetamide (aka Darapladib or Lp-PLA2 inhibitor '848) or a salt thereof. See U.S. 6,649,619.

According to the invention, a further small molecule inhibitor of Lp-$PLA_2$ is, 2-[[(2,3-difluorophenyl)methyl]thio]-N-[1-(2-methoxyethyl)-4-piperidinyl]-4-oxo-N-[[4'-(trifluoromethyl)[1,1'-biphenyl]-4-yl]methyl]-1(4H)-quinolineacetamide (alternatively named N-(1-(2-Methoxyethyl)piperidin-4-yl)-2-[2-(2,3-difluorobenzylthio)-4-oxo-4H-quinolin-1-yl]-N-(4'-trifluoromethylbiphenyl-4-ylmethyl)acetamide) (aka Rilapladib or Lp-PLA2 inhibitor '032) or a salt thereof. See U.S. 7,235,566.

A further small molecule inhibitor of Lp-$PLA_2$ is according to the invention N-[2-(dimethylamino)ethyl]-2-[[(4-fluorophenyl)methyl]thio]-5-(1-methyl-1H-pyrazol-4-yl)methyl]-4-oxo-N-[[4'-(trifluoromethyl)[1,1'-biphenyl]-4-yl]methyl]-1(4H)-pyrimidineacetamide (aka Lp-PLA2 inhibitor '495) or a salt thereof. See U.S. 6,953,803.

A last small molecule inhibitor of Lp-$PLA_2$ is, according to the invention, N-[2-(diethylamino)ethyl]-2-{2-[2-(2,3-difluorophenyl)ethyl]-4-oxo-4,5,6,7-tetrahydro-1H-cyclopenta[d]pyrimidin-1-yl}-N-{[4'-(trifluoromethyl)-4-biphenylyl]methyl}acetamide bitartrate (alternatively, N-(1-ethylpiperidin-4-yl)-2-(2-(2-(2,3-difluorophenyl)-ethyl)-4-oxo-4H-quninazolin-1-yl)-N-(4'-chloro-biphenyl-4-ylmethyl)-acetamide bitartrate) (aka Lp-PLA2 inhibitor '859) or a salt thereof.

In some embodiments where a subject is administered a pharmaceutical composition comprising an inhibitor

of Lp-PLA$_2$, the methods can further comprise administering to the subject additional therapeutic agents, for example but not limited to therapeutic agents used in the treatment of eye diseases, including macular edema of any cause, e.g., due to RVO, inflammation, post-surgical, traction, and the like; AMD; uveitis; diabetic eye diseases and disorders; diabetic retinopathy, and the like. It will be understood that the administration of therapeutic agents for treating ocular diseases may involve the application of certain procedures, for example, but not limited to, retinal focal laser photocoagulation, pan-retinal photocoagulation, intravitreal administered steroids, such as triamcinolone, intravitreal steroid implants containing fluocinolone acetonide, and intravitreal administered anti-VEGF therapeutics such as Lucentis®, Avastin® and Aflibercept®.

In some embodiments, the methods as disclosed herein for the treatment and/or prevention of macular edema, diabetic eye diseases, diabetic retinopathy, and other eye diseases or disorders as described herein, are applicable to subjects, for example mammalian subjects. In some embodiments, the subject is a human.

Further, the object of the present invention is defined as in the appended claims.

## BRIEF DESCRIPTION OF FIGURES

**Figure 1** shows evidence that diabetic/hypercholesterolemia (DMHC) causes Blood-Brain-Barrier (BBB) breakdown, leading to plasma influx into the brain tissue and binding of IgG to neurons. IgG-immunopositive microvascular leaks are commonly observed throughout the different brain regions of DMHC pigs. The number of leaks observed and the extent or magnitude of the plasma influx into the brain parenchyma varied greatly, supporting the sporadic or unpredictable nature of these leaks in terms of where they arise. Red outline encloses an isolated small arteriole with a small perivascular plasma leak cloud. Neurons (dark brown spots) are intensely IgG-positive in regions of leak.

**Figure 2B** shows that Darapladib reduces the density of leaks from arterioles in the cortex. **(Upper Panel):** The density of arteriolar leaks in the cortex (number of leaking arterioles per sq mm) was different among DMHC, Darapladib-treated DMHC (10mg/kg/day for 24 weeks) and untreated control pig. Arterioles are the main sites of detectable vascular leaks. The arteriolar leak density in the drug-treated group was reduced somewhat compared to the other groups.

**Figure 2A** shows that Darapladib reduces the amount of material that leaks from arterioles into the brain. Similarly, the extent of arteriolar leaks (i.e., the amount of material that leaks from arterioles) was also somewhat reduced in the cerebral cortex of DMHC pig brains treated with Darapladib (10mg/kg/day for 24 weeks).

**Figure 3** shows Intraneuronal Abeta42 in the pig cerebral cortex. A consistent finding in these studies is that the dominant neuronal cell type, the pyramidal neuron, is the only neuronal cell type to contain Abeta42 in the pig brain.

**Figure 4A** shows relative density of Abeta42-containing neurons entire brain. Darapladib (10mg/kg/day for 24 weeks) reduced the density of Abeta42-positive neurons in DMHC pig cerebral cortex across the entire pig brain to levels comparable to controls.

**Figure 4B** shows density of neurons containing Abeta42 entire Brain comparison of Cerebral Cortex Layers 2-3 (L2) with Layers 4-6 (L6). **(Lower Panel):** Although the density of Abeta42-positive neurons was slightly greater in cortical layers 2-3 of the DMHC group, this is to be expected in view of the generally smaller pyramidal neuron size in this layer.

**Figure 5A** shows the total amount of Abeta42 (Abeta42 Load) entire brain. Darapladib (10mg/kg/day for 24 weeks) reduced the total amount of Abeta42 in the cerebral cortex in DMHC pigs.

**Figure 5B** shows amount of Abeta42 per Abeta42-containing neuron entire brain. This reduction was apparently not a result of reducing the amount of Abeta42 per neuron but by reducing the number of neurons that load Abeta42 (as shown above in Figure 4 also).

**Figure 6** shows Lp-PLA2 levels in Alzheimer model rabbits over 10 weeks. Lp-PLA2 in 40 rabbits treated with cholesterol supplemented diet and Copper Sulphate supplanted drinking water in 5 groups of animals receiving daily s,c. Injections. The two groups receiving Lp-PLA2 inhibitor ’859 had significantly lower levels of Lp-PLA2 activity. Data is expressed as mean +/- SEM.

**Figure 7** shows the accumulation of sodium fluorescent in rabbits treated with vehicle of Lp-PLA2 inhibitor compounds. Identical data to that shown in Figure 5, NaF accumulation in normal-fed animals and animals fed a high cholesterol, $CuSO_4$ supplemented diet with or without treatment with Lp-PLA2 inhibitor ’859 (10mg/kg/day). Data is normalised with the BBB permeability of normal fed animals set at zero. Individual data points for individual rabbits are shown. Lines designate group mean values.

**Figure 8** shows induction of hyperglycaemia in Sprague-Dawley (SD) rats following treatment with streptozotocin (STZ). Data are expressed as mean +/- SEM.

**Figure 9** shows increase in plasma Lp-PLA2 activity in SD rats following induction of hyperglycemia with STZ and suppression with after dosing of Lp-PLA2 inhibitor ’495 (10mg/kg). Data is mean +/- SEM.

**Figure 10A-B** shows the extravasation of Evans-blue-albumin from plasma to retina in diabetic rats treated with vehicle or an Lp-PLA2 inhibitor compound. Effect of hyperglycaemia for 31 days and treatment of animals with Lp-PLA2 inhibitor ’495 (10mg/kg/day) for 28 days on Evans Blue dye extravasation into retinal parenchyma in SD rats. (A) shows values for individual animals with lines designating means. (B) shows mean values +/- SEM.

**Figure 11A-B** shows the incidence of sub-retinal fluid accumulation in diabetic rats as assessed by optical coherence tomography (OCT). **(A)** Incidence of retinal fluid accumulation (percent of all animals tested) as assessed by OCT imaging in normo-glycaemic, hyperglycaemic and hyperglycaemic SD rats treated with Lp-PLA2 inhibitor ’495 (10mg/kg/day) at day 14 (study 1) and day 17 (study II). **(B)** Initial study showing retinal fluid accumulation at day 17 in STZ-treated SD rats.

**Figure 12A-C** shows reduction in neuroretinal thickness as assessed by optical coherence tomography (OCT) in diabetic rats treated with vehicle or Lp-PLA2 inhibitor compounds. (A) Shows the thickness of the neuroretinal layer as assessed by OCT over time in animals induced for diabetes with STZ (50mg/kg/d i.p. for 3 days) and subsequently treated with vehicle from day 0, Lp-PLA2 inhibitor ’859 (10mg/kg/d i.p from day 4) or with Lp-PLA2 inhibitor ’495 (10mg/kg/d i.p from day 8). Shows the thickness of the neuroretinal layer at (A) 10 days after induction of diabetes and (B) 18 days after induction of diabetes (50mg/kg/d i.p. for 3 days) and subsequently treated with vehicle from day 0, Lp-PLA2 inhibitor ’859 (10mg/kg/d i.p from day 4) or with Lp-PLA2 inhibitor ’495 (10mg/kg/d i.p from day 8). Animals which were not treated with STZ are used to set the baseline at 0. * $p<0.05$ compared to animals treated with vehicle only. Data are expressed at mean +/- SEM.

**Figure 13A-B** shows reduction in neuroretinal thickness as assessed by optical coherence tomography in diabetic rats treated with vehicle or an Lp-PLA2 inhibitor compound. (A) Shows the thickness of the neuroretinal layer as assessed by OCT over time in untreated animals or animals induced for diabetes with STZ (50mg/kg/d i.p. for 3 days.) - treated animals were subsequently treated with Lp-PLA2 inhibitor ’495 (10mg/kg/d) or vehicle i.p. from day 4. ***$p<0.001$ STZ-treated and vehicle treated animals vs. STZ-treated and Lp-PLA2 inhibitor ’495 animals. ### $p<0.001$ non STZ-treated animals vs. STZ-treated animals by two-way repeated ANOVA. Data are expressed at mean +/- SEM (B) shows comparison of retinal thickness in vehicle-treated versus Lp-PLA2 inhibitor ’495 (10mg/kg/d) treated diabetic animals at 13 and 28 days. *** $P<0.001$ ; ** $P<0.01$ STZ vs. STZ+ Lp-PLA2 inhibitor ’495 by Tukey’s Post-hoc test

**Figure 14A-B** shows transendothelial electrical resistance and transport of Lucifer Yellow through an in vitro blood-brain (retinal) barrier model following addition of lysoPC. (A) Shows transport of Lucifer Yellow tracer across rat brain microvascular endothelial cell primary cultures, cultured on the apical side of transwell inserts in the presence of rat brain astrocytes cultured (in the bottom well) in response to the addition of lyso PC to the endothelial monolayer. *$p<0.001$ when compared to vehicle treated (B) Shows transendothelial electrical resistance both before and after addition of lysoPC to endothelial cell monolayers. *$p<0.001$ when compared to TEER before addition of lysoPC. Data are expressed as mean +/- SEM. Significant differences are determined by t-tests).

**Figure 15** shows the effect of hyperglycaemia for 31 days and treatment of animals with Lp-PLA2 inhibitor ’495 (10mg/kg i.p. QD) for 14 days on Evans Blue dye extravasation into retinal parenchyma in Brown Norway rats. Panels show values for individual animals with lines designating means and values +/- SEM. Non-diabetic (NDB CON); DB + PLACEBO Diabetic control treated with vehicle; DB + DRUG Diabetic control plus 10mg/kg Lp-PLA2 inhibitor ’495 (DRUG) (10mg/kg i.p. QD). In the 4 wk study diabetic vehicle treated animals vs. diabetic Lp-PLA2 inhibitor ’495-treated animals were statistically different $p<0.04$, t-test.

**Figure 16** shows retinal histology Images demonstrating a treatment effect of 20mg/kg Lp-PLA2 inhibitor ’495 QD i.p. on albumin leakage from retinal vessels in hyperglycaemic Brown Norway rats (2 weeks data). Cryosections: retinal blood vessels were identified by IB4 staining (red) and rat albumin (green). In diabetic retinae there is strong co-localization of both labels showing albumin is leaking from the retinal vasculature whereas in drug-treated animals albumin is contained intravascularly and co-localised with retinal vessels.

**Figure 17** shows retinal histology Images demonstrating a treatment effect of 10mg/kg Lp-PLA2 inhibitor ’495 QD i.p. on albumin leakage from retinal vessels in hyperglycaemic Brown Norway rats (4 weeks). Cryosections: retinal blood vessels were identified by IB4 staining (red) and rat albumin (green). In non-diabetic animals albumin is co-localised to retinal vessels whereas in diabetic retinae there is poor co-localization of labels showing albumin is leaking from the retinal vasculature Drug-treated animals are similar to non-diabetic controls in which albumin is contained intravascularly and co-localised with retinal vessels.

## DETAILED DESCRIPTION OF THE INVENTION

The inventors have discovered that Lp-$PLA_2$ inhibitors can be used in the treatment and/or prevention of ocular diseases, namely macular edema of any cause, e.g., due to RVO, inflammation, post-surgical, traction, and the like; AMD; uveitis; diabetic eye diseases and disorders; diabetic retinopathy, and the like, and disorders associated with breakdown of the inner blood retinal barrier.

Definitions

For convenience, certain terms employed in the entire application (including the specification, examples, and appended claims) are collected here. Unless defined otherwise, all technical and scientific terms used herein have the same meaning as commonly understood by one of ordinary skill in the art to which this invention belongs.

The term "disease" or "disorder" is used interchangeably herein, and refers to any alteration in state of the body or of some of the organs, interrupting or disturbing the performance of the functions and/or causing symptoms such as discomfort, dysfunction, distress, or even death to the person afflicted or those in contact with a person. A disease or disorder can also relate to a distemper, ailing, ailment, malady, disorder, sickness, illness, complaint or affectation.

The terms "blood-brain barrier" or "BBB" are used interchangeably herein, and are used to refer to the permeability barrier that exists in blood vessels as they travel through the brain tissue that severely restricts and closely regulates what is exchanged between the blood and the brain tissue. The blood brain barrier components include the endothelial cells that form the innermost lining of all blood vessels, the tight junctions between adjacent endothelial cells that are the structural correlate of the BBB, the basement membrane of endothelial cells and the expanded foot processes of nearby astrocytes which cover nearly all of the exposed outer surface of the blood vessel. The BBB prevents most substances in the blood from entering brain tissue, including most large molecules such as Ig, antibodies, complement, albumin and drugs and small molecules.

The terms "inner blood-retinal barrier" or "iBRB" are used interchangeably herein, and are used to refer to the permeability barrier that exists in blood vessels as they travel through the retinal tissue that severely restricts and closely regulates what is exchanged between the blood and the retinal tissue. The blood retinal barrier components include the endothelial cells that form the innermost lining of all blood vessels, the tight junctions between adjacent endothelial cells that are the structural correlate of the iBRB, the basement membrane of endothelial cells and the expanded foot processes of nearby astrocytic cells and pericytes, including glial cells, which cover nearly all of the exposed outer surface of the blood vessel. The iBRB prevents most substances in the blood from entering retinal tissue, including most large molecules such as Ig, antibodies, complement, albumin and drugs and small molecules.

The term "abnormal BBB" is used to refer to a dysfunctional BBB, for example, where the BBB does not allow transit of molecules that normally transit a functional BBB, for example nutrients and sugars such as glucose. An abnormal BBB can also refer to when the BBB is permeable to molecules that a normally functioning BBB would typically exclude, which is typically referred to "BBB permeability" herein.

The term "abnormal inner BRB" is used to refer to a dysfunctional iBRB, for example, where the iBRB does not allow transit of molecules that normally transit a functional iBRB, for example nutrients and sugars such as glucose. An abnormal iBRB can also refer to when the iBRB is permeable to molecules that a normally functioning iBRB would typically exclude, which is typically referred to "iBRB permeability" herein.

The terms "BBB permeability" or "permeable BBB" are commonly referred to by persons in the art as "leaky BBB". The terms are used interchangeably herein to refer to impaired BBB integrity and increased vascular permeability. For example, a permeable BBB allows transit of molecules through the BBB that an intact BBB would normally exclude from the brain tissue, for example, Ig molecules, complement proteins, serum albumin and numerous other proteins. An assay to determine the presence of a permeable BBB can be, for example, to assess the presence of extravascular Ig in the brain tissue which is normally restricted to the lumen of blood vessels when the BBB is functioning normally (i.e., when the BBB is not permeable).

The terms "iBRB permeability" or "permeable iBRB" are commonly referred to by persons in the art as "leaky iBRB". The terms are used interchangeably herein to refer to impaired iBRB integrity and increased vascular permeability. For example, a permeable iBRB allows transit of molecules through the iBRB that an intact iBRB would normally exclude from the retinal tissue, for example, Ig molecules, complement proteins, serum albumin and numerous other proteins. An assay to determine the presence of a permeable iBRB can be, for example, to assess the presence of extravascular Ig in the retinal tissue which is normally restricted to the lumen of blood vessels when the iBRB is functioning normally (i.e., when the BRB is not permeable).

The term "agent" or "compound" as defined in the claims, refers to the small molecules as described above.

The term "inhibiting" as used herein means that the expression or activity of Lp-$PLA_2$ protein or variants or homologues thereof is reduced to an extent, and/or for a time, sufficient to produce the desired effect, for example, wherein inhibition of the Lp-$PLA_2$ protein reduces or stops a symptom of macular edema, uveitis, diabetic retinopathy, etc. The reduction in activity can be due to affecting one or more characteristics of Lp-$PLA_2$ including decreasing its catalytic activity or by inhibiting a co-factor of Lp-$PLA_2$ or by binding to Lp-PLA2 with a degree of activity that is such that the outcome is that of treating or preventing macular edema of any cause, e.g., due to RVO, inflammation, post-surgical, traction, and the like; age-related macular degeneration (AMD); uveitis; diabetic eye diseases and disorders; diabetic retinopathy, and the like. In particular, inhibition of Lp-$PLA_2$ can be determined using an assay for Lp-$PLA_2$ inhibition, for example, but not limited to using the bioassay for Lp-$PLA_2$ protein as disclosed herein.

As used herein, the term "Lp-$PLA_2$" refers to the protein target inhibited by the methods as disclosed herein.

Lp-$PLA_2$ is used interchangeably with lipoprotein associated phospholipase $A_2$, also previously known in the art as Platelet Activating Factor Acetyl Hydrolase (PAF acetyl hydrolase). Human Lp-$PLA_2$ is encoded by nucleic acid corresponding to accession No: U20157 (SEQ ID NO:1) or Ref Seq ID: NM_005084 (SEQ ID NO:2) or and the human Lp-$PLA_2$ corresponds to protein sequence corresponding to accession No: NP_005075 (SEQ ID NO:3), which are disclosed in U.S. Patent 5,981,252, which is specifically incorporated herein in its entirety by reference.

The terms "patient", "subject" and "individual" are used interchangeably herein, and refer to an animal, particularly a human, to whom treatment including prophylaxic treatment is provided. The term "subject" as used herein refers to human and non-human animals. The term "non-human animals" and "non-human mammals" are used interchangeably herein includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. In one embodiment, the subject is human. In another embodiment, the subject is an experimental animal or animal substitute as a disease model.

As used herein, the term "treating" includes reducing, alleviating or preventing at least one adverse effect or symptom of a condition, disease or disorder associated with the eye diseases and disorders described herein. Methods for measuring positive outcomes of treatment include, but are not limited to reduction or maintenance of sub-retinal edema, measured by OCT, reduction in the loss or maintenance of vision, or the gain of vision as assessed by best corrected visual acuity.

The term "effective amount" as used herein refers to the amount of therapeutic agent of pharmaceutical composition to reduce, stop, alleviate or prevent at least one symptom of the eye diseases or disorders disclosed herein. For example, an effective amount using the methods as disclosed herein would be considered as the amount sufficient to reduce or prevent a symptom of the disease or disorder, for example a complete or partial resolution and/or maintenance of macula edema as measured by OCT or an increase and/or maintenance in best corrected visual acuity greater than 5 letters (as assessed by EDTRS eye chart). An effective amount for treating diabetic macular edema would include an amount sufficient to improve vision, usually achieved by reducing the amount of macular edema caused by leakage from intraretinal capillaries. The amount of edema can be estimated by the amount of retinal thickening detected by a noninvasive technique such as OCT and/or by the leakage detected on fluorescein angiography. An effective amount as used herein would also include an amount sufficient to prevent or delay the development of macula edema and associated vision loss. An effective amount as used herein would also include an amount sufficient to prevent or delay the development of a symptom of the disease, alter the course of a symptom of the disease (for example but not limited to, slow the progression of a symptom of the disease), or reverse a symptom of the disease.

As used herein, the terms "administering," and "introducing" are used interchangeably and refer to the placement of the agents that inhibit Lp-$PLA_2$ as disclosed herein into a subject by a method or route which results in at least partial localization of the agents at a desired site. The compounds of the present invention can be administered by any appropriate route which results in an effective treatment in the subject.

The articles "a" and "an" are used herein to refer to one or to more than one (i.e., to at least one) of the grammatical object of the article. By way of example, "an element" means one element or more than one element.

## Lp-$PLA_2$: General Information

Lp-$PLA_2$ is also referred to in the art as aliases Lp-$PLA_2$, LDL-$PLA_2$, lipoprotein associated phospholipase $A_2$ PLA2G7, phospholipase A2 (group VII), or Platelet Activating Factor Acetyl Hydrolase (PAF acetyl hydrolase or PAFAH). Human Lp-$PLA_2$ is encoded by nucleic acid corresponding to GenBank Accession No: U20157 (SEQ ID NO:1) or Ref Seq ID: NM_005084 (SEQ ID NO:2) and the human Lp-$PLA_2$ corresponds to protein sequence corresponding to GenBank Accession No: NP_005075 (SEQ ID NO:3), which are disclosed in U.S. Patent 5,981 ,252.

Phospholipase $A_2$ enzyme Lipoprotein Associated Phospholipase $A_2$ (Lp-$PLA_2$), the sequence, isolation and purification thereof, isolated nucleic acids encoding the enzyme, and recombinant host cells transformed with DNA encoding the enzyme are disclosed in WO 95/00649 (SmithKline Beecham plc). A subsequent publication from the same group further describes this enzyme (Tew D et al., Arterioscler Thromb Vas Biol 1996:16; 591-9) wherein it is referred to as LDL-$PLA_2$ and later patent application (WO 95/09921, Icos Corporation) and a related publication in Nature (Tjoelker et al., vol. 374, 6 April 1995, 549) describe the enzyme PAFAH which has essentially the same sequence as Lp-$PLA_2$.

It has been shown that Lp-$PLA_2$ is responsible for the conversion of phosphatidylcholine to lysophosphatidylcholine, during the conversion of low density lipoprotein (LDL) to its oxidized form. The enzyme is known to hydrolyze the sn-2 ester of the oxidized phosphatidylcholine to give lysophosphatidylcholine and an oxidatively modified fatty acid. Both products of Lp-$PLA_2$ action are biologically active with lysophosphatidylcholine, in particular having several pro-atherogenic activities ascribed to it including monocyte chemotaxis and induction of endothelial dysfunction, both of which facilitate monocyte-derived macrophage accumulation within the artery wall.

## Agents that inhibit Lp-$PLA_2$

In some embodiments, the present invention relates to the inhibition of Lp-$PLA_2$. In some embodiments, inhibition is inhibition of nucleic acid transcripts encoding Lp-$PLA_2$, for example inhibition of messenger RNA (mRNA). In alternative embodiments, inhibition of Lp-$PLA_2$ is inhibition of the expression and/or inhibition of activity of the gene product of Lp-$PLA_2$, for example the polypeptide or protein of Lp-$PLA_2$, or isoforms thereof. As used herein, the term "gene product" refers to RNA transcribed from a gene, or a polypeptide encoded by a gene or translated from RNA.
Inhibition of Lp-$PLA_2$ is by an agent or compound. These agents/compounds are as herein defined and according to the claims.
Other agents inhibiting Lp-$PLA_2$ have been described n WO 2008/140449; corresponding to USSN 2008/0279846.
It is known that nhibitors of Lp-$PLA_2$ can be a chemicals, small molecule, large molecule or entity or moiety, including without limitation synthetic and naturally-occurring non-proteinaceous entities.

## Small molecules

Agents or compounds according to the present invention that inhibit Lp-$PLA_2$ are small molecules as described herein. Irreversible or reversible inhibitors of Lp-$PLA_2$ are known.
Irreversible inhibitors of Lp-$PLA_2$ are disclosed in patent applications WO 96/13484, WO96/19451, WO 97/02242, WO97/12963, WO97/21675, WO97/21676, WO 97/41098, and WO97/41099 (SmithKline Beecham plc) and disclose *inter alia* various series of 4-thionyl/sulfinyl/sulfonyl azetidinone compounds which are inhibitors of the enzyme Lp-$PLA_2$. These are irreversible, acylating inhibitors (Tew et al., Biochemistry, 37, 10087, 1998).
Lp-$PLA_2$ inhibitors effective in humans are commonly known by persons of ordinary skill and include those undergoing evaluation, for example undergoing pre-clinical and clinical assessment including Phase II clinical trials. A number of applications have been filed and published by SmithKline Beecham and its successor GlaxoSmithKline. A list of relevant published applications assigned to same is: WO99/24420, WO00/10980, WO00/66566, WO00/66567, WO00/68208, WO01/60805, WO02/30904, WO02/30911, WO03/015786, WO03/016287, WO03/041712, WO03/042179, WO03/042206, WO03/042218, WO03/086400, WO03/087088, WO05/003118, WO05/021002, WO08/048866, WO08/140449, WO08/141176, WO08/048867, US 2008/0280829, US 2008/0103156, US 2008/0090851, US 2008/0090852 and Attorney Docket Number PC64333 corresponding to PCT/CN2010/0771 54.
The compound N-[2-(diethylamino)ethyl]-2-[[(4-fluorophenyl)methyl]thio]-4,5,6,7-tetrahydro-4-oxo-N-[[4'-(trifluoromethyl)[1,1'-biphenyl]-4-yl]methyl]-1H-cyclopentapyrimidine-1-acetamide (also used herein interchangeably with the term Darapladib and the alternate nomenclature of 1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one; or Lp-PLA2 inhibitor '848) is a particularly effective Lp-$PLA_2$ inhibitor and is according to this invention.
The compound 2-[[(2,3-difluorophenyl)methyl]thio]-N-[1-(2-methoxyethyl)-4-piperidinyl]-4-oxo-N-[[4'-(trifluoromethyl)[1,1'-biphenyl]-4-yl]methyl]-1(4h)-quinolineacetamide (also used herein interchangeably with the term Rilapladib; or Lp-PLA2 inhibitor '032 and the alternate nomenclature of *N*-(1-(2-Methoxyethyl)piperidin-4-yl)-2-[2-(2,3-difluorobenzylthio)-4-oxo-4H-quinolin-1-yl]-*N*-(4'-trifluoromethylbiphenyl-4-ylmethyl)acetamide) is a particularly effective Lp-$PLA_2$ inhibitor and is according to this invention.
Other Lp-$PLA_2$ inhibitors are described in published patent applications, for example WO2006063791-A1, WO2006063811-A1, WO2006063812-A1, WO2006063813-A1, all in the name of Bayer Healthcare; and US2006106017-A1 assigned to Korea Res. Inst. Bioscience & Biotechnology. Lp-$PLA_2$ inhibitors also include known agents, for example but not limited to include the use of statins with Niacin (see www.genengnews.com/news/bnitem.aspx?name=6724568) and fenofibrate (see www.genengnews.com/news/bnitem.aspx?name=14817756&taxid=19).
It is believed that the compounds disclosed for the purpose of the the present invention and defined as in the appended claims, are useful for prophylaxis or treatment of macular edema of any cause, e.g., due to RVO, inflammation, post-surgical, traction, and the like; AMD; uveitis; diabetic eye diseases and disorders; diabetic retinopathy, and the like. The models described herein as exemplified in the Examples can be used by one of ordinary skill in the art to determine which of the disclosed compounds or other inhibitors of Lp-$PLA_2$, for example antibodies, or RNAi are effective for the treatment or prevention of macular edema of any cause, e.g., due to RVO, inflammation, post-surgical, traction, and the like; age-related macular degeneration (AMD); uveitis; diabetic eye diseases and disorders; diabetic retinopathy, and the like, as claimed herein.
Lp-$PLA_2$ inhibitors are disclosed in U.S. patent 6,649,619 and 7,153,861, (and International Application WO 01/60805) and U.S. patent 7,169,924 (and International Patent Application WO 02/30911), and in U.S. publication No. 2005/0033052A1, and International Patent Applications WO 02/30904, WO 03/042218, WO 03/042206, WO03/042179, WO 03/041712, WO 03/086400, and WO 03/87088 are reversible Lp-$PLA_2$ inhibitors.
**Formula (I)** A group of reversible Lp-$PLA_2$ inhibitors are disclosed in international application WO 01/60805,

from which arose U.S. patents 6,649,619 and 7,153,861. A narrower group of compounds are of formula (I) described in WO 01/60805 and claimed in U.S. patents 6,649,619 and 7,153,861, namely:

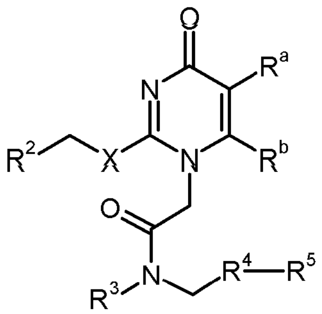

(I)

wherein:

$R^a$ and $R^b$ together with the pyrimidine ring carbon atoms to which they are attached form a fused 5-membered carbocyclic ring;
$R^2$ is phenyl, substituted by one to three fluorine atoms;
$R^3$ is methyl or $C_{(1-3)}$alkyl substituted by $NR^8R^9$; or
$R^3$ is Het-$C_{(0-2)}$alkyl in which Het is a 5- to 7- membered heterocyclyl ring having N and in which N is unsubstituted or substituted by $C_{(1-6)}$alkyl;
$R^4$ and $R^5$ together form a 4-(4-trifluoromethylphenyl)phenyl moiety;
$R^8$ and $R^9$ which can be the same or different are selected from the group consisting of hydrogen, or $C_{(1-6)}$alkyl);
X is S, or a pharmaceutically acceptable salt thereof.

Of even more interest are the following compounds, all within the scope of formula (I) and disclosed in the application and patents noted above:

1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one, used in the pig study described herein;
1-(N-(2-(diethylamino)ethyl)-N-(4-(4-trifluoromethylphenyl)benzyl)-aminocarbonylmethyl)-2-(2,3-difluorobenzyl)thio-5,6-trimethylenepyrimidin-4-one;
1-(N-(2-(diethylamino)ethyl)-N-(4-(4-trifluoromethylphenyl)benzyl)amino-carbonylmethyl)-2-(3,4-difluorobenzyl)thio-5,6-trimethylenepyrimidin-4-one;
1-(N-(2-(diethylamino)ethyl)-N-(4-(4-trifluoromethylphenyl)benzyl)amino-carbonylmethyl)-2-(2,3,4-trifluorobenzyl)thio-5,6-trimethylenepyrimidin-4-one;
1-(N-(2-(diethylamino)ethyl)-N-(4-(4-trifluoromethylphenyl)benzyl)amino-carbonylmethyl)-2-(2-fluorobenzyl)thio-5,6-trimethylenepyrimidin-4-one;
1-(N-methyl-N-(4-(4-trifluoromethylphenyl)benzyl)aminocarbonylmethyl)-2-(4-fluorobenzyl)thio-5,6-trimethylenepyrimidin-4-one;
1-(N-(2-(1-piperidino)ethyl)-N-(4-(4-trifluoromethylphenyl)benzyl)amino-carbonylmethyl)-2-(4-fluorobenzyl)thio-5,6-trimethylenepyrimidin-4-one;
1-(N-(1-ethylpiperidin-4-yl)-N-(4-(4-trifluoromethylphenyl)benzyl)amino-carbonylmethyl)-2-(4-fluorobenzyl)thio-5,6-trimethylenepyrimidin-4-one;
1-(N-(2-ethylamino-2-methylpropyl)-N-(4-(4-trifluoromethylphenyl)benzyl)-aminocarbonylmethyl)-2-(4-fluorobenzyl)thio-5,6-trimethylenepyrimidin-4-one;
N-(2-tert-butylaminoethyl)-N-(4-(4-trifluoromethylphenyl)benzyl)amino-carbonylmethyl)-2-(4-fluorobenzyl)thio-5,6-trimethylenepyrimidin-4-one;
1-(N-(1-methylpiperidin-4-yl)-N-(4-(4-trifluoromethylphenyl)benzyl)-aminocarbonylmethyl)-2-(4-fluorobenzyl)thio-5,6-trimethylenepyrimidin-4-one;
1-(N-(1-isopropylpiperidin-4-yl)-N-(4-(4-trifluoromethylphenyl)benzyl)-aminocarbonylmethyl)-2-(4-fluorobenzyl)thio-5,6-trimethylenepyrimidin-4-one;
1-(N-(1-(2-methoxyethyl)piperidin-4-yl)-N-(4-(4-trifluoromethylphenyl)benzyl)-aminocarbonylmethyl)-2-(4-fluorobenzyl)thio-5,6-trimethylenepyrimidin-4-one;
1-(N-(2-(ethylamino)ethyl)-N-(4-(4-trifluoromethylphenyl)benzyl)-aminocarbonylmethyl)-2-(4-fluorobenzyl)thio-5,6-trimethylenepyrimidin-4-one; or a pharmaceutically acceptable salt of these compounds.

Methods for preparing these compounds are disclosed in the noted documents.

A second process for making 1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one can be found in application WO 03/016287 (U.S. 7,232,902).

**Formula (II)**

Other compounds are disclosed in WO 02/30911; US patent 7,169,924 corresponds to this international application. The generic formula in that case, represented here as formula (II), is as follows:

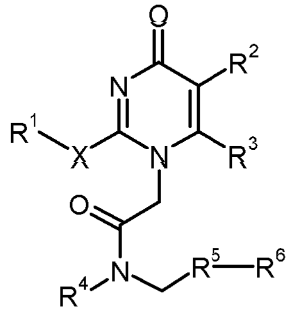

(II)

in which:

$R^1$ is an aryl group, optionally substituted by 1, 2, 3 or 4 substituents which can be the same or different selected from $C_{(1-6)}$alkyl, $C_{(1-6)}$alkoxy, $C_{(1-6)}$alkylthio, hydroxy, halogen, CN, and mono to perfluoro-$C_{(1-4)}$alkyl;

$R^2$ is halogen, $C_{(1-3)}$alkyl, $C_{(1-3)}$alkoxy, hydroxy$C_{(1-3)}$alkyl, $C_{(1-3)}$alkylthio, $C_{(1-3)}$alkylsulphinyl, amino$C_{(1-3)}$alkyl, mono- or di-$C_{(1-3)}$alkylamino$C_{(1-3)}$alkyl, $C_{(1-3)}$alkylcarbonylamino$C_{(1-3)}$alkyl, $C_{(1-3)}$alkoxy$C_{(1-3)}$alkylcarbonylamino$C_{(1-3)}$alkyl, $C_{(1-3)}$alkylsulphonylamino$C_{(1-3)}$alkyl, $C_{(1-3)}$alkylcarboxy, $C_{(1-3)}$alkylcarboxy$C_{(1-3)}$alkyl, and

$R^3$ is hydrogen, halogen, $C_{(1-3)}$alkyl, or hydroxy$C_{(1-3)}$alkyl; or

$R^2$ and $R^3$ together with the pyrimidone ring carbon atoms to which they are attached form a fused 5-or 6-membered carbocyclic ring; or

$R^2$ and $R^3$ together with the pyrimidone ring carbon atoms to which they are attached form a fused benzo or heteroaryl ring ring optionally substituted by 1, 2, 3 or 4 substituents which can be the same or different selected from halogen, $C_{(1-4)}$alkyl, cyano, $C_{(1-6)}$alkoxy, $C_{(1-6)}$alkylthio or mono to perfluoro-$C_{(1-4)}$alkyl;

$R^4$ is hydrogen, $C_{(1-6)}$alkyl which can be unsubstituted or substituted by 1, 2 or 3 substituents selected from hydroxy, halogen, $OR^7$, $COR^7$, carboxy, $COOR^7$, $CONR^9R^{10}$, $NR^9R^{10}$, $NR^7COR^8$, mono- or di-(hydroxy$C_{(1-6)}$alkyl)amino and N-hydroxy$C_{(1-6)}$alkyl-N-$C_{(1-6)}$alkylamino; or

$R^4$ is Het-$C_{(0-4)}$alkyl in which Het is a 5- to 7- membered heterocyclyl ring comprising N and optionally O or S, and in which N can be substituted by $COR^7$, $COOR^7$, $CONR^9R^{10}$, or $C_{(1-6)}$alkyl optionally substituted by 1, 2 or 3 substituents selected from hydroxy, halogen, $OR^7$, $COR^7$, carboxy, $COOR^7$, $CONR^9R^{10}$ or $NR^9R^{10}$, for instance, piperidin-4-yl, pyrrolidin-3-yl;

$R^5$ is an aryl or a heteroaryl ring optionally substituted by 1, 2, 3 or 4 substituents which can be the same or different selected from $C_{(1-6)}$alkyl, $C_{(1-6)}$alkoxy, $C_{(1-6)}$alkylthio, aryl$C_{(1-6)}$alkoxy, hydroxy, halogen, CN, $COR^7$, carboxy, $COOR^7$, $NR^7COR^8$, $CONR^9R^{10}$, $SO_2NR^9R^{10}$, $NR^7SO_2R^8$, $NR^9R^{10}$, mono to perfluoro-$C_{(1-4)}$alkyl and mono to perfluoro-$C_{(1-4)}$alkoxy;

$R^6$ is an aryl or a heteroaryl ring which is further optionally substituted by 1, 2, 3 or 4 substituents which can be the same or different selected from $C_{(1-18)}$alkyl, $C_{(1-18)}$alkoxy, $C_{(1-6)}$alkylthio, $C_{(1-6)}$alkylsulfonyl, aryl$C_{(1-6)}$alkoxy, hydroxy, halogen, CN, $COR^7$, carboxy, $COOR^7$, $CONR^9R^{10}$, $NR^7COR^8$, $SO_2NR^9R^{10}$, $NR^7SO_2R^8$, $NR^9R^{10}$, mono to perfluoro-$C_{(1-4)}$alkyl and mono to perfluoro-$C_{(1-4)}$alkoxy, or $C_{(5-10)}$alkyl;

$R^7$ is hydrogen or $C_{(1-12)}$alkyl, for instance $C_{(1-4)}$alkyl (e.g. methyl or ethyl);

$R^8$ is hydrogen, O$C_{(1-6)}$alkyl, or $C_{(1-12)}$alkyl, for instance $C_{(1-4)}$alkyl (e.g. methyl or ethyl);

$R^9$ and $R^{10}$ which can be the same or different is each selected from hydrogen, or $C_{(1-12)}$alkyl, or $R^9$ and $R^{10}$ together with the nitrogen to which they are attached form a 5-to 7 membered ring optionally containing one or more further heteroatoms selected from oxygen, nitrogen and sulphur, and optionally substituted by one or two substituents selected from hydroxy, oxo, $C_{(1-4)}$alkyl, $C_{(1-4)}$alkylcarboxy, aryl, e.g. phenyl, or aralkyl, e.g. benzyl, for instance morpholine or piperazine; and

X is $C_{(2-4)}$alkylene, optionally substituted by 1,2 or 3 substituents selected from methyl and ethyl, or CH=CH.

All salts of formula (II), as well, can be used in the instant method of treatment.

Of particular interest are the compounds of formula (II) here, where, as noted in WO 02/30911 for formula (I) there, $R^1$ can be a phenyl group optionally substituted by 1, 2, 3 or 4 substituents which can be the same or different selected from halo, $C_1$-$C_6$ alkyl, trifluoromethyl or $C_1$-$C_6$ alkoxy. More specifically, phenyl is unsubstituted or substituted by 1, 2, 3 or 4 halogen substituents, particularly, from 1 to 3 fluoro groups, and most particularly, 2,3-difluoro, 2,4-difluoro or 4-fluoro.

A further embodiment of formula (II) here is where X is $-CH_2CH_2-$.

In addition, of interest are compounds of formula (II) where $R^2$ is hydrogen, by default, or is halo, $C_1$-$C_6$ alkyl, mono to perfluoro- $C_1$-$C_4$ alkyl, mono to perfluoro $C_1$-$C4_6$ alkoxy, or $C_1$-$C_6$ alkoxy; particularly mono to perfluoro- $C_1$-$C_4$ alkyl, mono to perfluoro- $C_1$-$C_4$ alkoxy, or $C_1$-$C_6$ alkoxy. Of particular interest are the compounds of formula (II) where $R^2$ is other than hydrogen, n in $(R^2)_n$ is 1, 2, or 3, and the substitution pattern is meta and/or para, particularly para, i.e. a 4-position substituent. See also those compounds where $R^2$ is 4-trifluoromethyl or 4-trifluoromethoxy.

$R^3$ and $R^4$ can be the same or different and are methyl, ethyl, n-propyl, or n-butyl. Of particular interest are those compounds of formula (II) herein where $R^3$ and $R^4$ are the same and are methyl, or ethyl; methyl is of particular interest.

$R^5$ can be hydrogen, $-C_{(1-6)}$ alkyl which is a straight chain, or branched. Of particular interest is methyl, ethyl, propyl, isopropyl, n-butyl, sec-butyl, *iso*-butyl, *t*-butyl, *n*-pentyl or *n*-hexyl.

It will be appreciated that within the compounds of formula (II) herein there is a further sub-group of compounds in which:

$R^1$ is phenyl substituted by 2,3-difluoro;
$R^2$ and $R^3$, together with the pyrimidine ring carbon atoms to which they are attached, form a fused 5-membered cyclopentenyl ring;
$R^4$ is 2-(diethylamino)ethyl;
$R^5$ is phenyl;
$R^6$ is phenyl substituted by trifluoromethyl at the 4-position, or thien-2-yl substituted by trifluoromethyl in the 5-position; and
X is $-(CH_2)_2$.

Of particular interest are the compounds of formula (II) here, where, as noted in WO 02/30911 for formula (I) there, $R^1$ can be a phenyl group optionally substituted by 1, 2, 3 or 4 substituents which can be the same or different selected from halo, $C_1$-$C_6$ alkyl, trifluoromethyl or $C_1$-$C_6$ alkoxy. More specifically, phenyl is unsubstituted or substituted by 1, 2, 3 or 4 halogen substituents, particularly, from 1 to 3 fluoro groups, and most particularly, 2,3-difluoro, 2,4-difluoro or 4-fluoro.

A further embodiment of formula (II) here is where X is $-CH_2CH_2-$; $R^2$ and $R^3$, together with the pyrimidine ring carbon atoms to which they are attached, form a fused 5-membered benzo ring; $R^4$ is Het-$C_{(0-4)}$alkyl in which Het is a 5- to 7- membered heterocyclyl ring comprising N, and in which N can be substituted by $C_{(1-6)}$alkyl, for instance, piperidin-4-yl, pyrrolidin-3-yl; $R^5$ is an aryl ring; $R^6$ is an aryl ring which is further optionally substituted by 1, 2, 3 or 4 substituents which can be the same or different selected from $C_{(1-18)}$alkyl, halogen, in particular, 4-chloro.

Particular compounds of formula (II) herein of interest are:

*N*-(1-ethylpiperidin-4-yl)-2-(2-(2-(2,3-difluorophenyl)-ethyl)-4-oxo-4*H*-quninazolin-1-yl)-*N*-(4'-chloro-biphenyl-4-ylmethyl)acetamide bitartrate;
*N*-(1-Ethyl-piperidin-4-yl)-2-(2-(2-(2,3-difluorophenyl)-ethyl)-4-oxo-4*H*-quinazolin-1-yl)-*N*-(4'-trifluoromethyl-biphenyl-4-ylmethyl)acetamide bitartrate;
*N*-(2-diethylaminoethyl)-2-[2-(2-(2,3-difluorophenyl)ethyl)-4-oxo-4,5,6,7-tetrahydro-cyclopentapyrimidin-1-yl]-*N*-(4'-trifluoromethyl-biphenyl-4-ylmethyl)acetamide;
*N*-(2-diethylaminoethyl)-2-[2-(2-(2,3-difluorophenyl)ethyl)-4-oxo-4,5,6,7-tetrahydro-cyclopentapyrimidin-1-yl]-*N*-(4'-trifluoromethyl-biphenyl-4-ylmethyl)acetamide bitartrate;
*N*-(2-ethylamino-2-methyl-propyl)-2-(2-(2-(2,3-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-*t*-butylaminoethyl)-2-(2-(2-(2,3-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(1-ethyl-piperidin-4-yl)-2-(2-(2-(2,3-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(4-fluoro-2-(trifluoromethyl)phenyl)-ethyl)-4-oxo-4,5,6,7-tetrahydro-cyclopentapyrimidin-1-yl)-*N*-(4'-trifluoromethyl-biphenyl-4-ylmethyl)-acetamide bitartrate;
*N*-(2-diethylaminoethyl)-2-(2-(2-(4-fluoro-3-(trifluoromethyl)phenyl)-ethyl)-4-oxo-4,5,6,7-tetrahydro-cyclopentapyrimidin-1-yl)-*N*-(4'-trifluoromethyl-biphenyl-4-ylmethyl)-acetamide bitartrate;
*N*-(2-diethylaminoethyl)-2-(2-(2-(3-chloro-4-fluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
(+/-)-*N*-(2-diethylaminoethyl)-2-(2phenyl-propyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(2,4-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(2,5-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(3,4-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(2-fluorophenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(3-fluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(3-chlorophenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(4-chlorophenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(4-methylphenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(4-(trifluoromethyl)phenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(4-methoxyphenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(4-(trifluoromethoxy)phenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
or the free base of any of the bitartrate salts , or another pharmaceutically acceptable salt.

Methods for preparing these compounds are disclosed in the noted documents.

**Formula (III)**

Further, are compounds of formula (III), disclosed in WO 02/30904:

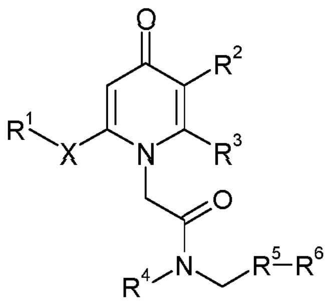
(III)

in which:

$R^1$ is an aryl group, optionally substituted by 1, 2, 3 or 4 substituents which can be the same or different selected from $C_{(1-6)}$alkyl, $C_{(1-6)}$alkoxy, $C_{(1-6)}$alkylthio, hydroxy, halogen, CN, mono to perfluoro-$C_{(1-4)}$alkyl, mono to perfluoro-$C_{(1-4)}$alkoxyaryl, and aryl$C_{(1-4)}$alkyl;
$R^2$ is halogen, $C_{(1-3)}$alkyl, $C_{(1-3)}$alkoxy, hydroxy$C_{(1-3)}$alkyl, $C_{(1-3)}$alkylthio, $C_{(1-3)}$alkylsulphinyl, amino$C_{(1-3)}$alkyl, mono- or di-$C_{(1-3)}$alkylamino$C_{(1-3)}$alkyl, $C_{(1-3)}$alkylcarbonylamino$C_{(1-3)}$alkyl, $C_{(1-3)}$alkoxy$C_{(1-3)}$alkylcarbonylamino$C_{(1-3)}$alkyl, $C_{(1-3)}$alkylsulphonylamino$C_{(1-3)}$alkyl, $C_{(1-3)}$alkylcarboxy, $C_{(1-3)}$alkylcarboxy$C_{(1-3)}$alkyl, and
$R^3$ is hydrogen, halogen, $C_{(1-3)}$alkyl, or hydroxy$C_{(1-3)}$alkyl; or
$R^2$ and $R^3$ together with the pyridone ring carbon atoms to which they are attached form a fused 5-or 6-membered carbocyclic ring; or

$R^2$ and $R^3$ together with the pyridone ring carbon atoms to which they are attached form a fused benzo or heteroaryl ring optionally substituted by 1, 2, 3 or 4 substituents which can be the same or different selected from halogen, $C_{(1-4)}$alkyl, cyano, $C_{(1-3)}$alkoxy$C_{(1-3)}$alkyl, $C_{(1-4)}$alkoxy or $C_{(1-4)}$alkylthio, or mono to perfluoro-$C_{(1-4)}$alkyl;
$R^4$ is hydrogen, $C_{(1-6)}$alkyl which can be unsubstituted or substituted by 1, 2 or 3 substituents selected from hydroxy, halogen, $OR^7$, $COR^7$, carboxy, $COOR^7$, $CONR^9R^{10}$, $NR^9R^{10}$, $NR^7COR^8$, mono- or di-(hydroxy$C_{(1-6)}$alkyl)amino and N-hydroxy$C_{(1-6)}$alkyl-N-$C_{(1-6)}$alkylamino; or
$R^4$ is Het-$C_{(0-4)}$alkyl in which Het is a 5- to 7- membered heterocyclyl ring comprising N and optionally O or S, and in which N can be substituted by $COR^7$, $COOR^7$, $CONR^9R^{10}$, or $C_{(1-6)}$alkyl optionally substituted by 1, 2 or 3 substituents selected from hydroxy, halogen, $OR^7$, $COR^7$, carboxy, $COOR^7$, $CONR^9R^{10}$ or $NR^9R^{10}$, for instance, piperidin-4-yl, pyrrolidin-3-yl;
$R^5$ is an aryl or a heteroaryl ring optionally substituted by 1, 2, 3 or 4 substituents which can be the same or different selected from $C_{(1-6)}$alkyl, $C_{(1-6)}$alkoxy, $C_{(1-6)}$alkylthio, aryl$C_{(1-6)}$alkoxy, hydroxy, halogen, CN, $COR^7$, carboxy, $COOR^7$, $NR^7COR^8$, $CONR^9R^{10}$, $SO_2NR^9R^{10}$, $NR^7SO_2R^8$, $NR^9R^{10}$, mono to perfluoro-$C_{(1-4)}$alkyl and mono to perfluoro-$C_{(1-4)}$alkoxy;
$R^6$ is an aryl or a heteroaryl ring which is further optionally substituted by 1, 2, 3 or 4 substituents which can be the same or different selected from $C_{(1-6)}$alkyl, $C_{(1-6)}$alkoxy, $C_{(1-6)}$alkylthio, $C_{(1-6)}$alkylsulfonyl, aryl$C_{(1-6)}$alkoxy, hydroxy, halogen, CN, $COR^7$, carboxy, $COOR^7$, $CONR^9R^{10}$, $NR^7COR^8$, $SO_2NR^9R^{10}$, $NR^7SO_2R^8$, $NR^9R^{10}$, mono to perfluoro-$C_{(1-4)}$alkyl and mono to perfluoro-$C_{(1-4)}$alkoxy, or $C_{(5-10)}$alkyl;
$R^7$ and $R^8$ are independently hydrogen or $C_{(1-12)}$alkyl, for instance $C_{(1-4)}$alkyl (e.g. methyl or ethyl);
$R^9$ and $R^{10}$ which can be the same or different is each selected from hydrogen, or $C_{(1-12)}$alkyl, or $R^9$ and $R^{10}$ together with the nitrogen to which they are attached form a 5-to 7 membered ring optionally containing one or more further heteroatoms selected from oxygen, nitrogen and sulphur, and optionally substituted by one or two substituents selected from hydroxy, oxo, $C_{(1-4)}$alkyl, $C_{(1-4)}$alkylcarboxy, aryl, e.g. phenyl, or aralkyl, e.g., benzyl, for instance morpholine or piperazine; and
X is a $C_{(2-4)}$alkylene group (optionally substituted by 1, 2 or 3 substituents selected from methyl and ethyl), CH=CH, $(CH_2)_nS$ or $(CH_2)_nO$ where n is 1, 2 or 3;
or a pharmaceutically acceptable salt thereof.

Of particular interest are those compounds of formula (III) where $R^2$ and $R^3$ together with the pyridone ring carbon atoms to which they are attached form a fused benzo or heteroaryl ring optionally substituted by 1, 2, 3 or 4 substituents which can be the same or different selected from halogen, $C_{(1-4)}$alkyl, cyano, $C_{(1-4)}$alkoxy or $C_{(1-4)}$alkylthio, or mono to perfluoro-$C_{(1-4)}$alkyl. Suitably, $R^1$ is phenyl optionally substituted by halogen, $C_{(1-6)}$alkyl, trifluoromethyl, $C_{(1-6)}$alkoxy, Suitably, from 1 to 3 fluoro, more Suitably, 2,3-difluoro. Representative examples of $R^4$ include piperidin-4-yl substituted at the 1-position by methyl, isopropyl, 1-(2-methoxyethyl), 1-(2-hydroxyethyl), t-butoxycarbonyl or ethoxycarbonylmethyl; ethyl substituted at the 2-position by aminoethyl; 1-ethylpiperidinylmethyl; piperidin-4-yl; 3-diethylaminopropyl; 4-pyrrolidin-1-ylbutyl and 1-ethylpyrrolidin-3-yl. Suitably $R^4$ is 1-(2-methoxyethyl)piperidin-4-yl, 1-methylpiperidin-4-yl or 1-ethylpyrrolidin-3-yl. Representative examples of $R^5$ include phenyl and pyridyl. Suitably, $R^5$ is phenyl. Representative examples of $R^6$ include phenyl optionally substituted by halogen, or trifluoromethyl, Suitably at the 4-position and hexyl. Suitably, $R^6$ is phenyl substituted by trifluoromethyl at the 4-position. Further representative examples of $R^6$ include phenyl substituted by 1 or more $C_{(1-3)}$alkyl. Suitably, $R^6$ is phenyl substituted by ethyl in the 4-position. Suitably, $R^5$ and $R^6$ together form a 4-(phenyl)phenyl or a 2-(phenyl)pyridinyl substituent in which the remote phenyl ring can be optionally substituted by halogen or trifluoromethyl, suitably at the 4-position. Preferably X is $C_{(2-4)}$alkylene, more preferably $C_{(2-3)}$alkylene, most preferably, $(CH_2)_2$, or $CH_2S$.

It will be appreciated that within the group of compounds comprising formula (III) there is sub-group of compounds in which:

$R^1$ is phenyl substituted by 2,3-difluoro;
$R^2$ and $R^3$, together with the pyridone ring carbon atoms to which they are attached, form a fused benzo or pyrido ring;
$R^4$ is 1-(2-methoxyethyl)piperidin-4-yl;
$R^5$ and $R^6$ together form a 4-(phenyl)phenyl substituent in which the remote phenyl ring is substituted by trifluoromethyl, preferably at the 4-position; and
X is $CH_2S$ or $(CH_2)_2$.

The following compounds of formula (III) are of interest:

*N*-(2-diethylaminoethyl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-4-oxo-4*H*-quinolin-1-yl]-*N*-(4'-trifluoromethylbiphenyl-4-ylmethyl)acetamide;
*N*-(2-diethylaminoethyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2,3-difluorobenzylthio)-4-oxo-5,6-trimethylenepyridin-1-yl]-*N*-(4'-trifluoromethylbiphenyl-4-ylmethyl)acetamide bitartrate;
*N*-(1-(2-methoxyethyl)piperidin-4-yl)-2-[2-(2,3-difluorobenzylthio)-4-oxo-4*H*-quinolin-1-yl]-*N*-(4'-trifluoromethylbiphenyl-4-ylmethyl)acetamide;
*N*-(1-methylpiperidin-4-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-methylpiperidin-4-yl)-2-[2-(2-(2,3-difluorophenyl)ethyl)-4-oxo-4*H*-[1,8]naphthyridin-1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-4-oxo-4*H*-[1,8]naphthyridin-1-yl]-*N*-(4'-trifluoromethylbiphenyl-4-ylmethyl)acetamide;
*N*-(1-(2-methoxyethyl)piperidin-4-yl)-2-[2-(2,3-difluorobenzylthio)-4-oxo-4*H*-[1,8]naphthyridin-1-yl]-*N*-(4'-trifluoromethylbiphenyl-4-ylmethyl)acetamide bitartrate;
*N*-(1-ethylpiperidin-4-yl)-2-[2-(2,3-difluorobenzylthio)-4-oxo-4*H*-quinolin-1-yl]-*N*-(4'-ethylbiphenyl-4-ylmethyl)acetamide bitartrate;
*N*-(1-ethylpiperidin-4-yl)-2-[5-(2-(2,3-difluorophenyl)ethyl)-2-methyl-7-oxo-7*H*-thiazolo[4,5-b]pyridin-4-yl]-*N*-(4'-trifluoromethylbiphenyl-4-ylmethyl)acetamide bitartrate;
(±)*N*-(1-ethylpyrrolidin-3-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
(±)*N*-(1-ethylpyrrolidin-3-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-(2-methoxyethyl)piperidin-4-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-(2-methoxyethyl)piperidin-4-yl)-2-[2-(2,3-difluorobenzylthio)-4-oxo-4*H*-quinolin-1-yl]-*N*-(4'-trifluoromethylbiphenyl-4-ylmethyl)acetamide dihydrochloride;
*N*-(1-(2-methoxyethyl)piperidin-4-yl)-2-[2-(2,3-difluorobenzylthio)-4-oxo-4*H*-quinolin-1-yl]-*N*-(4'-trifluoromethylbiphenyl-4-ylmethyl)acetamide mono paratoluenesulphonate;
*N*-(1-(2-methoxyethyl)piperidin-4-yl)-2-[2-(2-(2,3-difluorophenyl)ethyl)-4-oxo-4*H*-[1,8]naphthyridin -1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-4-oxo-4*H*-[1,8]naphthyridin -1-yl]-*N*-(4'-trifluoromethylbiphenyl-4-ylmethyl)acetamide monohydrochloride;
*N*-(1-(2-methoxyethyl)piperidin-4-yl)-2-[2-(2-(2,3-difluorophenyl)ethyl)-4-oxo-4*H*-[1,8]naphthyridin-1-yl]-*N*-(4'-trifluoromethylbiphenyl-4-ylmethyl)acetamide dihydrochloride;
*N*-(2-diethylaminoethyl)-2-[2-(4-fluorobenzylthio)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(4-fluorobenzylthio)-4-oxo-5,6-trimethylenepyridin-1-yl]-*N*-(4'-trifluoromethyl-biphenyl-4-ylmethyl)acetamide bitartrate;
*N*-(2-diethylaminoethyl)-2-[2-(2,3-difluorobenzylthio)-4-oxo-5,6-trimethylenepyridin-1-yl]-*N*-(4'-trifluoromethyl-biphenyl-4-ylmethyl)acetamide bitartrate;
*N*-(2-diethylaminoethyl)-2-[2-(2-(4-fluorophenyl)ethyl)-4-oxo-4*H*-quinolin-1-yl]-*N*-(4'-trifluoromethylbiphenyl-4-ylmethyl)acetamide;
*N*-(2-diethylaminoethyl)-2-[2-(2-(3,4-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2-(2-fluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2-(3-chlorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-4-oxo-4*H*-[1,8]naphthyridin-1-yl)]-*N*-(4'-trifluoromethylbiphenyl-4-ylmethyl)acetamide bitartrate;
*N*-(1-ethylpiperidin-4-yl)-2-[2-(2-(2,3-difluorophenyl)ethyl)-4-oxo-4*H*-[1,8]naphthyridin-1-yl)]-*N*-(4'-trifluoromethylbiphenyl-4-ylmethyl)acetamide;
*N*-(1-ethylpiperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-pyrrolidin-1-ylethyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(1-isopropylpiperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-piperidin-1-ylethyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2,3-difluorobenzylthio)7-fluoro-4-oxo-4*H*-quinolin-1-yl]-*N*-(4'-trifluoromethylbiphenyl-4-ylmethyl)acetamide bitartrate;
*N*-(2-diethylaminoethyl)-5-[2-(2-(2,3-difluorophenyl)ethyl)-2-methyl-7-oxo-7*H*-thieno[3,2-b]pyridin-4-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-5,6-dimethyl-4-oxo-4*H*-pyridin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-5-ethyl-4-oxo-4*H*-pyridin-1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(1-methylpiperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-4-oxo-4*H*-thieno[3,4-b]pyridin-1-yl]-*N*-(4'-trifluoromethylbiphenyl-4-ylmethyl)acetamide bitartrate;
*N*-(1-ethylpiperidin-4-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(2-pyrrolidin-1-ylethyl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-ethylpiperidin-4-yl)-2-[6-(2-(2,3-difluorophenyl)ethyl)-2-methyl-4-oxo-4*H*-pyrazolo[3,4-b]pyridin-7-yl]-*N*-(4'-trifluoromethylbiphenyl-4-ylmethyl)acetamide bitartrate;
*N*-(1-isopropylpiperidin-4-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-ethylpiperidin-4-ylmethyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(3-diethylaminopropyl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(4-pyrrolidin-1-ylbutyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(3-diethylaminopropyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(4-pyrrolidin-1-ylbutyl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-ethylpiperidin-4-yl)-2-[5-(2,3-difluorobenzylthio)-7-oxo-7*H*-thieno[3,2-b]pyridin-4-yl]-*N*-(4'-trifluoromethylbiphenyl-4-ylmethyl)acetamide bitartrate;
*N*-(2-diethylaminoethyl)-2-[5-(2-(2,3-difluorophenyl)ethyl)-2-methyl-7-oxo-7*H*-thiazolo[4,5-b]pyridin-4-yl]-*N*-(4'-trifluoromethylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N*-(4'-ethylbiphenyl-4-ylmethyl)acetamide bitartrate;
*N*-(2-diethylaminoethyl)-2-[2-(2-(2,3-difluorophenyl)ethyl)-4-oxo-4*H*-quinolin-1-yl]-*N*-(4'-ethylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N*-(4'-isopropylbiphenyl-4-ylmethyl)acetamide bitartrate;
*N*-(2-diethylaminoethyl)-2-[2-(2-(2,3-difluorophenyl)ethyl)-4-oxo-4*H*-quinolin-1-yl]-*N*-(4'-isopropylbiphenyl-4-ylmethyl)acetamide bitartrate;
*N*-(2-diethylaminoethyl)-2-[2-(2,3-difluorobenzylthio)-4-oxo-4*H*-[1,8]naphthyridin-1-yl]-*N*-(4'-trifluoromethylbiphenyl-4-ylmethyl)acetamide bitartrate;
*N*-(1-ethylpiperidin-4-yl)-2-[2-(2,3-difluorobenzylthio)-4-oxo-4*H*-[1,8]naphthyridin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-4-oxo-4*H*-quinolin-1-yl]-*N*-(4'-methylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N*-(4'-methylbiphenyl-4-ylmethyl)acetamide bitartrate;
*N*-(1-ethoxycarbonylmethylpiperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(1-isopropylpiperidin-4-yl)-2-[2-(2-(2,3-difluorophenyl)ethyl)-4-oxo-4*H*-[1,8]naphthyridin-1-yl]-*N*-(4'-trifluoromethylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N*-(3',4'-dimethylbiphenyl-4-ylmethyl)acetamide bitartrate;
*N*-(1-(t-butoxycarbonyl)piperidin-4-yl)-2-[2-(2-(2,3-difluorophenyl)ethyl)-4-oxo-4*H*-[1,8]naphthyridin-1-yl]-*N*-(4'-trifluoromethylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N*-(3',4'-difluorobiphenyl-4-ylmethyl)acetamide bitartrate;
*N*-(2-diethylaminoethyl)-2-[6-(2,3-difluorobenzylthio)-4-oxo-4*H*-thieno[2,3-b]pyridin-7-yl]-*N*-(4'-trifluoromethylbiphenyl-4-ylmethyl)acetamide bitartrate;
*N*-(1-methylpiperidin-4-yl)-2-[2-(2,3-difluorobenzylthio)-4-oxo-4*H*-[1,8]naphthyridin-1-yl]-*N*-(4'-trifluoromethylbiphenyl-4-ylmethyl)acetamide bitartrate;
*N*-(1-ethylpiperidin-4-yl)-2-[2-(2-(2,3,4-trifluorophenylethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[6-(2,3-difluorobenzylthio)-2-methyl-4-oxo-2,4-dihydro-pyrazolo[3,4-b]pyridin-7-yl]-*N*-(4'-trifluoromethylbiphenyl-4-ylmethyl)acetamide bitartrate;
*N*-(1-ethylpiperidin-4-yl)-2-[6-(2-(2,3-difluorophenyl)ethyl)-2-ethyl-4-oxo-2,4-dihydropyrazolo[3,4-b]pyridin-7-yl]-*N*-(4'-trifluoromethylbiphenyl-4-ylmethyl)acetamide bitartrate;
*N*-(1-ethylpiperidin-4-yl)-2-[6-(2-(2,3-difluorophenyl)ethyl)-2-isopropyl-4-oxo-2,4-dihydropyrazolo[3,4-b]pyridin-7-yl]-*N*-(4'-trifluoromethylbiphenyl-4-ylmethyl)acetamide bitartrate;
*N*-(1-ethylpiperidin-4-yl)-2-[2-(2-(2,3-difluorophenyl)ethyl)-4-oxo-4*H*-quinolin-1-yl]-*N*-(4'-ethylbiphenyl-4-ylmethyl)acetamide bitartrate;
*N*-(1-isopropylpiperidin-4-yl)-2-[5-(2-(2,3-difluorophenyl)ethyl)-2-methyl-7-oxo-7*H*-thiazolo[4,5-b]pyridin-4-yl]-*N*-(4'-trifluoromethylbiphenyl-4-ylmethyl)acetamide bitartrate;
*N*-(1-(2-methoxyethyl)piperidin-4-yl)-2-[5-(2-(2,3-difluorophenyl)ethyl)-2-methyl-7-oxo-7*H*-thiazolo[4,5-b]pyridin-4-yl]-*N*-(4'-trifluoromethylbiphenyl-4-ylmethyl)acetamide bitartrate;
*N*-(1-ethylpiperidin-4-yl)-2-[2-(2-(2,3-difluorophenyl)ethyl)-4-oxo-5,6-trimethylene-pyridin-1-yl]-*N*-(4'-trifluoromethylbiphenyl-4-ylmethyl)acetamide bitartrate;
*N*-(1-methylpiperidin-4-yl)-2-[2-(2-(2,3-difluorophenyl)ethyl)-4-oxo-5,6-trimethylene-pyridin-1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-4-oxo-5,6-trimethylenepyridin-1-yl]-*N*-(4'-trifluoromethylbiphenyl-4-ylmethyl)acetamide bitartrate;
*N*-(1-isopropylpiperidin-4-yl)-2-[2-(2-(2,3-difluorophenyl)ethyl)-4-oxo-5,6-trimethylenepyridin-1-yl]-*N*-(4'-trifluoromethylbiphenyl-4-ylmethyl)acetamide bitartrate;
*N*-(1-ethylpiperidin-4-yl)-2-[5-(2-(2,3-difluorophenyl)ethyl)-2-methyl-7-oxo-2,7-dihydropyrazolo[4,3-b]pyridin-4-yl]-*N*-(4'-trifluoromethylbiphenyl-4-ylmethyl)acetamide bitartrate;
*N*-(1-ethylpiperidin-4-yl)-2-[5-(2-(2,3-difluorophenyl)ethyl)-1-methyl-7-oxo-1,7-dihydropyrazolo[4,3-b]pyridin-4-yl]-*N*-(4'-trifluoromethylbiphenyl-4-ylmethyl)acetamide bitartrate;
*N*-(1-ethylpiperidin-4-yl)-2-[2-(2-(2,3-difluorophenyl)ethyl)-4-oxo-5,6-trimethylene-pyridin-1-yl]-*N*-(4'-trifluoromethylbiphenyl-4-ylmethyl)acetamide bitartrate;
*N*-(1-ethylpiperidin-4-yl)-2-[2-(2-(2,3-difluorophenyl)ethyl)-7-methyl-4-oxo-4*H*-[1,8]naphthyridin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-7-methyl-4-oxo-4*H*-[1,8]naphthyridin-1-yl]-*N*-(4'-trifluoromethylbiphenyl-4-ylmethyl)acetamide bitartrate;
*N*-(1-methylpiperidin-4-yl)-2-[2-(2-(2,3-difluorophenyl)ethyl)-7-methyl-4-oxo-4*H*-[1,8]naphthyridin-1-yl]-*N*-(4'-trifluoromethylbiphenyl-4-ylmethyl)acetamide bitartrate;
*N*-(1-isopropylpiperidin-4-yl)-2-[2-(2-(2,3-difluorophenyl)ethyl)-7-methyl-4-oxo-4*H*-[1,8]naphthyridin-1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-7-methyl-4-oxo-4*H*-[1,8]naphthyridin-1-yl]-*N*-(4'-trifluoromethylbiphenyl-4-ylmethyl)acetamide bitartrate;
*N*-(1-methylpiperidin-4-yl)-2-[2-(2,3-difluorobenzylthio)-4-oxo-5,6-trimethylene-pyridin-1-yl]-*N*-(4'-trifluoromethylbiphenyl-4-ylmethyl)acetamide bitartrate;
*N*-(1-ethylpiperidin-4-yl)-2-[2-(2,3-difluorobenzylthio)-4-oxo-5,6-trimethylenepyridin-1-yl]-*N*-(4'-trifluoromethylbiphenyl-4-ylmethyl)acetamide bitartrate;
*N*-(1-isopropylpiperidin-4-yl)-2-[2-(2,3-difluorobenzylthio)-4-oxo-5,6-trimethylene-pyridin-1-yl]-*N*-(4'-trifluoromethylbiphenyl-4-ylmethyl)acetamide bitartrate;
*N*-(1-(2-methoxyethyl)piperidin-4-yl)-2-[2-(2,3-difluorobenzylthio)-4-oxo-5,6-trimethylenepyridin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-4-oxo-5,6-tetramethylene-pyridin-1-yl]-*N*-(4'-trifluoromethylbiphenyl-4-ylmethyl)acetamide bitartrate;
*N*-(1-methylpiperidin-4-yl)-2-[2-(2,3-difluorobenzylthio)-4-oxo-4*H*-quinolin-1-yl]-*N*-(4'-chlorobiphenyl-4-ylmethyl)acetamide bitartrate;
*N*-(1-methylpiperidin-4-yl)-2-[2-(2-(2,3-difluorophenyl)ethyl)-4-oxo-4*H*-quinolin-1-yl]-*N*-(4'-chlorobiphenyl-4-ylmethyl)acetamide bitartrate;
*N*-(1-ethylpiperidin-4-yl)-2-[2-(2-(2,3-difluorophenyl)ethyl)-4-oxo-4*H*-quinolin-1-yl]-*N*-(4'-chlorobiphenyl-4-ylmethyl)acetamide bitartrate;
*N*-(1-(2-methoxyethyl)piperidin-4-yl)-2-[2-(2-(2,3-difluorophenyl)ethyl)-4-oxo-4*H*-quinolin-1-yl]-*N*-(4'-chlorobiphenyl-4-ylmethyl)acetamide bitartrate;
*N*-(1-isopropylpiperidin-4-yl)-2-[2-(2-(2,3-difluorophenyl)ethyl)-4-oxo-4*H*-quinolin-1-yl]-*N*-(4'-chlorobiphenyl-4-ylmethyl)acetamide bitartrate;
*N*-(2-diethylaminoethyl)-2-[6-(2-(2,3-difluorophenyl)ethyl)-2-methyl-4-oxo-4*H*-pyrazolo[3,4-b]pyridin-7-yl]-*N*-(4'-trifluoromethylbiphenyl-4-ylmethyl)acetamide bitartrate;
*N*-(1-(*t*-butoxycarbonyl)piperidin-4-yl)-2-[2-(2,3-difluorobenzylthio)-4-oxo-4*H*-[1,8]naphthyridin-1-yl]-*N*-(4'-trifluoromethylbiphenyl-4-ylmethyl)acetamide;
*N*-(1-ethylpiperidin-4-yl)-2-[6-(2-(2,3-difluorophenyl)ethyl)-2-(2-methoxyethyl)-4-oxo-4*H*-pyrazolo[3,4-b]pyridin-7-yl]-*N*-(4'-trifluoromethylbiphenyl-4-ylmethyl)acetamide bitartrate;
*N*-(2-diethylaminoethyl)-2-[4-oxo-2-(2-(2,3,4-trifluorophenyl)ethyl)-4*H*-quinolin-1-yl]-*N*-(4'-trifluoromethyl-biphenyl-4-ylmethyl)acetamide bitartrate;
*N*-(2-diethylaminoethyl)-2-[2-(2-(2,4-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2-(3-fluorophenyl)ethyl)-4-oxo-4*H*-quinolin-1-yl]-*N*-(4'-trifluoromethylbiphenyl-4-ylmethyl)acetamide bitartrate;
*N*-(piperidin-4-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(piperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(piperidin-4-yl)-2-[2-(2-(2,3-difluorophenyl)ethyl)-4-oxo-5,6-trimethylenepyridin-1-yl]-*N*-(4'-trifluoromethylbiphenyl-4-ylmethyl)acetamide bitartrate;
*N*-(piperidin-4-yl)-2-[2-(2-(2,3-difluorophenyl)ethyl)-4-oxo-4*H*-[1,8]naphthyridin-1-yl]-*N*-(4'-trifluoromethylbiphenyl-4-ylmethyl)acetamide bitartrate;
*N*-(piperidin-4-yl)-2-[2-(2,3-difluorobenzylthio)-4-oxo-4*H*-[1,8]naphthyridin-1-yl]-*N*-(4'-trifluoromethylbiphenyl-4-ylmethyl)acetamide trifluoroacetate;
*N*-(2-ethylaminoethyl)-2-[2-(2,3-difluorobenzylthio)-4-oxo-4*H*-quinolin-1-yl]-*N*-(4'-trifluoromethylbiphenyl-4-ylmethyl)acetamide;
*N*-(2-ethylaminoethyl)-2-[2-(2-(2,3-difluorophenyl)ethyl)-4-oxo-4*H*-quinolin-1-yl]-*N*-(4'-trifluoromethylbiphenyl-4-ylmethyl)acetamide;
*N*-(1-(2-hydroxyethyl)piperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate; or the free base thereof, or another pharmaceutically acceptable salt.

Methods for preparing these compounds are disclosed in the noted documents.

**Formula (IV)**

Also known are compounds of formula (IV)

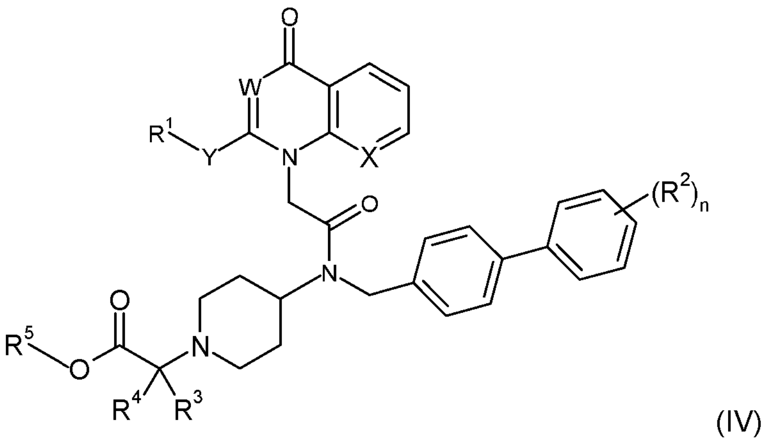

(IV)

wherein:

$R^1$ is an aryl group, unsubstituted or substituted by 1, 2, 3 or 4 substituents which can be the same or different selected from the group consisting of $C_1$-$C_6$ alkyl, $C_1$-$C_6$ alkoxy, $C_1$-$C_6$ alkylthio, aryl $C_1$-$C_6$ alkoxy, hydroxy, halo, CN, $COR^6$, $COOR^6$, $NR^6COR^7$, $CONR^8R^9$, $SO_2NR^8R^9$, $NR^6SO_2R^7$, $NR^8R^9$, halo $C_{1-}C_4$ alkyl, and halo $C_{1-}C_4$ alkoxy;
W is CH and X is N, or W is N and X is CH, W and X are both CH, or W and X are N;
Y is $C_2$-$C_4$alkyl,
$R^2$ is hydrogen, $C_1$-$C_6$ alkyl, $C_1$-$C_6$ alkoxy, $C_1$-$C_6$ alkylthio, aryl $C_1$-$C_6$ alkoxy, hydroxy, halo, CN, $COR^6$, carboxy, $COOR^6$, $NR^6COR^7$, $CONR^8R^9$, $SO_2NR^8R^9$, $NR^6SO_2R^7$, $NR^8R^9$, mono to perfluoro- $C_1$-$C_6$ alkyl, or mono to perfluoro- $C_1$-$C_6$ alkoxy;
n is 0-5;
$R^3$ is $C_1$-$C_4$ alkyl;
$R^4$ is $C_1$-$C_4$ alkyl;
$R^5$ is hydrogen, $C_1$-$C_{10}$ alkyl, $C_{2-}C_{10}$ alkenyl, $C_{2-}C_{10}$ alkynyl, halo $C_{1-}C_4$ alkyl, $C_3$-$C_8$ cycloalkyl, $C_3$-$C_8$ cycloalkyl, $C_3$-$C_8$ cycloalkyl $C_1$-$C_4$ alkyl, $C_5$-$C_8$cycloalkenyl, $C_5$-$C_8$cycloalkenyl $C_1$-$C_4$ alkyl, 3-8-membered heterocycloalkyl, 3-8-membered heterocycloalkyl $C_1$-$C_4$ alkyl, $C_6$-$C_{14}$ aryl, $C_6$-$C_{14}$ aryl $C_1$-$C_{10}$ alkyl, heteroaryl, or heteroaryl $C_{1-}C_{10}$alkyl; wherein each group is optionally one or more times by the same and/or a different group which is $C_1$-$C_6$ alkoxy, $C_1$-$C_6$ alkylthio, aryl $C_1$-$C_6$ alkoxy, hydroxy, halo, CN, $NR^8R^9$, or halo $C_1$-$C_4$ alkoxy
$R^6$ and $R^7$ are independently hydrogen or $C_{1-}C_{10}$ alkyl;
$R^8$ and $R^9$ are the same or different and are hydrogen or $C_1$-$C_{10}$ alkyl, or $R^9$ and $R^{10}$ together with the nitrogen to which they are attached form a 5- to 7 membered ring optionally containing one or more further heteroatoms selected from oxygen, nitrogen and sulphur, and optionally substituted by one or two substituents selected from the group consisting of hydroxy, oxo, $C_{1-}C_4$ alkyl, $C_{1-}C_4$ alkylcarboxy, aryl, and aryl $C_{1-}C_4$ alkyl; or a pharmaceutically acceptable salt thereof.

Without intending to exclude any defined substituents and/or their recited radicals from the scope of formula (IV), the following R groups and the associated radicals are of particular interest:

As regards $R^1$, it can be an phenyl group optionally substituted by 1, 2, 3 or 4 substituents which can be the same or different selected from halo, $C_1$-$C_6$ alkyl, trifluoromethyl or $C_1$-$C_6$ alkoxy. More specifically, phenyl is unsubstituted or substituted by 1, 2, 3 or 4 halogen substituents, particularly, from 1 to 3 fluoro groups, and most particularly, 2,3-difluoro, 2,4-difluoro or 4-fluoro.

A further embodiment of formula (I) is where Y is $-CH_2CH_2-$.

The invention also provides a compound of formula (I) in which $R^2$ is hydrogen, by default, or is halo, $C_1$-$C_6$ alkyl, mono to perfluoro- $C_1$-$C_4$ alkyl, mono to perfluoro $C_{1-}C4_6$ alkoxy, or $C_1$-$C_6$ alkoxy; particularly mono to perfluoro- $C_1$-$C_4$ alkyl, mono to perfluoro- $C_1$-$C_4$ alkoxy, or $C_1$-$C_6$ alkoxy. Of particular interest are the compounds where $R^2$ is other than hydrogen, n in $(R^2)_n$ is 1, 2, or 3, and the substitution pattern is meta and/or para, particularly para, i.e. a 4-position substituent. Exemplified compounds include those where $R^2$ is 4-trifluoromethyl or 4-trifluoromethoxy.

$R^3$ and $R^4$ can be the same or different and are methyl, ethyl, *n*-propyl, or *n*-butyl. Of particular interest are those compounds of formula (I) where $R^3$ and $R^4$ are the same and are methyl, or ethyl; methyl is of particular interest.

$R^5$ can be hydrogen, $C_{(1-6)}$ alkyl which is a straight chain, or branched. Of particular interest is methyl, ethyl, propyl, isopropyl, *n*-butyl, *sec*-butyl, *iso*-butyl, *t*-butyl, *n*-pentyl or *n*-hexyl.

Methods for preparing these compounds are disclosed in the noted documents.

Any of the compounds described herein above can be prepared in crystalline or non-crystalline form, and, if

crystalline, can be solvated, e.g. as the hydrate. This invention includes within its scope stoichiometric solvates (e.g., hydrates).

Certain of the compounds described herein can contain one or more chiral atoms, or can otherwise be capable of existing as two enantiomers. The compounds according to the invention and useful in the methods as described herein include mixtures of enantiomers as well as purified enantiomers or enantiomerically enriched mixtures. Also included within the scope of the invention are the individual isomers of these compounds. The different isomeric forms can be separated or resolved one from the other by conventional methods, or any given isomer can be obtained by conventional synthetic methods or by stereospecific or asymmetric syntheses.

**Syntheses of the Compounds of Formula (I), (II), (III) and (IV)**

Methods for preparing compounds of formula (I), (II) and (III) have been published in the patent literature. For example, methods for making formula (I) can be found in WO 01/60805 and WO03/016287. Methods for making compounds of formula (II) have been set out in WO 02/30911. Methods for making compounds of formula (III) can be found in WO 02/30904. Methods for preparing compounds of formula (IV) can be found in WO08/048866, and WO08/048867.

Some examples of syntheses are provided below. To differentiate between the several generic groups of compounds in the examples herein, materials relating to formula (I) will be labeled as " Example of Synthesis Approach (I)-1" et seq., for formula (II) "Example of Synthesis Approach (II)-1" et seq., for formula (III), "Example of Synthesis Approach (III)-1 et seq., and for formula (I), "Example of Synthesis Approach (IV)-1, et seq.

**Synthesis of Formula (I)**

Compounds of formulae (I) can be prepared by processes scheme I, as disclosed in WO 01/60805:

Scheme I

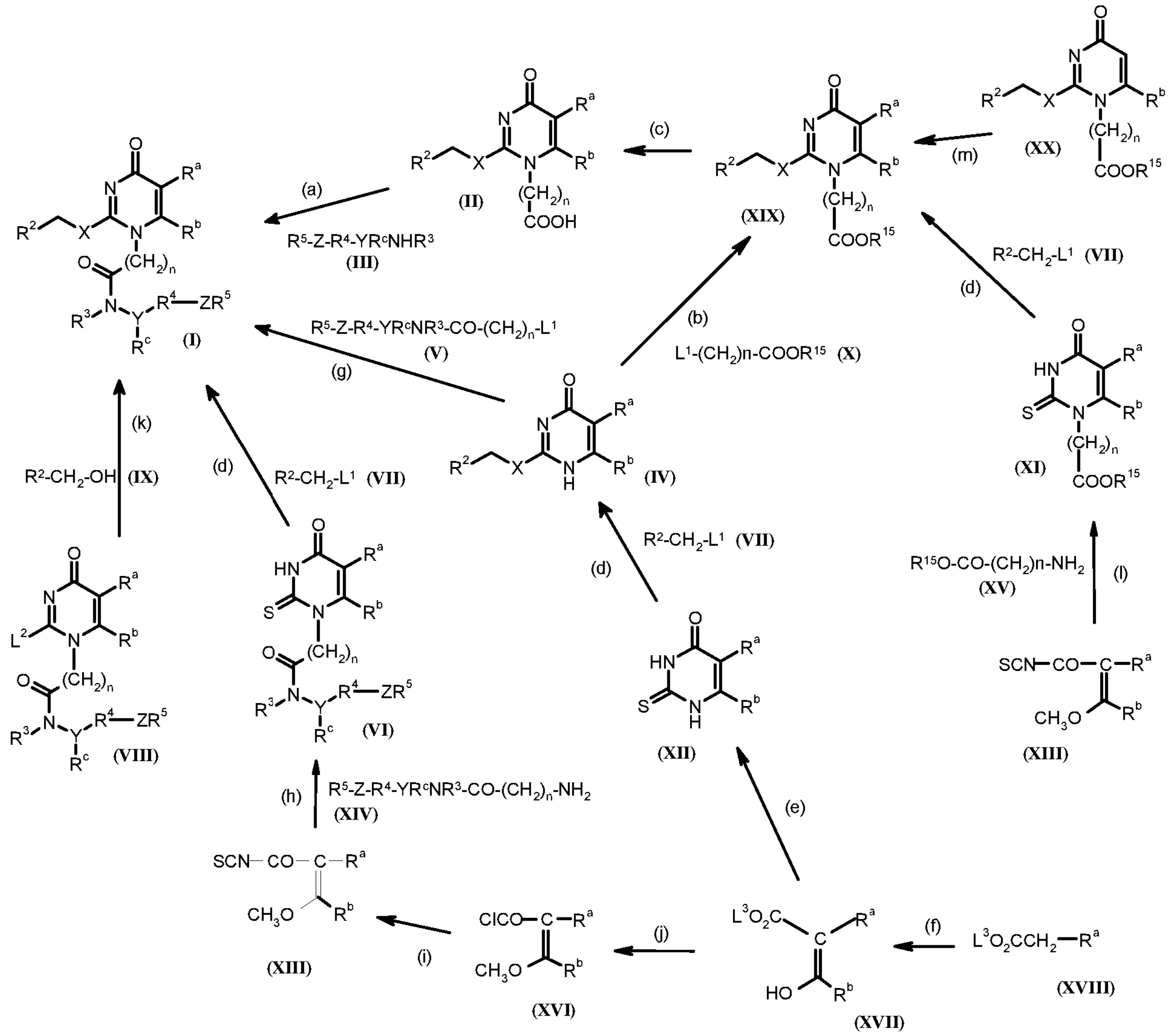


in which:

- $L^3$ is a C(1-6)alkyl group, for instance methyl;
- $R^{15}$ is a $C_{(1-6)}$alkyl group, for instance ethyl or t-butyl and
- $L^1$, $L^2$, $R^a$, $R^b$, $R^c$, $R^2$, $R^3$, $R^4$, $R^5$, n, X, Y and Z are as defined in WO 01/60805.

An exemplary reaction for making a compound of formula (I) of interest has been described in the art, for example, WO 01/60805, and is set forth below.

*Example of Synthesis Approach (I)-1(a)*

1-(N-(2-(Diethylamino)ethyl)-N-(4-(4-trifluoromethylphenyl)benzyl)aminocarbonyl-methyl)-2-(4-fluorobenzyl)thio-5,6-trimethylenepyrimidin-4-one

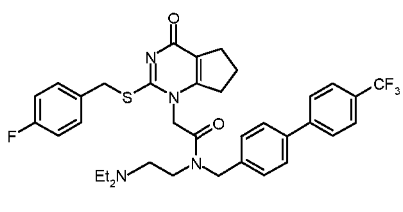

As disclosed in WO 01/60805, intermediate B69 of WO 01/60805 (87.1g, 0.26 mol.) was suspended in dichloromethane (2.9 liter). 1-Hydroxybenzotriazole hydrate (35.2g, 0.26 mol.) and 1-(3-dimethylaminopropyl)-3-ethylcarbodiimide hydrochloride (99.7g, 0.52 mol.) were added and the suspension stirred for 45 minutes by which time complete solution had been obtained. Intermediate A30 of WO 01/60805 (91.2g, 0.26 mol.) was added as a solution in dichloromethane (100ml) over 5 minutes and the solution stirred for 4 hours. Saturated ammonium chloride solution:water mixture (1:1, 1 liter) was added and the solution stirred for 10 minutes. The organic phase was separated and extracted with saturated ammonium chloride:water mixture (1:1, 1 liter), extracts were pH 6. The organic phase was separated and extracted with water (1 liter) containing acetic acid (10ml), extract pH 5. The dichloromethane layer was separated and extracted with saturated sodium carbonate solution:water:saturated brine mixture (1:3:0.2, 1 liter), pH 10.5, then with saturated brine:water mixture (1:1, 1 liter). The brown solution was dried over anhydrous sodium sulfate in the presence of decolorizing charcoal (35g), filtered and the solvent removed *in vacuo* to give a dark brown foam. The foam was dissolved in *iso*-propyl acetate (100ml) and the solvent removed *in vacuo.* The dark brown gummy residue was dissolved in boiling *iso*-propyl acetate (500ml), cooled to room temperature, seeded and stirred overnight. The pale cream solid produced was filtered off and washed with iso-propyl acetate (100ml). The solid was sucked dry in the sinter for 1 hour then recrystallized from *iso*-propyl acetate (400ml). After stirring overnight the solid formed was filtered off, washed with iso-propyl acetate (80ml) and dried *in vacuo* to give the title compound, 110g, 63.5% yield. 1H NMR ($CDCl_3$, ca 1.9:1 rotamer mixture) $\delta$ 0.99 (6H, t), 2.10 (2H, m), 2.50 (4H, q), 2.58/2.62 (2H, 2 x t), 2.70/2.82 (2H, 2 x t), 2.86 (2H, t), 3.28/3.58 (2H, 2 x t), 4.45/4.52 (2H, 2 x s), 4.68/4.70 (2H, 2 x s), 4.93 (2H, s), 6.95 (2H, m), 7.31 (2H, d), 7.31/7.37 (2H, 2 x m), 7.48/7.52 (2H, d), 7.65 (2H, m), 7.72 (2H, m); MS (APCI) $(M+H)^+$ 667; mp 125❑C (by DSC - asymmetric endotherm).

*Example of Synthesis Approach (I)-1(b)*

As disclosed in WO 01/60805, 1-(N-(2-(Diethylamino)ethyl)-N-(4-(4-trifluoromethylphenyl)benzyl)aminocarbonylmethyl)-2-(4-fluorobenzyl)thio-5,6-trimethylenepyrimidin-4-one bitartrate

Prepared from intermediates A30 and B69 in WO 01/60805 by the method of Example 1 in WO 01/60805. $^1$H-NMR ($d_6$-DMSO, ca 1:1 rotamer mixture) $\delta$ 0.92/0.99 (6H,2x t), 1.99 (2H,m), 2.54 (6H,m), 2.68/2.74 (4H,m), 3.36 (2H,m), 4.21 (2H,s), 4.37/4.44 (2H,2x s), 4,63/4.74 (2H,2x s), 4,89/5.13 (2H,2x s), 7.08/7.14 (2H,2x m), 7.36-7.50 (4H, m), 7.64/7.70 (2H,2x d), 7.83 (4H,m); MS (APCI+) found (M+1) = 667; $C_{36}H_{38}F_4N_4O_2S$ requires 666.

*Example of Synthesis Approach (I)-1(c)*

1-(N-(2-(Diethylamino)ethyl)-N-(4-(4-trifluoromethylphenyl)benzyl)aminocarbonyl-methyl)-2-(4-fluorobenzyl)thio-5,6-trimethylenepyrimidin-4-one hydrochloride

As disclosed in WO 01/60805, the free base from Example (I)-1(a) (3.00g, 0.0045 mol) was suspended with stirring in isopropanol (30 ml) and warmed to 45°C to give a clear solution. The solution was then cooled to ambient temperature and conc. hydrochloric acid (0.40 ml, 0.045 mol) was added. The resultant slurry was then stirred at ambient temperature for 35 minutes, before being cooled to 0°C for 35 minutes. The slurry was then filtered and washed with isopropanol (10 ml), followed by heptane (30 ml), before being dried under vacuum to give the title compound as a white solid (3.00 g, 95%). $^1$H NMR ($CDCl_3$) $\delta$ .38 (6H, t), 2.08 (2H, m), 2.82 (2H, t), 2.99 (2H, t), 3.19 (4H, m), 3.35 (2H, m), 3.97 (2H, s), 4.42 (2H, s), 4.81 (2H, s), 4.99 (2H, s), 6.87 (2H, t), 7.26 (2H, t), 7.33 (2H, d), 7.41 (2H, d), 7.53 (2H, d), 7.71 (2H, d), 11.91 (1H, s).

**Synthesis of Formula (II)**

A description of how to make the compounds of formula (II) and examples of intermediates and final products for the compounds named above can be found in published international application WO 02/3091 1. A last-step method for making a compound useful in this invention is Example (II)-1.

*Example of Synthesis Approach (II)-1*

*N*-(2-Diethylaminoethyl)-2-[2-(2-(2,3-difluorophenyl)ethyl)-4-oxo-4,5,6,7-tetrahydro-cyclopentapyrimidin-1-yl]-*N*-(4'-trifluoromethyl-biphenyl-4-ylmethyl)acetamide bitartrate

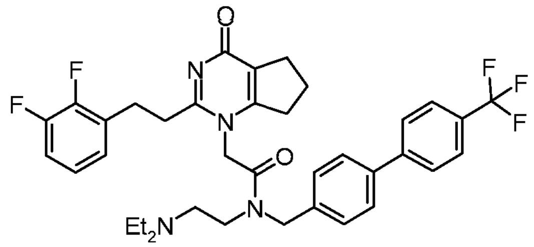

As disclosed in WO 02/30911, a solution of *N,N*-diethyl-*N'*-(4'-trifluoromethyl-biphenyl-4-ylmethyl)-ethane-1,2-diamine (Int D4 in WO 02/30911) (0.50g, 1.44mmol), 1-(3-dimethylaminopropyl)-3-ethylcarbodiimide (0.56g, 1.45mmol), 1-hydroxybenzotriazole hydrate (0.12g) and 2-(2-[2-(2,3-difluorophenyl)-ethyl]-4-oxo-4,5,6,7-tetrahydro-cyclopentapyrimidin-1-yl)-acetic acid (Int C1 in WO 02/30911) (0.48g, 1.44mmol) in dichloromethane (10ml) was stirred at ambient temperature overnight then diluted with dichloromethane (30ml), washed with aqueous sodium bicarbonate and evaporated. The residue was purified by chromatography (10g silica cartridge, ethyl acetate-acetone) to give the title compound as a yellow foam (free base) (0.50g, 52%). $^1$H-NMR (DMSO, rotamer mixture) δ 0.83-0.89 (6H, m), 1.98 (2H, m), 2.40 (4H, m), 2.45-2.82 (10H, m), 3.02 (2H, m), 4.64/4.75 (2H,2x s), 4.96/5.19 (2H,2x s), 7.11-7.40 (5H, m), 7.65 (2H, m), 7.84 (4H, m); MS (APCI+) found (M+1) = 667; $_{37}H_{39}F_5N_4O_2$ requires 666.

As disclosed in WO 02/30911, *d*-Tartaric acid (0.09g, 0.60mmol) was added to a solution of the free base (0.40g, 0.60mmol) in methanol (10ml) with stirring. The resulting solution was evaporated to yield the salt (0.49g). $^1$H-NMR (DMSO, rotamer mixture) □ 0.85-0.97 (6H, m), 1.91-2.00 (2H, m), 2.40-2.49 (4H, m), 2.54-2.82 (10H, m), 3.02-3.46 (2H, m), 4.20 (2H, s), 4.64/4.75 (2H, 2x s), 4.97/5.18 (2H, 2x s), 7.11-7.40 (5H, m), 7.65 (2H, m), 7.84 (4H, m); MS (APCI+) found (M+1) = 667; $C_{37}H_{39}F_5N_4O_2$ requires 666.

Following this process, or alternatively other processes described in WO 02/30911, one can prepare the other compounds named above that have the structure of formula (II).

As disclosed in WO 02/30911, *N*-(1-ethylpiperidin-4-yl)-2-(2-(2-(2,3-difluorophenyl)-ethyl)-4-oxo-4*H*- quninazolin-1-yl)-*N*-(4'-chloro-biphenyl-4-ylmethyl)acetamide bitartrate (Example 135) was prepared by reacting Intermediate C43 with Intermediate D82, according to the disclosure in WO 02/30911; US patent 7,169,924:

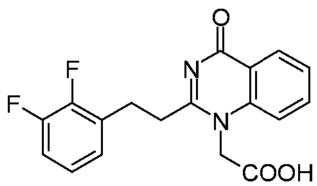

Intermediate C43:

Intermediate C43: 2-(2-(2-(2,3-Difluorophenyl)-ethyl)-4-oxo-4*H*-quinazolin-1-yl)acetic acid: As disclosed in WO 02/30911, a solution of 2-(2-(2-(2,3-difluorophenyl)-ethyl)-4-oxo-4*H*-quinazolin-1-yl)-acetic acid ethyl ester (B65) (6.8g, 18.3mmol) in methanol (30ml) and 2M sodium hydroxide solution (18.0ml, 36mmol) was stirred at ambient temperature overnight. The solvent was removed *in vacuo* and the residue dissolved in water (10ml). Acidification to pH 1 with 2M hydrochloric acid gave a solid that was filtered, washed with water and dried *in vacuo* to give the desired product (5.9g, 94%) as a white solid. $^1$H-NMR (DMSO) δ 3.11-3.30 (4H, m), 5.31 (2H, s), 7.16-7.33 (3H, m), 7.61 (1H, t), 7.68 (1H, d), 7.89 (1H, t), 8.18 (1H, d); MS (APCI+) found (M+1) = 345; $C_{18}H_{14}F_2N_2O_3$ requires 344.

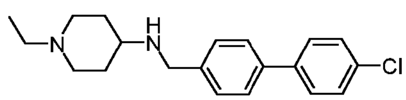

Intermediate D82:

Intermediate D82: *N*-(1-ethylpiperidin-4-yl)-4-(chlorophenyl)-benzylamine: Intermediate D82 was made by the method of Intermediate D8 as disclosed in WO 02/30911; US patent 7,169,924: Piperidone precursors were either commercially available, or readily prepared from commercially available materials by literature methods or minor modifications thereof.

**Synthesis of Formula (III)**

The overall synthesis of compounds of formula (III) is illustrated in the following scheme III, as presented in WO02/30904:

Scheme III

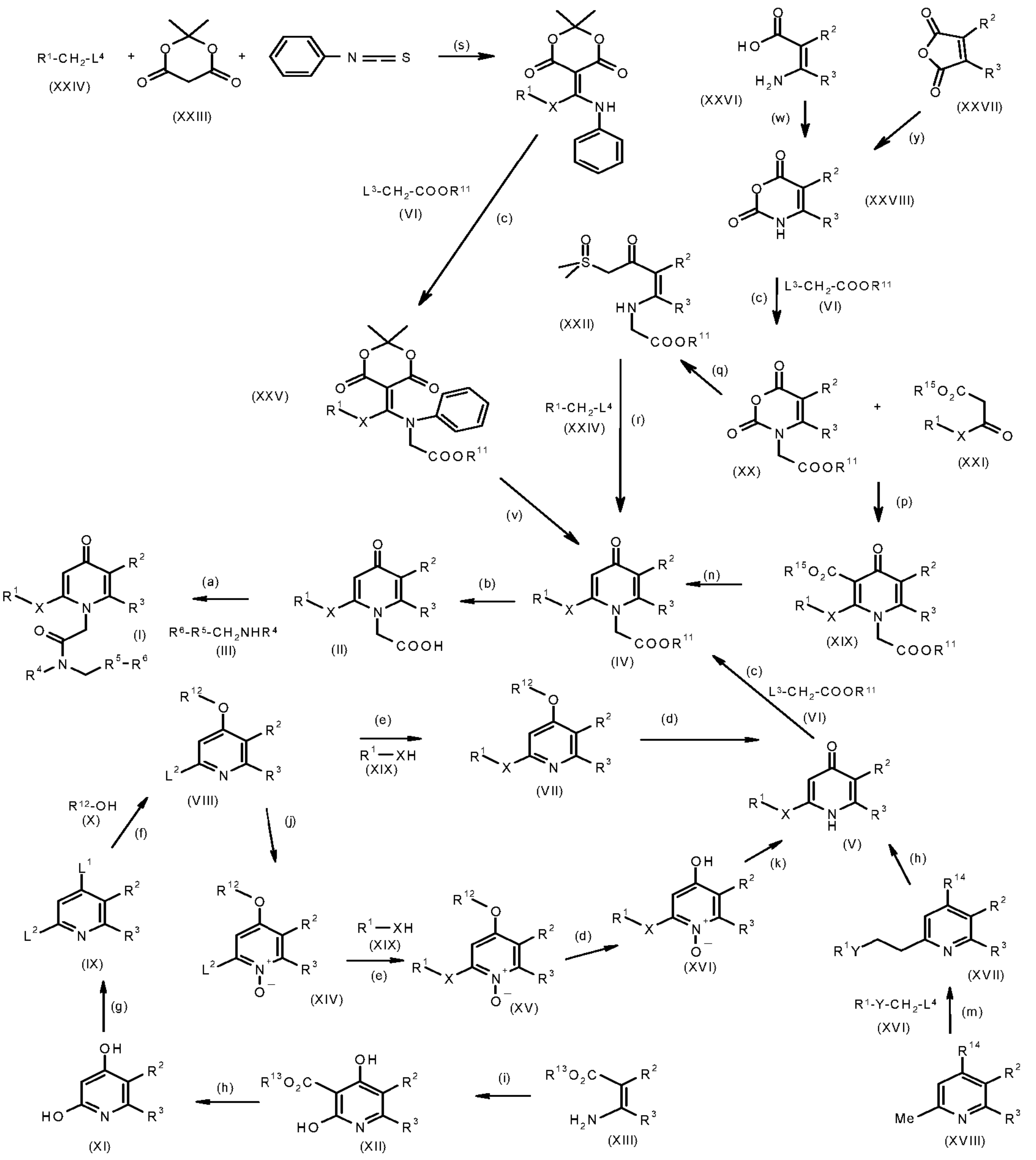


Referring to this scheme, the ester (IV) can be prepared by N-1 alkylation of (V) using (VI), in which $R^{11}$ is as hereinbefore defined e.g. (VI) is t-butyl bromoacetate or ethyl bromoacetate, in the presence of a base e.g. BuLi in THF or sodium hydride in N-methyl pyrrolidinone (NMP) (step c).

When X is $CH_2S$, the key intermediate (IV) can be synthesized by reacting (XX) with dimethyloxosulfonium methylide, generated via the treatment of trimethylsulfoxonium iodide with sodium hydride at low temperature, to yield a sulfur ylid (XXII) (step q). Subsequent treatment of (XXII) with carbon disulfide in the presence of diisopropylamine, followed by $R^1CH_2$-$L^4$, where $L^4$ is a leaving group, yields intermediate (IV) (step r).

Alternatively, when X is $CH_2S$, the $R^1X$ substituent can be introduced by displacement of a leaving group $L^2$ (e.g. Cl) (step e) either on a pyridine (VIII) or pyridine N-oxide (XIV), to give 2-substituted pyridines (VII) and (XV). Transformation of (VII) or (XV) to the 4-pyridone (V) is accomplished by deprotection of the 4-oxygen (e.g. using $(Ph_3P)_3RhCl$ when in aq. ethanol when $R^{12}$ = allyl) (step d), followed, for (XVI), by removal of the N-oxide substituent,

using hydrogen in the presence of Pd/C in acetic acid (step k). The pyridine (VIII) or pyridine N-oxide (XIV) can be prepared by steps (i), (h), (g), (f), and (j), in which:

(j) treatment of (VIII) with m-chloroperbenzoic acid in dichloromethane;
(f) treatment of (IX) with $R^{12}OH$ (X), in which $R^{12}$ is allyl, and sodium hydride in DMF;
(g) treatment of (XI) with phosphorus oxychloride;
(h) treatment of (XII) with aq HCl with heating;
(i) treatment of (XIII) with di-lower alkyl malonate and sodium alkoxide in alcohol (in which $R^{13}$ is $C_{(1-6)}$alkyl, typically $R^{13}$ = Et); and

$R^1$- $CH_2SH$ (XIX) is typically prepared from the thioacetate, which is formed from the corresponding alkyl bromide $R^1$-$CH_2Br$.

Alternatively, when X is $CH_2S$ and $R^2$ and $R^3$, together with the pyridone ring carbon atoms to which they are attached, can form a fused benzo ring, intermediate (IV) can be synthesized from known starting materials by steps (s), (c) and (v) in which:

(s) treatment of Meldrum's acid (XXIII) with sodium hydride at low temperature, followed by reaction with phenylisothiocyanate and subsequent treatment with $R^1$ $CH_2$-$L^4$;
(c) as hereinbefore discussed;
(v) treatment of (XXV) with trifluoroacetic acid.

When X is alkylene, it is preferable to use steps (m) and (h) (intermediates (XVII), (XVIII)) or steps (n) and (p) (intermediates (XIX), (XX), (XXI)) in which:

(h) transformation of a 4-substituted pyridine into a 4-pyridone e.g. by treatment of (XVII) $R^{14}$ = Cl with aq HCl and dioxan, or deprotection of $R^{14}$ = $OR^{12}$, e.g. using conditions of step (d).
(m) chain extension of a 2-alkyl pyridine, e.g. where X = $YCH_2CH_2$ by treatment of a 2-methylpyridine (XVIII) with $R^1$-Y-$CH_2$-$L^4$ (XVI) in which $L^4$ is a leaving group and a strong base, such as BuLi, in THF.

In the alternative route, the 3-ester group is removed from intermediate (XIX) $R^{15}$ = $C_{(1-6)}$alkyl by heating in diphenyl ether where $R^{15}$ = tBu (step n); Intermediate (XIX) is formed from the 2,6-dioxo-1,3-oxazine (XX) and ester (XXI) by treatment with a base such as NaH in DMF or 1,8-diazabicyclo[5.4.0]undec-7-ene in dichloromethane.

Synthesis of (XX) from known starting materials can be achieved via steps (w) and (c) or steps (y) and (c) in which:

(w) treatment of (XXVII) with azidotrimethylsilane in THF;
(y) treatment of (XXVI) with phosgene;
(c) as hereinbefore described.

See WO02/30904, for additional details and exposition of how to make compounds of formula (III).

*Example of Synthesis Approach (III)-1*

*N*-(1-(2-Methoxyethyl)piperidin-4-yl)-2-[2-(2,3-difluorobenzylthio)-4-oxo-4*H*-quinolin-1-yl]-*N*-(4'-trifluoromethylbiphenyl-4-ylmethyl)acetamide

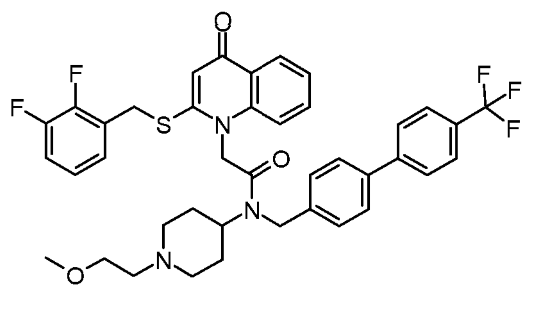

As disclosed in WO02/30904, the free base was prepared from Int. E1 and Int. A42 by the method of Example 1, except using DMF as solvent in place of dichloromethane. 1.97g of this material was crystallized from n.butyl acetate (10ml) to give the title compound (1.35g). $^1$H-NMR ($CD_3OD$) $\delta$ 1.7-2.05 (4H, m), 2.05-2.3 (2H, 2xt), 2.5-2.65 (2H, m), 2.95-3.1 (2H, m), 3.3 (3H, s), 3.45-3.55 (2H, m), 3.9-4.05 + 4.4-4.5 (1H, 2xm), 4.37 + 4.48 (2H, 2xs), 4.71 + 4.87 (2H, 2xbr s), 5.31 + 5.68 (2H, 2xs), 6.44 + 6.52 (1H, 2xs), 6.95-7.3 (3H, m), 7.35-7.85 (11H, m), 8.2-8.35 (1H, m); MS (APCI+) found (M+1) 736; $C_{40}H_{38}F_5N_3O_3S$ requires 735.

**Synthesis of Formula (IV)**

The following flow chart illustrates a process for making the compounds of this invention.

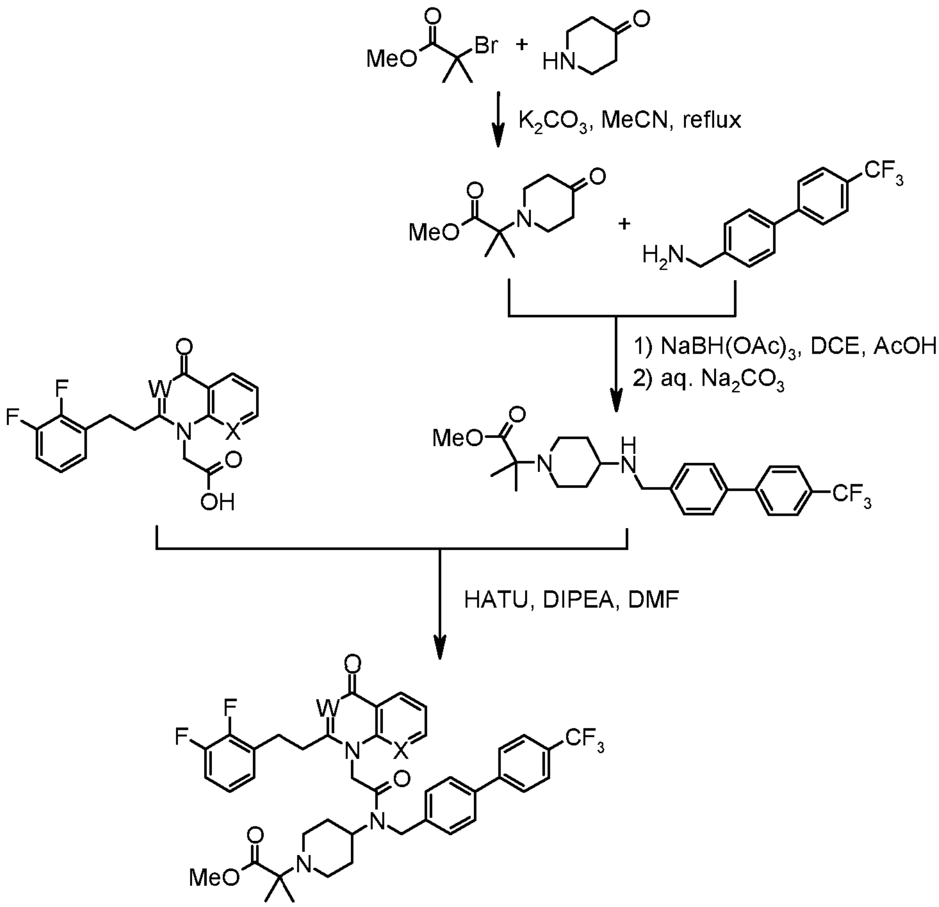


In addition, the reader is referred to published PCT application WO 03/016287 for chemistries that can be useful in preparing some of the intermediates set out in this flow chart. Those chemistries, to the extent they are useful in this case, are incorporated herein by reference as though it was fully set out herein. In addition, reference is made to the syntheses set out in published PCT applications WO 01/60805, WO 02/30911, WO 02/30904, WO 03/042218, WO 03/042206, WO 03/041712, WO 03/086400, and WO 03/87088, US 2008/0090851, US 2008/0090852, US applications US 2008/0090853 and US 2008/0103156. To the extent the reader wishes to prepare the compounds of formula (IV) by using intermediates, reagents, solvents, times, temperatures, etc., other than those in the route on the foregoing page, these publications can provide useful guidance.

**Bioassay for Identifying Lp-$PLA_2$ Inhibitors:**

Screen for inhibition of Lp-$PLA_2$ protein

In some embodiments, the methods of the present invention relate to use of inhibitors of Lp-$PLA_2$ for the treatment of eye diseases, that is macular edema of any cause, e.g., due to RVO, inflammation, post-surgical, traction, and the like; age-related macular degeneration (AMD); uveitis; diabetic eye diseases and disorders; diabetic retinopathy, and the like. Where necessary, agents that inhibit Lp-$PLA_2$ protein are assessed using a bioassay, for example, as

disclosed in U.S. Patent 5,981,252

The compounds as disclosed herein for example as disclosed in the sections entitled of Examples of Synthesis, were tested and were found to have $IC_{50}$ values in the range 0.1 to 10 nM.

## EXAMPLES

The examples presented herein relate to the methods and compositions for the prevention and/or treatment of eye diseases, being macular edema of any cause, e.g., due to RVO, inflammation, post-surgical, traction, and the like; age-related macular degeneration (AMD); uveitis; diabetic eye diseases and disorders; diabetic retinopathy, and the like. by inhibition of Lp-$PLA_2$.

In some embodiments, agents inhibiting Lp-$PLA_2$ can be assessed in animal models disclosed herein for effect in reducing CNS vascular permeability.

In some embodiments, agents inhibiting Lp-$PLA_2$ can be assessed in animal models, for example, STZ- induced diabetic rats, disclosed herein.

In some embodiments, agents inhibiting Lp-$PLA_2$ can be assessed in animal models showing increased blood/retinal barrier permeability induced by a combination of diabetes and hypercholesterolemia.

### Example 1

### Effect of Lp-$PLA_2$ inhibitors in reducing CNS vascular permeability

### Lp-PLA2 inhibitors in Diabetic (DM)/hypercholesterolemic (HC) pigs

### Methods:

#### Induction of Diabetes in Pigs

Yorkshire domestic farm pigs (-25-35 kg; Archer Farms) were induced for diabetes with a single intravenous injection of 125 mg/kg streptozotocin (Mohler et al., 2008). Blood glucose and cholesterol were closely monitored. Three days after diabetes induction, a hyperlipidemic diet was used to achieve hypercholesterolemia with serum cholesterol concentrations clamped between 400 and 800 mg/dl. Because pigs have a variable serum cholesterol response to a high cholesterol diet, cholesterol concentrations were checked every two months. One month after DMHC induction, pigs were randomly assigned into either a control group or a treatment group receiving 10 mg/kg/d orally of the selective Lp-$PLA_2$ inhibitor Darapladib (GlaxoSmithKline). Pigs were sacrificed after 28 weeks after induction (that is, 24 weeks after initiation of treatment). At the time of completion of the study, there were 17 pigs in the control group and 20 in the Darapladib-treated group, as three pigs in the control group were excluded from analysis because of persistently elevated cholesterol levels. Three pigs did not undergo DM-HC induction and acted as age-matched controls. The specific experiment was published (Wilensky et al., 2008) but no vascular leak data was presented in that publication.

#### Preparation of pig brain tissues for morphological and quantitative Immunohistochemical analyses.

Specimens from the brains of 29 pigs, included 3 untreated controls; 13 DMHC and 13 DMHC treated with Darapladib (Rx), were embedded in paraffin wax. Each brain specimen was serially sectioned, and the first section representing each sample was stained with Hematoxylin and Eosin (H+E) which permitted cortical from non-cortical regions based on differential staining to be determined. Full (large) face tissue blocks were used throughout this study in an effort to maximize the amount of tissue analyzed so as to favor subsequent quantification. The total areas (in sq. mm) of cortical and non-cortical brain tissue in each section were measured in H + E- stained sections using computer-assisted image analysis and Image Pro Plus software.

#### Immunohistochemical detection of the leak of immunoglobulin G (IgG) from brain blood vessels as an indicator of BBB integrity and permeability.

Sections representing each specimen block were immunostained with antibodies specific for pig immunoglobulin G (IgG) in order to locate any nonvascular IgG in the brain interstitial space or associated with neurons or astrocytes. In normal brains with a presumably intact BBB, IgG is efficiently excluded from the brain parenchyma (especially in the cerebral cortex) by virtue of the integrity of the BBB. Based on this, the presence of interstitial IgG, perivascular leak clouds containing IgG and IgG associated with neurons and/or astrocytes were interpreted as evidence of a local plasma leak and increased permeability or breakdown of the BBB. Immunohistochemistry for paraffin-embedded tissues was

carried out as previously described (D'Andrea et al., 2001; Nagele et al., 2002). In brief, brain tissue sections were deparaffinized using xylene, rehydrated through a graded series of decreasing concentrations of ethanol. Protein antigenicity was enhanced by microwaving sections in citrate buffer. Endogenous peroxidase was quenched by treating sections with 0.3% $H_2O_2$ for 30 min. Sections were first incubated in blocking serum and then treated with primary antibody (anti-swine Ig) at appropriate dilutions for 1 hr at room temperature. After a thorough rinse in PBS, biotin-labelled secondary antibody was applied for 30 min. Sections were treated with the avidin-peroxidase-labelled biotin complex (ABC, Vector Labs, Foster City, CA) and visualized by treating with 3-3-diaminobenzidine-4-HCL (DAB)/ $H_2O_2$ (Biomeda, Foster City, CA). Sections were then lightly counterstained with hematoxylin, dehydrated through increasing concentrations of ethanol, cleared in xylene and mounted in Permount. Controls consisted of brain sections treated with non-immune serum or omission of the primary antibody. Specimens were examined and photographed with a Nikon FXA microscope, and digital images were recorded using a Nikon DXM1200F digital camera and processed using Image Pro Plus (Phase 3 Imaging, Glen Mills, PA) image software.

**Quantitative analyses of the density of blood vessels showing IgG leak**

The density of vascular (BBB) leaks (= the number of leaks per unit area in sections of brain tissue) was calculated as follows: the total areas (in sq. mm) of cortical and non-cortical brain tissue in each H + E- stained section using computer-assisted image analysis and Image Pro Plus software. The H + E staining procedure was modified slightly so as to make it possible to readily distinguish cortex from non-cortex based on differences in color. The ability of this staining procedure to delineate cortex from non-cortex was confirmed by detailed microscopic examination. After area determinations for each section were completed, the next consecutive histological section was immunostained as described above with anti-swine IgG antibodies in order to detect the presence of any IgG in the brain tissue. An internal positive control in each section was provided by the blood vessels which, of course, contain intravascular IgG. Each immunostained section was evaluated by counting the total number of profiles of blood vessels appearing in the cortex and non-cortex that also displayed perivascular leak clouds. Only leak clouds containing blood vessel profiles were included in the count, despite the fact that many other leak clouds were also observed in which the source vessel was either above or below the plane of section. Blood vessels associated with each leak cloud were recorded as arterioles, venules or capillaries and were designated as either cortical or non-cortical. The density of leaks was calculated as the number of leaks per unit area (sq. mm.) of cortex or non-cortex.

**Quantitative analysis of the extent of vascular (BBB) leaks**

Individual leak clouds appearing in the brain sections were scored for the extent of the leak, specific region of the brain, the type of vessel (arteriole, venule or capillary), and its cortical or non-cortical location. It should be noted here that, although perivascular leak clouds appear as circles with the source blood vessel at or near the center, these clouds are actually cylindrical in three-dimensions. Because they are cylindrical, a doubling of the diameter of the leak cloud as seen in sections actually translates into a roughly four-fold increase in the volume (amount) of the leak cloud. Thus, the values used and presented here are underestimates of the real "3D" values. Lastly, the relative size of the leak cloud have been used as a measure of "amount of the leak" or extent of the leak.

**Quantitative analysis of the amount and distribution of amyloid beta1-42 (Abeta42) peptide throughout the cerebral cortex of the DMHC pig brain.**

Abeta42 was detected and quantified in histological sections of pig brain cerebral cortex using quantitative immunohistochemistry and antibodies specific for this peptide. Sections representing each specimen block were immunostained with antibodies specific for Abeta42 and examined and photographed with a Nikon FXA microscope, and images were recorded using a Nikon DXM1200F digital camera and analyzed using Image Pro Plus (Phase 3 Imaging, Glen Mills, PA) image software. At each of five randomly selected cortical locations within the tissue, four 20 x images were taken; two of the nearest cortical layers 2-3 regions and two at the corresponding cortical layers 4-6 regions. In this way, a maximum of twenty 20x images were obtained from each slide. For image analysis, an automated subprogram operating within the Image Pro Plus image analysis program was used. Control images showing little or no Abeta42 immunoreactivity were used to set the baseline threshold under conditions of identical light intensity, light filters, condenser and aperture settings and photoamplification by the digital camera. Total Abeta42 present in each 20x histological section was measured automatically and the data was downloaded into an Excel spreadsheet and analyzed.

**Observations**

**Effect of Lp-PLA2 inhibitor ’848 on vascular leak in Diabetic (DM)/hypercholesterolemic (HC) pigs.**

Pigs treated with pharmacological inhibitors of Lp-PLA2 showed that animals dosed with 10mg/kg/day Darapladib experienced a general wellness effect (increased activity and alertness) in comparison to DM/HC control animals (lethargic, generally unresponsive). Upon necropsy, the brains of SB480848-treated DM/HC animals, control DM/HC animals and non-diabetic/non-hypercholesterolemic animals were examined. In normal brains with an intact blood-brain barrier (BBB), IgG is efficiently excluded from the brain parenchyma (especially in the cerebral cortex) by virtue of the integrity of the BBB. Thus the presence of interstitial IgG, perivascular leak clouds containing IgG and IgG associated with neurons and/or astrocytes has been interpreted as evidence of a local plasma leak and increased permeability or breakdown of the BBB. Control DM/HC animals showed evidence of significant vascular leak from all types of blood vessels in the brain which may account for the notable behavioural aspects. This was evidenced by strong histochemical IgG immunoreactivity in brain parenchyma in the region of blood-vessels containing intravascular IgG. Darapladib was effective at reducing the extent of leaks in all vessel types within the brain microvasculature (see Figures 1 & 2).

The blood is the main source of Abeta42 peptide being 10 fold more concentrated in the serum than cerebrospinal fluid and is found to leak into the brain during periods of increased BBB permeability. Once in the brain the Abeta42 peptide can accumulate within specific neuronal cell types (Clifford et al., 2007). DM/HC pigs showed increased amyloid deposition selectively in pyramidal neurons of the cerebral cortex (and in all regions of brains tested) compared to control animals. Darapladib reduced amyloid deposition in DM/HC pig brains throughout all layers of the cortex when compared to untreated DM/HC pigs. The effect was determined to be due to a reduction in the number (density) of Abeta42 peptide positive neurones rather than the amount of Abeta42peptide associated with specific neurons which were modest (see Figures 3, 4 & 5).

Taken together, the IgG staining and Abeta42 peptide localization in the brain of DM/HC pigs demonstrate that luminal material from cerebral vessels is escaping from the brain vasculature into normal tissue parenchyma and that such vascular leak is attributable to the metabolic stress induced by hypercholesterolemia and diabetes. Treatment with Darapladib appears not only to induce behavioural changes in metabolically stressed animals but also appears to have a highly efficacious effect in reducing the permeability of the BBB which is induced by metabolic stress.

**Example 2**

**Effect of Lp-$PLA_2$ inhibitors in hypercholesterolemic copper-fed rabbits**

**Methods:**

**Induction of hypercholesterolemia in rabbits**

3-4 month SPF male New Zealand White rabbits were purchased from Covance (Denver, PA), individually housed and had ad libitum access to rabbit chow and water on a 12/12h light/dark cycle. Experiments were repeated twice with animals arriving 3 weeks apart. Rabbits were randomly assigned to five treatment groups with a sample size of 8 animals per group. 32 Rabbits were placed on cholesterol/copper diet and 8 rabbits fed normal chow. Cholesterol/copper diet consisted of 160g per day of commercially produced diet (Purina Mills High Fibre Diet) supplemented or not with 2% cholesterol. Copper sulphate (0.12mg/l) was added to drinking water of rabbits assigned the cholesterol supplemented diet.

**Animal dosing**

Animals were weighed and drug or vehicle administration delivered at 3ml/kg to affect a 10mg/kg dose by subcutaneous injection in the neck fold.

**Treatment groups**

Six groups of animals consisting of 8 animals were assigned to the following treatment groups. Groups 1-5 were maintained on the cholesterol supplemented diet with copper sulphate in the drinking water. *N*-(1-ethylpiperidin-4-yl)-2-(2-(2-(2,3-difluorophenyl)-ethyl)-4-oxo-4*H*- quninazolin-1-yl)-*N*-(4’-chloro-biphenyl-4-ylmethyl)acetamide bitartrate is an Lp-PLA2 inhibitor compound, i.e., Lp-PLA2 inhibitor ’859.

Group 1: Treated daily with vehicle from day 29-72

Group2: Treated with Lp-PLA2 inhibitor ’859 (10mg/kg) daily from day 29-72

Group 3: Treated daily with vehicle from day 1-72

Group 4: Treated with Lp-PLA2 inhibitor ’859 (10mg/kg) daily from day 1-72

Group 5: Animals on a normal diet receiving no treatment.

**Measurement of rabbit plasma Lp-PLA2 activity**

Lp-PLA2 activity was measured in plasma samples collected at days 1, 29, 32 and 72 using proprietary GSK techniques.

**Rabbit blood-brain barrier permeability**

Three rabbits in each of the five treatment groups for a total of 15 rabbits were injected with tracer for the assessment of blood brain barrier permeability. Changes in BBB permeability were assessed using the fluorescent tracer sodium fluorescein (NaF). The procedure performed was a modification of previously described methods (Lenzser et al., 2005; Phares et al., 2007; Morrey et al., 2008). Animals were anesthetized with isoflurane and injected i.v. with 5ml of 2% NaF in PBS which was allowed to circulate for 30 min. Animals were then transcardially perfused (45ml/min) with PBS for 5 min until colorless perfusate was observed. After perfusion animals were decapitated, brains removed, left and right hemispheres isolated. The left hemisphere was weighed and placed in 10 volumes of 50%w/v trichloroacetic acid, homogenized, centrifuged at 13,000g for 10 min and the supernatant neutralized with 5M sodium hydroxide. NaF quantitation was determined at excitation/emission wavelengths of 44/525nm. Fluorescent dye content was calculated by reference to standards with a range of 10-200ng/ml.

**Observations**

**Treatment of rabbits with Lp-PLA2 inhibitor ’859 on plasma Lp-PLA2.**

Lp-PLA2 activity was analyzed at 4 time points by repeated measures analysis of variance (ANOVA). The effect group was significant ($p<0.0001$). Post hoc analysis of the significant group effect indicated that rabbits treated with Lp-PLA2 inhibitor ’859 from the initiation of the high fat diet had significantly lower levels of Lp-PLA2 activity throughout the course of the 72 day time course whereas animals which had received treatment from day 29 showed a reduction in Lp-PLA2 activity at day 72 (see Figure 6)

**Blood-Brain Barrier Permeability**

There was a lower BBB permeability in the rabbits maintained on a normal diet whereas rabbits on a cholesterol and copper sulphate supplemented diet showed a profound increase in BBB permeability. A one-way analysis of variance comparing permeability in the 6 groups was statistically significant $F(5,16)=15.31$, $p<0.0001$. Dunnetts post-hoc tests indicated a significant difference between permeability in the normal diet animals and animals on the high cholesterol/$CuSO_4$ diet. A numerical decrease in permeability was observed in the Lp-PLA2 inhibitor ’859 treated animals in comparison to vehicle treated groups in the high cholesterol/ $CuSO_4$-fed animals. This was evident when the drug was dosed between days 29-72 and days 1-27 (see Figure 7). However, the sample sizes were too small for these differences to be statistically significant. One animal was omitted from the analysis due to poor perfusion.

**Example 3**

**Lp-PLA2 inhibitors in Streptozotocin (STZ)-induced diabetic rat**

STZ-induced diabetes is a commonly used model of type-1 diabetes and STZ-diabetic rats are the animal species most often used as preclinical models as their retinae exhibit most of the pathological features of background diabetic retinopathy seen in humans, including blood vessel dilation, basement membrane thickening, neuronal and glial dysfunction, and iBRB breakdown. This model of diabetic retinopathy has been widely used for assessing drug efficacy.

## Methods

### Animals, induction of diabetes and drug dosing

Male SD rats, 6 weeks of age and weighing 180-200g, were used in this study; the rats had free access to food and water and were maintained in cages in an environmentally controlled room with a 12 hour light- dark cycle. Diabetes was induced by following dosing of animals with streptozotocin (50mg/kg/day dosed i.p. for each of the first 3 days of the protocol) in 10mM sodium citrate-buffer pH 4.6. N-[2-(dimethylamino)ethyl]-2-[[(4-fluorophenyl)methyl]thio]-5-(1-methyl-1H-pyrazol-4-yl)methyl]-4-oxo-N-[[4'-(trifluoromethyl)[1,1'-biphenyl]-4-yl]methyl]-1(4H)-pyrimidineacetamide (Lp-PLA2 inhibitor '495) was solubilized in 10% Hydroxypropyl-beta-cyclodextrin or DMSO: 1% methylcellulose (1:99) and Lp-PLA2 inhibitor '859 in DMSO: 1% methylcellulose (1:99) and dosed by i.p. injection at 10mg/kg/day from day 4 to day 31 and day 4 to day 18 respectively. Animals which maintained an elevated blood glucose level of over 9mM were deemed diabetic.

### Determination of blood glucose in SD rats

Between 25 and 40 ul blood was drawn from the tail vein after overnight fasting and blood glucose level determined using a Johnson (Lifescan) OneTouch UltraEasy Blood Glucose Monitoring System and was operated according to the manufacturer's instructions. Blood glucose levels were determined both prior to, and after, administration of streptozotocin.

### Determination of plasma Lp-PLA2 activity in SD rats

Lp-PLA2 activity was determined in serum samples using the commercial Lp-PLA2 assay kit AZWell auto-PAF-AH assay (Cosmo Bio Co Ltd, Japan, www.cosmobio.co.jp) and procedures carried out according to manufacturer's instructions.

### Experimental Optical Coherence Tomography (OCT system)

The retinal images were scanned by a commercially available Fourier-domain OCT (RTVue-100; version 2.1; Optovue, Inc., California, USA). Super-luminescent diode light source with a central wavelength of 840 nm and a full bandwidth of 50 nm was adapted to the OCT. The retinal thickness was accessed by the automatic software of RTVue scanner with manual adjusting. The retinal thickness was defined from layer of retinal pigment eitheluim (RPE) to retinal ganglion cell layer (GCL).

### Procedure of Optical Coherence Tomography (OCT) imaging and determination of retinal thickness and retinal fluid exudation

Two separate studies were conducted. Retinal images were taken on experimental day 4, 10, 13, 17, 22, 28 and 31 for the second study and day 0, 4, 7, 10, 14 and 18 for the first study. Animals were anesthetized by inhalation of 5% isoflourane and pupils were diluted with 1% tropicamide and 1% phenylephrine. Prior to OCT analysis animals were fixed on a support frame and the eye to be imaged positioned to align with the center of the scan beam perpendicular to the fundus. Line scan pattern (1024 axial scans) with a 2mm scan length was used to generate the images. After imaging, the animals were recovered and returned to cages. Retinal thickness from six time points of OCT measurement were analyzed by repeated measures analysis of variance (ANOVA) using Prism software. When ANOVA showed significant main effect, student t-test was further used to reveal differences between compound treatment groups with vehicle controls. All data were expressed as mean value $\pm$ SEM.

### Determination of retinal vascular permeability using Evans blue

This methodology was performed according to previously published methods (Xu at el., 2001) with the exception that LC-MS/MS was used to determine Evans blue present in retinae from experimental animals. Animals were anesthetized with 30mg/kg avertin, skin and muscle of the abdomen was incised, the iliac artery and vein were carefully exposed and were cannulated with 0.28- and 0.58-mm internal diameter polyethylene tubing respectively, and filled with heparinised saline. Evans-blue (30mg/ml in saline) was injected through the iliac vein over 10s at a dosage of 45mg/kg. Two minutes after injection of Evans-blue, 50ul blood was drawn from the iliac artery to obtain the initial Evans-blue plasma concentration. Subsequently, at 30 minute intervals, 50ul blood was drawn from the iliac artery up to 2 hours after injection to obtain the time-averaged Evans-blue plasma concentration. At exactly 2 hours after infusion, 50ul blood

was drawn from the left ventricle to obtain the final Evans-blue plasma concentration. Results are expressed in micrograms of Evans blue per microliter of plasma. Immediately after perfusion, both eyes were enucleated and bisected at the equator. The retinae were then carefully dissected away under an operating microscope. Once dissected, retinal samples were dried in a speed-vac and then weighed before addition of methanol (20μl/mg tissue). Tissue was subsequently homogenized and protein precipitated by centrifugation at 12000 rpm for 10 min. 30 μl of supernatant was diluted with 30 μl of $H_2O$ and 6 μl of internal standard solution (2,3-Dihydro-5-methyl-n-[6-[(2-methyl-3-pyridinyl)oxy]-3-pyridinyl]-6-(trifluoromethyl)-1H-indole-1-carboxamide, disclosed in WO 97/48699, 20ng/ml in $H_2O$). After further vortexing and centrifugation at 4000rmp for 5min, 10 μl of supernatant was injected into LC-MS/MS system to determinate the concentration of Evans blue in retinal samples. The mobile phase of the LC method was (A) 1mM $CH_3COONH_4$-$CH_3CN$ (9/1, v/v) and (B) $CH_3CN$-$CH_3OH$ (4/1, v/v). The gradient was run as defined in Table 1.

**Table 1:** LC parameters for Evans blue determination in retinal tissues

| Time (min) | A% | B% |
|---|---|---|
| Initial | 95 | 5 |
| 0.50 | 95 | 5 |
| 1.80 | 10 | 90 |
| 2.50 | 10 | 90 |
| 2.60 | 95 | 5 |
| 3.00 | 95 | 5 |

**Table 2:** MS parameters for LC-MS/MS determination of Evans blue in retinal tissues are shown in Tables 2A and 2B

**Table 2A**

| **Ionization Mode** | **IS** | **CUR** | **GS1** | **GS2** | **TEM** | **CAD** | **MS Duration** |
|---|---|---|---|---|---|---|---|
| ESI, Negative | -4400 | 30 | 50 | 70 | 650 | high | 0.50-3.0 min |

**Table 2B**

| **Name** | **MRM** | **DP** | **CE** | **CXP** | **Dwell Time** (ms) |
|---|---|---|---|---|---|
| Internal Standard | 427.1/200.0 | -75 | -26 | -14 | 50 |
| Evans Blue | 217.2/249.1 | -40 | -20 | -12 | 70 |

The dry weight of retinas and the Evans-blue dye leakage into the retinas were assayed.

Blood-retinal barrier breakdown were calculated using the following equation:

$$\frac{\text{Evans blue (ug)/retina dry weight (g)}}{\text{Time averaged Evans blue concentration (ug)/plasma (ul) X circulation time (h)}}$$

**Isolation of rat brain microvascular endothelial cells (BMEC)**

Three-week-old Sprague-Dawley (SD) rats were sacrificed by cervical dislocation. After white matter, meninges and macroscopic pial vessels were removed, the gray matter was thoroughly triturated in ice-cold Dulbecco's modified Eagle's medium (DMEM). The tissue was then digested in DMEM (7 ml for 5 rat brains) containing collagenease II (1 mg/ml) and DNase I (39 U/ml) at 37°C for 1 h with shaking speed of 130 rpm. The digested tissue was diluted with the same volume of DMEM and centrifuged at 1000 g for 8 min at 4°C. The cell pellet was re-suspended in a 20% bovine serum albumin (BSA)-DMEM solution and centrifuged at 1000 g for 20 min at 4°C. The microvessels obtained in the pellet were further digested with collagenase-dispase (1 mg/mL) and DNase I (39 U/ml) in DMEM (3 ml for 5 rat brains) for 0.5 h at 37°C. The digested microvessel solution was diluted with the same volume of DMEM and centrifuged at 700

g for 6 min at 4°C. The pellet was re-suspended and layered over a 33% continuous Percoll gradient and centrifuged at 1000 g for 10 min at 4°C. The microvessel layer was collected and washed twice with the same volume of DMEM. The resulting microvessel fragments were plated at a density of $6\times10^5$ cells/$cm^2$ onto ECM-collagen-coated Transwell inserts (0.33 $cm^2$) in supplemented endothelial basal medium (EBM®-2 containing hydrocortisone, hEGF, VEGF, hFGF-B, R3-IGF-1, ascorbic acid, gentamicin/amphotericin-B and 5% FBS, (Lonza)). The BMEC were allowed to attach and migrate for 24 h before the medium was changed to supplemented EBM®-2 containing 4 μg/ml puromycin. After 2 days of puromycin treatment, the culture medium was replaced with supplemented EBM®-2 for another 2 days of maintenance before they were ready for construction of co-cultured model.

**Isolation and culture of rat astrocytes**

Rat cerebral astrocytes were obtained from neonatal SD rats. Brain cortex free of meninges was minced and digested in 0.125% trypsin-EDTA for 10 min (3 mL/rat brain). The activity of trypsin was terminated by adding the same volume of 10% FBS-DMEM. The suspension was dispersed and centrifuged at 1000 g for 5 min. The cell pellet was re-suspended with 10% FBS-DMEM solution and forced subsequently through a 100μm and 40μm filter. The filtrate was centrifuged and resuspended in 10% FBS-DMEM and then was plated on Poly-L-lysine coated flask at a density of $1\times10^5$ cells/$cm^2$. The medium was changed every 3 days. After 9 days of culture, the flasks was shaken over night in an atmosphere of 37°C, 95% relative humidity, and 5% $CO_2$ in order to eliminate the contaminating microglia. The resulting astrocytes were further passaged or cryo-preserved to -80°C.

**Construction of co-cultured in vitro BBB model and determination of transendothelial electrical resistance**

The astrocytes suspended in 10% FBS-DMEM were seeded on to Poly-L-lysine coated 24-well culture plate at passage between 1 and 4 at a density of $1.5\times10^4$/$cm^2$. After twenty-four hours, BMECs cultured on a Transwell for 5 days were transferred to the 24-well culture plates containing astrocytes. The medium in the luminal and abluminal compartment was replaced with serum-free supplemented EBM®-2 containing 550 nM hydrocortisone for 2 days. Transendothelial electrical resistance (TEER) of the monolayers were determined everyday using Millicell-ERS (Millipore, Bedford, MA) and when the TEER > 150 $\Omega\cdot cm^2$, the monolayer was ready for transport experiment. Identical TEER measurements were taken after treatment on BMEC monolayers with lysophospahtidylcholine (lyso-PC). Statistical analysis was performed by two-tailed unpaired Student's t test.

**Permeability of Lucifer Yellow in BMEC-astrocyte co-cultures**

BMEC monolayers were treated with 0.3, 1, 3, 6 and 10 μg/mL of lysoPC added to the apical side of Transwell system (n =4 for each concentration) co-culture plate followed by addition of 2 μL of lucifer yellow similarly placed to the apical side resulting in a final concentration of 100 μM Lucifer yellow. The tissue culture fluid on the apical side of each well was thoroughly mixed by several times of drawing up and dispensing. The dosed BMEC monolayers were incubated for 90 min in a humidified chamber at 37°C, 95% relative humidity, and 5% $CO_2$ while mixing on an orbital shaker at 130 rpm. After the 90 min incubation, 50 μL of donor medium (apical) and 100 μL of receiver (basolateral) medium were aspirated. The lucifer yellow concentration in the donor and receiver compartment was measured using fluorescence detection (excitation wavelength of 485 nm and emission wavelength of 530 nm). The permeability of lucifer yellow was determined using the following equation.

$$P_{exact} = -\left(\frac{V_D V_R}{(V_D + V_R)At}\right)\ln\left\{1 - \frac{(V_D + V_R)C_R(t)}{(V_D C_D(t) + V_R C_R(t))}\right\} \times 10^7 \text{ nm/s}$$

where $V_D$ and $V_R$ are donor and receiver medium volumes in mL, respectively
A is the membrane surface area in $cm^2$, t is the transport time in seconds
$C_R(t)$ is the measured concentration of lucifer yellow in the receiver compartment at time t (90 minutes)
$C_D(t)$ is the measured concentration of lucifer yellow in the donor compartment at time t (90 minutes)
Statistical analysis was performed by two-tailed unpaired Student's t test.

**Observations**

Induction of diabetes in SD rats with STZ for three days led to an increase in fasted blood glucose which was maintained over the course of the study. As expected treatment with Lp-PLA2 inhibitor '495 (10mg/kg/day) did not alter the hyperglycaemia induced by STZ. Animals which were not treated with STZ maintained a normal blood glucose level

of around 5mM throughout the course of the study (see Figure 8). Treatment of SD rats with STZ also resulted in an increase in plasma Lp-PLA2 activity which was subsequently suppressed following a single dose of Lp-PLA2 inhibitor ’495 (10mg/kg) observable at 3 hours post-dose and 24 hours post dose. The effect was even more pronounced 24 hours after the second daily dose of 10mg/kg Lp-PLA2 inhibitor ’495 as plasma Lp-PLA2 activity has risen further in animals which were treated with STZ but not Lp-PLA2 inhibitor ’495 (see Figure 9). These data provide direct evidence of the *in vivo* activity of Lp-PLA2 inhibitor ’495 in inhibiting plasma Lp-PLA2 activity.

Induction of hyperglycaemia in SD rats led to an increase in the extravasation of albumin associated-Evans Blue dye in the retina which provides direct evidence that such hyperglycaemia is able to result in an increase in the blood-retinal barrier permeability. Evans blue permeability and extravasation into the retinal parenchyma has been used many times and is an established technique to show in vivo blood-retinal barrier permeability in response to hyperglycaemia and other stimuli (Xu et al., 2001). The induction of hyperglycaemia for 31 days increased the permeability of the blood-retinal barrier in the majority of animals in comparison to that in non-diabetic animals. The increase in retinal permeability was attenuated by daily treatment of animals with Lp-PLA2 inhibitor ’495 (10mg/kg). However this was not to the level exhibited by non-diabetic controls and although not statistically significantly different from untreated diabetic animals there was a clear trend of effect (see Figure 10, A, B).

An additional methodology to assess retinal permeability *in vivo* is to directly image the retina using optical coherence tomography (OCT) which allows individual layers of the retina to be distinguished in live animals and any perturbation in the structure of the retinal layers to be assessed. It is also possible to discern the presence of sub-retinal fluid within the retina and such instrumentation is used clinically to diagnose and monitor treatment of diabetic eye disease. This technique was used to assess sub-retinal exudation of fluid in normo-glycaemic, hyperglycaemic and hyperglycaemic animals treated with Lp-PLA2 inhibitor ’495 (10mg/kg/day) for 28 days. It was noted that no normoglycaemic animal showed evidence of sub-retinal fluid accumulation by day 14 whereas hyperglycaemic animals had 40% of animals screened showing signs of retinal fluid accumulation. However hyperglycaemic animals which were treated with Lp-PLA2 inhibitor ’495 (10mg/kg/day) showed no animals to have overt retinal fluid accumulation as evidenced by OCT (see Figure 11). This data supports the Evans blue permeability data in demonstrating an effect of this class of compound in moderating retinal permeability secondary to acute hyperglycaemia in SD rats.

A consequence of increased blood-retinal barrier permeability is a loss of homeostasis in the retina which leads to degeneration of the neural retina, a feature observed in both pre-clinical models and human diabetic macula edema patients (Bursell et al., 1996; Ahlers et al., 2009). In pre-clinical models this manifests as a reduction in the number of neuronal cells (which are organised in layers in the retina) and consequently a thinning of the whole neuroretinal layer, defined as the layers between the retinal ganglion cell layer (RGC) and the retinal pigment epithelium (RPE). OCT was used to determine the thickness of the retinal layers. Two individual studies were performed in which treatment of animals with 50mg/kg/d i.p STZ for 3 days led to a dramatic time dependent reduction in retinal thickness. In the first study the STZ-induced retinal thinning was partially but significantly inhibited by treatment with 10mg/kg/day Lp-PLA2 inhibitor ’495 after treatment was initiated at 8 days and statistical differences assessed at 10 days and 18 days (see Figure 12, A, B, C, $p<0.05$ at both time points). Treatment with Lp-PLA2 inhibitor ’859 (initiated at day 4) also showed a reduction in retinal thickness but this effect was not statistically significantly different from vehicle only treated animals (Figure 12, A, B and C), although it should be noted that Lp-PLA2 inhibitor ’859 is a significantly less potent inhibitor than Lp-PLA2 inhibitor ’495 on the rat Lp-PLA2 enzyme.

In a second independent study, animals which were treated with STZ (50mg/kg/d i.p) for 3 days led to a dramatic time dependent reduction in retinal thickness, which was significantly different from animals which were not treated to induce diabetes ($p<0.001$ t-test). STZ-treated animals which were subsequently treated with Lp-PLA2 inhibitor ’495 STZ showed a significant attenuation in the development of retinal thinning when compared to vehicle-treated diabetic rats ($p<0.001$ t-test) (see Figure 13).

The action of Lp-PLA2 on oxidised phospholipids mediates the generation of lysophosphatidylcholine which has been proposed as a powerful inflammatory lipid and known to induce leukocyte recruitment and inflammation (Tan et al., 2009). When lysoPC is added to an in vitro model of the blood-CNS barrier (brain or retina) this agent is able to mediate increased transport of substances such as Lucifer Yellow which is normally not permeable across such in-vitro cultures and are completely excluded from the CNS in *in vivo* studies (Sarker et al., 2000). Studies using rat brain microvascular endothelial cells co-cultured with rat brain astrocytes to induce a tight endothelial monolayer demonstrated that addition of lysoPC to the apical surface of the endothelial monolayer resulted in a dose-dependent reduction in the transport of Lucifer yellow (see Figure 14 A). The increase in transport of Lucifer Yellow was also associated with a reduction in the transendothelial electrical resistance (TEER) measurements which are an indication of tight junctional integrity of the cellular monolayer (see Figure 14B). The action of lysoPC in mediating increased permeability has also been observed in human coronary artery endothelial cells where it has been demonstrated to increase monolayer permeability and reduce the expression of both tight-junction and adherens-junction associated proteins (Yan et al., 2005). The action of lysoPC also resulted in significant superoxide generation in these studies and treatment with an anti-oxidant resulted in a significant inhibition of lysoPC-stimulated monolayer permeability (Yan et al., 2005). The action

of the product of Lp-PLA2 activity, namely, lysoPC, in regulating endothelial monolayer permeability further supports the notion that Lp-PLA2 activity in the retinal vasculature is in part responsible for increased vascular permeability which occurs in diabetic macula edema. The generation of lysoPC can of course subsequently lead to the generation of lysophosphatidic acid (LPA) through the action of the serum enzyme lysophospholipase D (Umezu-Goto et al., 2002) which has a wide array of pharmacological activities on both the vasculature and leukocytes.

## Example 4

### Lp-PLA2 inhibitors in Streptozotocin (STZ)-induced diabetic Brown-Norway rat

STZ-induced diabetes is a commonly used model of type-1 diabetes and STZ-diabetic BN rats are an animal species most often used as preclinical models as their retinae exhibit most of the pathological features of background diabetic retinopathy seen in humans, including blood vessel dilation, basement membrane thickening, neuronal and glial dysfunction, and iBRB breakdown. This model of diabetic retinopathy has been widely used for assessing drug efficacy.

### Methods

### Animals, induction of diabetes and drug dosing

Brown Norway (BN) rats, 6 weeks of age and weighing 180-200g, were used in this study; the rats had free access to food and water and were maintained in cages in an environmentally controlled room with a 12 hour light- dark cycle. Diabetes was induced by following dosing of animals with streptozotocin (65mg/kg/day dosed i.p. for each of the first 3 days of the protocol) in 10mM sodium citrate-buffer pH 4.6. Lp-PLA2 inhibitor ’495 was solubilized in 10% Hydroxypropyl-beta-cyclodextrin or DMSO: 1% methylcellulose (1:99) and Lp-PLA2 inhibitor ’859 in DMSO: 1% methylcellulose (1:99) and dosed by i.p. injection at 10mg/kg/day from day 4 to day 31 and day 4 to day 18 respectively. Animals which maintained an elevated blood glucose level of over 9mM were deemed diabetic.

### Determination of retinal vascular permeability using Evans blue in BN rats

This methodology was performed according to previously published methods (Xu at el., 2001) as has previously been described for SD rats with the exception that ED was determined spectrophotometrically.

### Immunohistochemical detection of the leak of rat immunoglobulin G (IgG) from retinal blood vessels as an indicator of Blood-Retinal Barrier integrity and permeability.

Eyes were enucleated, hemisected along the ora serrata, and the vitreous humor removed. Eyecups were immersion fixed in 4% (w/v) paraformaldehyde for 30 min, washed in PBS, and the retinas detached. Fixed retinas were cryoprotected, embedded in Tissue-Tek OCT compound snap frozen in liquid nitrogen-cooled isopentane, and 12-μm cryosections prepared. Sections was immunostained with antibodies specific for rat immunoglobulin G (IgG) in order to locate any nonvascular IgG and isolectin B4 to determine the location of retinal blood vessels in the retinal sections and viewed on a confocal microscope. Fluorescence was visualized by using a Nikon TE-2000 C1 confocal system (Nikon Ltd, Kingston upon Thames, UK). In some cases retinal blood vessels were highlighted using propridium iodide staining

### Observations

### Assessment of retinal vascular permeability by Evans blue (albumin determination)

In the 2 week study, sustained hyperglycaemia in the streptozotocin-treated group was evidenced by regular blood glucose monitoring throughout the study and HbA1c determinations. Sustained hyperglycaemia resulted in a small increase in albumin leakage in the retinae. However due to the inherent variability of this model, and the generally small extent of the hyperglycemia-induced increase in EB leakage, this increase was not statistically significant. Treatment with Lp-PLA2 inhibitor ’495 (10mg/kg i.p. QD) reduced this leak to levels exhibited by the normo-glycaemic group, but again due to large variability this data was not statistically robust (Figure 3, upper and middle panels). However treatment of BN rats for with Lp-PLA2 inhibitor ’495 i.p. QD for 4 weeks following the induction of diabetes showed a statistically significant effect of Lp-PLA2 inhibitor ’495 (10mg/kg i.p. QD) in restricting the increase in vascular permeability secondary to hyperglycemia to albumin ($p<0.04$ vs. diabetic vehicle treated animals, Figure 15). Dosing of 10mg/kg i.p. QD in BN rats yielded trough Lp-$PLA_2$ inhibition levels of 89.7% (2 weeks study) and 93,2% (4 week study) in hyperglycemic rats which compares favorably with the 160mg dose of Lp-PLA2 inhibitor ’848 which produced 88% inhibition of Lp-$PLA_2$.ac-

tivity after 12 weeks in the clinic (Study LPL104884). These observations are consistent with the effect of Lp-PLA2 inhibitor ’495 in identical animals in which the extravasation of albumin into the retina as determined by immunohistochemistry was easily observable.

**Assessment of retinal vascular permeability by immunological detection**

Retinae from animals which were not processed for Evans blue were used for immunohistochemical albumin detection. Retinal blood vessels within cryosections were highlighted by either propriduim iodide (PI) staining or isolectin B4 staining (IB4). Rat albumin is highlighted using an anti-albumin mAb. In non-diabetic retinae there is strong co-localization of both labels showing albumin is contained intravascularly. The vessels of the superficial (SP) and deep plexus (DP) can be recognized. This demonstrates a normal functioning iBRB. In diabetic rats (31 d post STZ treatment) there is albumin localization to vascular compartment and the neuropile especially around regions adjacent to the vascular layers (arrows) indicating breakdown of the iBRB to proteins ~40KDa in size and thus the leakage of serum into the retinae. Diabetic animals treated with SB435495 show less leakage and extensive co-localization of albumin with retinal blood vessels when compared to vehicle only treated hyperglycaemic animals. These findings are apparent in sections derived from animals in the 2 and 4 week study. There is a large amount of albumin in the choroid (as expected) as this is a naturally leaky vascular bed (See Figures 16 & 17) These experiments clearly demonstrate an ability of a 10mg/kg (Figure 17) and 20mg/kg i.p QD (Figure 16) doses of SB435495 to limit the hyperglycemia-induced increase in vascular permeability as evidenced by the extravasation of albumin (protein), however increases in vascular permeability in CNS vessels can be specifically associated with changes with regulating the vascular permeability to molecules or different size.

Taken together, data in the rabbit and rat showing increased blood retinal barrier permeability induced by a combination of diabetes and hypercholesterolemia is responsive to treatment with pharmacologically active Lp-PLA2 inhibitors demonstrates that intervention with such pharmacological inhibitors in diseases in which a major contributing factor in disease progression is the breakdown of CNS vasculature barrier, such as diabetic macula edema, will be beneficial. The brain pig data is consistent with this retinal data. The consequences of CNS blood-barrier breakdown are often both ischemia and edema, which ultimately results in both neurodegeneration and vasodegeneration of the affected tissue, leading to cell death/cell loss. Neurodegeneration is often manifested and easily measured as a decrease in retinal thickness. It is notable that induction of diabetes in the STZ-treated rat led to reduced retinal thickness and this was partially reversed by treatment with the disclosed Lp-PLA2 pharmacological inhibitors. The effects of these agents on permeability and retinal protection as assessed by maintaining retinal thickness provides a compelling case to suggest that these compounds will be beneficial in protecting the retina from the vasodegenerative processes which are ongoing in diabetes patients suffering from the ocular consequences of this disease. Significant pharmacological effects of a range of active Lp-PLA2 inhibitors have shown therapeutically beneficial effects in a range of species in which disease was initiated with hyperglycaemia or hyperglycaemia and hypercholesterolemia. In addition to the effects of Lp-PLA2 compounds in illustrative studies there is extensive evidence that the product of the activity of Lp-PLA2 is a highly inflammatory lipid which is likely responsible for mediating the vascular permeability effects and inflammatory effects associated with these human diseases which are secondary to hypoglycaemia and hyperlipidemia.

In some embodiments, the optimum dosage of agents that inhibit Lp-$PLA_2$ is one that reduces activity and/or expression of Lp-$PLA_2$, for example, reduced expression of nucleic acid, for example mRNA encoded by Lp-$PLA_2$ gene or reduced expression or activity of Lp-$PLA_2$ protein. In other embodiments, the optimum dosage of agents that inhibit Lp-$PLA_2$ is one that generates the maximum protective effect in treating or preventing an ocular disease or disorder including, for example, but not limited to, macular edema of any cause, e.g., due to RVO, inflammation, post-surgical, traction, and the like; AMD; uveitis; diabetic eye diseases and disorders; diabetic retinopathy, and the like; or reducing a symptom of such an ocular disease or disorder.

In some embodiments, the patient population to be treated is adult DME patients with center involvement.

In another embodiment, the patient population has diabetes mellitus (type 1 or type 2).

In one embodiment, confirmation of DME is made using fluorescein angiography.

In one embodiment, retinal thickening (DME) involving the center of the fovea is determined by SD-OCT central subfield thickness >330 microns for Heidelberg Spectralis and >310 for Zeiss Cirrus.

In some embodiments, dosing is via intravitreal injection.

In some embodiments, the dosing is via oral delivery. A particularly effective dosage is a maximum oral daily dose not to exceed 160 mg. Suitably, the effective dosage is formulated as an enteric coat micronized free base tablet formulation, of the Lp-PLA2 inhibitor. A more particularly effective dosage is a maximum oral daily dose not to exceed 160 mg enteric coat micronized free base tablet formulation, of Darapladib.

In some embodiments, best-corrected visual acuity (BCVA) and retinal thickness, especially as compared to baseline before treatment for DME, are the indicators of effectiveness of the treatment with the Lp-PLA2 inhibitors disclosed herein.

In some embodiments, dosing will occur for a period of time, before treatment effects are realized. In a particular

embodiment, treatment is a daily oral dose of a pharmaceutical composition containing an Lp-PLA2 inhibitor, recommended for 30 days. In another embodiment, treatment is a daily oral dose of a pharmaceutical composition containing an Lp-PLA2 inhibitor, recommended for 60 days. In yet another embodiment, treatment is a daily oral dose of a pharmaceutical composition containing an Lp-PLA2 inhibitor, recommended for 90 days.

In one embodiment, to determine the effectiveness of the administration of an Lp-PLA2 inhibitor in subjects with center-involved DME, measurements are taken on BCVA. In another embodiment, to determine the effectiveness of the administration of an Lp-PLA2 inhibitor in subjects with center-involved DME, analysis is made using spectral domain OCT (SD-OCT imaging) center subfield of the eye.

In another embodiment, changes in retinal anatomy as assessed by one or more of fluorescein angiography (leakage area), fundus photography (retinal thickening area) and SD-OCT (macular volume, subretinal fluid, intraretinal cysts) of the eye, is used to determine efficacy of the treatment.

In some embodiments, in order to assess efficacy of the treatment, a subject would not have one or more the following additional eye diseases or disorders. Including cataract, glaucoma, ischemic optic neuropathy, retinitis pigmentosa, diabetic retinopathy, ischemic maculopathy, choroidal neovascularization, intraocular surgery.

## Formulations of compositions

Compounds, that is, agents inhibiting Lp-$PLA_2$ as disclosed herein, can be used as a medicament or used to formulate a pharmaceutical composition with one or more of the utilities disclosed herein. They can be administered in vitro to cells in culture, in vivo to cells in the body, or ex vivo to cells outside of an individual that can later be returned to the body of the same individual or another. Such cells can be disaggregated or provided as solid tissue.

Compounds, that is agents inhibiting Lp-$PLA_2$ as disclosed herein can be used to produce a medicament or other pharmaceutical compositions. Use of agents inhibiting Lp-$PLA_2$ which further comprise a pharmaceutically acceptable carrier and compositions which further comprise components useful for delivering the composition to an individual are known in the art. Addition of such carriers and other components to the agents as disclosed herein is well within the level of skill in this art.

In some embodiments, the compositions may be administered as a formulation adapted for systemic delivery. In some embodiments, the compositions may be administered as a formulation adapted for delivery to specific organs, for example but not limited to the liver, bone marrow, or systemic delivery.

Alternatively, pharmaceutical compositions can be added to the culture medium of cells ex vivo. In addition to the active compound, such compositions can contain pharmaceutically-acceptable carriers and other ingredients known to facilitate administration and/or enhance uptake (e.g., saline, dimethyl sulfoxide, lipid, polymer, affinity-based cell specific-targeting systems). The composition can be incorporated in a gel, sponge, or other permeable matrix (e.g., formed as pellets or a disk) and placed in proximity to the endothelium for sustained, local release. The composition can be administered in a single dose or in multiple doses which are administered at different times.

Pharmaceutical compositions can be administered by any known route. By way of example, the composition can be administered by a mucosal, pulmonary, oral, topical, or other localized or systemic route (e.g., enteral and parenteral). The phrases "parenteral administration" and "administered parenterally" as used herein means modes of administration other than enteral and topical administration, usually by injection, and includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection, infusion and other injection or infusion techniques, without limitation. The phrases "systemic administration," "administered systemically", "peripheral administration" and "administered peripherally" as used herein mean the administration of the agents as disclosed herein such that it enters the animal's system and, thus, is subject to metabolism and other like processes, for example, subcutaneous administration.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject agents from one organ, or portion of the body, to another organ, or portion of the body. Each carrier must be "acceptable" in the sense of being compatible with the other ingredients of the formulation, for example the carrier does not decrease the impact of the agent on the treatment. In other words, a carrier is pharmaceutically inert.

Suitable choices in amounts and timing of doses, formulation, and routes of administration can be made with the goals of achieving a favorable response in the subject with diabetic ocular diseases or a risk thereof (i.e., efficacy), and avoiding undue toxicity or other harm thereto (i.e., safety). Therefore, "effective" refers to such choices that involve

routine manipulation of conditions to achieve a desired effect.

A bolus of the formulation administered to an individual over a short time once a day is a convenient dosing schedule. Alternatively, the effective daily dose can be divided into multiple doses for purposes of administration, for example, two to twelve doses per day. Dosage levels of active ingredients in a pharmaceutical composition can also be varied so as to achieve a transient or sustained concentration of the compound or derivative thereof in an individual and to result in the desired therapeutic response or protection. But it is also within the skill of the art to start doses at levels lower than required to achieve the desired therapeutic effect and to gradually increase the dosage until the desired effect is achieved.

The amount of agents inhibiting Lp-$PLA_2$ administered is dependent upon factors known to a person skilled in the art such as bioactivity and bioavailability of the compound (e.g., half-life in the body, stability, and metabolism); chemical properties of the compound (e.g., molecular weight, hydrophobicity, and solubility); route and scheduling of administration, and the like. It will also be understood that the specific dose level to be achieved for any particular individual can depend on a variety of factors, including age, gender, health, medical history, weight, combination with one or more other drugs, and severity of disease.

The term "treatment", with respect to treatment of ocular diseases refers to, inter alia, preventing the development of the disease, or altering the course of the disease (for example, but not limited to, slowing the progression of the disease), or reversing a symptom of the disease or reducing one or more symptoms and/or one or more biochemical markers in a subject, preventing one or more symptoms from worsening or progressing, promoting recovery or improving prognosis, and/or preventing disease in a subject who is free there from as well as slowing or reducing progression of existing disease. For a given subject, improvement in a symptom, its worsening, regression, or progression can be determined by an objective or subjective measure.

Prophylactic methods (e.g., preventing or reducing the incidence of relapse) are also considered treatment.

In some embodiments, treatment can also involve combination with other existing modes of treatment, for example existing agents for treatment of ocular diseases , such as anti VEGF therapeutics e.g. Lucentis®, Avastin® and Aflibercept® and steroids, e.g., triamcinolone, and steroid implants containing fluocinolone acetonide.

In some embodiments, agents that inhibit Lp-$PLA_2$ as disclosed herein can be combined with other agent, for example therapeutic agent to prevent and/or treat neurodegenerative diseases. Such agents can be any agent currently in use or being developed for the treatment and/or prevention of a neurodegenerative disease or disorder, where the agent can have a prophylactic and/or a curative effect and/or reduce a symptom of an ocular disorder or disease.

Thus, combination treatment with one or more agents that inhibit Lp-$PLA_2$ with one or more other medical procedures can be practiced.

In addition, treatment can also comprise multiple agents to inhibit Lp-$PLA_2$ expression or activity.

The amount which is administered to a subject is preferably an amount that does not induce toxic effects which outweigh the advantages which result from its administration. Further objectives are to reduce in number, diminish in severity, and/or otherwise relieve suffering from the symptoms of the disease in the individual in comparison to recognized standards of care.

Production of compounds according to present regulations will be regulated for good laboratory practices (GLP) and good manufacturing practices (GMP) by governmental agencies (e.g., U.S. Food and Drug Administration). This requires accurate and complete record keeping, as well as monitoring of QA/QC. Oversight of patient protocols by agencies and institutional panels is also envisioned to ensure that informed consent is obtained; safety, bioactivity, appropriate dosage, and efficacy of products are studied in phases; results are statistically significant; and ethical guidelines are followed. Similar oversight of protocols using animal models, as well as the use of toxic chemicals, and compliance with regulations is required.

Dosages, formulations, dosage volumes, regimens, and methods for analyzing results aimed at inhibiting Lp-$PLA_2$ expression and/or activity can vary. Thus, minimum and maximum effective dosages vary depending on the method of administration. Suppression of the clinical and histological changes associated with ocular diseases can occur within a specific dosage range, which, however, varies depending on the organism receiving the dosage, the route of administration, whether agents that inhibit Lp-$PLA_2$ are administered in conjunction with other co-stimulatory molecules, and the specific regimen of inhibitor of Lp-$PLA_2$ administration. For example, in general, nasal administration requires a smaller dosage than oral, enteral, rectal, or vaginal administration.

For oral or enteral formulations for use with the present invention, tablets can be formulated in accordance with conventional procedures employing solid carriers well-known in the art. Capsules employed for oral formulations to be used with the methods of the present invention can be made from any pharmaceutically acceptable material, such as gelatin or cellulose derivatives. Sustained release oral delivery systems and/or enteric coatings for orally administered dosage forms are also contemplated, such as those described in U.S. Pat. No. 4,704,295, "Enteric Film-Coating Compositions," issued Nov. 3, 1987; U.S. Pat. No. 4, 556,552, "Enteric Film- Coating Compositions," issued Dec. 3, 1985; U.S. Pat. No. 4,309,404, "Sustained Release Pharmaceutical Compositions," issued Jan. 5, 1982; and U.S. Pat. No. 4,309,406, "Sustained Release Pharmaceutical Compositions," issued Jan. 5, 1982.

A particularly effective dosage for use herein is 160 mg enteric coat micronized free base tablet formulation, of the Lp-PLA2 inhibitor. A more particularly effective dosage is 160 mg enteric coat micronized free base tablet formulation, of Darapladib.

Examples of solid carriers include starch, sugar, bentonite, silica, and other commonly used carriers. Further non-limiting examples of carriers and diluents which can be used in the formulations of the present invention include saline, syrup, dextrose, and water.

Enteric Coated Formulation

As regards formulations for administering the small chemical entities for inhibitors of Lp-$PLA_2$ of the likes of formulas (I) - (IV) as disclosed herein, one particularly useful embodiment is a tablet formulation comprising the Lp-PLA inhibitor with an enteric polymer casing. An example of such a preparation can be found in WO2005/021002. The active material in the core can be present in a micronized or solubilized form. In addition to active materials the core can contain additives conventional to the art of compressed tablets. Appropriate additives in such a tablet can comprise diluents such as anhydrous lactose, lactose monohydrate, calcium carbonate, magnesium carbonate, dicalcium phosphate or mixtures thereof; binders such as microcrystalline cellulose, hydroxypropylmethylcellulose, hydroxypropyl-cellulose, polyvinylpyrrolidone, pre-gelatinized starch or gum acacia or mixtures thereof; disintegrants such as microcrystalline cellulose (fulfilling both binder and disintegrant functions) cross-linked polyvinylpyrrolidone, sodium starch glycollate, croscarmellose sodium or mixtures thereof; lubricants, such as magnesium stearate or stearic acid, glidants or flow aids, such as colloidal silica, talc or starch, and stabilizers such as desiccating amorphous silica, coloring agents, flavors etc. Suitably the tablet comprises lactose as diluent. When a binder is present, it is suitably hydroxypropylmethyl cellulose. Suitably, the tablet comprises magnesium stearate as lubricant. Suitably the tablet comprises croscarmellose sodium as disintegrant. Suitably, the tablet comprises microcrystalline cellulose.

The diluent can be present in a range of 10 - 80% by weight of the core. The lubricant can be present in a range of 0.25 - 2% by weight of the core. The disintegrant can be present in a range of 1 - 10% by weight of the core. Microcrystalline cellulose, if present, can be present in a range of 10 - 80% by weight of the core.

The active ingredient suitably comprises between 10 and 50% of the weight of the core, more suitably between 15 and 35% of the weight of the core (calculated as free base equivalent). The core can contain any therapeutically suitable dosage level of the active ingredient, but suitably contains up to 150mg as free base of the active ingredient. Particularly suitably, the core contains 20, 30, 40, 50, 60, 80 or 100mg as free base of the active ingredient. The active ingredient can be present as the free base, or as any pharmaceutically acceptable salt. If the active ingredient is present as a salt, the weight is adjusted such that the tablet contains the desired amount of active ingredient, calculated as free base of the salt.

The core can be made from a compacted mixture of its components. The components can be directly compressed, or can be granulated before compression. Such granules can be formed by a conventional granulating process as known in the art. In an alternative embodiment, the granules can be individually coated with an enteric casing, and then enclosed in a standard capsule casing.

The core is surrounded by a casing which comprises an enteric polymer. Examples of enteric polymers are cellulose acetate phthalate, cellulose acetate succinate, methylcellulose phthalate, ethylhydroxycellulose phthalate, polyvinylacetate pthalate, polyvinylbutyrate acetate, vinyl acetate-maleic anhydride copolymer, styrene-maleic monoester copolymer, methyl acrylate-methacrylic acid copolymer or methacrylate-methacrylic acid-octyl acrylate copolymer. These can be used either alone or in combination, or together with other polymers than those mentioned above. The casing can also include insoluble substances which are neither decomposed nor solubilized in living bodies, such as alkyl cellulose derivatives such as ethyl cellulose, crosslinked polymers such as styrenedivinylbenzene copolymer, polysaccharides having hydroxyl groups such as dextran, cellulose derivatives which are treated with bifunctional crosslinking agents such as epichlorohydrin, dichlorohydrin or 1, 2-, 3, 4-diepoxybutane. The casing can also include starch and/or dextrin.

Suitable enteric coating materials are the commercially available Eudragit enteric polymers such as Eudragit L, Eudragit S and Eudragit NE used alone or with a plasticizer. Such coatings are normally applied using a liquid medium, and the nature of the plasticizer depends upon whether the medium is aqueous or non-aqueous. Plasticizers for use with aqueous medium include propylene glycol, triethyl citrate, acetyl triethyl citrate or Citroflex or Citroflex A2. Non-aqueous plasticizers include these, and also diethyl and dibutyl phthalate and dibutyl sebacate. A suitable plasticizer is triethyl citrate. The quantity of plasticizer included will be apparent to those skilled in the art.

The casing can also include an anti-tack agent such as talc, silica or glyceryl monostearate. Suitably the anti-tack agent is glyceryl monostearate. Typically, the casing can include around 5 - 25 wt% plasticizer and up to around 50 wt % of anti tack agent, suitably 1-10 wt % of anti-tack agent.

If desired, a surfactant can be included to aid with forming an aqueous suspension of the polymer. Many examples of possible surfactants are known to the person skilled in the art. Suitable examples of surfactants are polysorbate 80, polysorbate 20, or sodium lauryl sulphate. If present, a surfactant can form 0.1 - 10% of the casing, Suitably 0.2 - 5% and particularly Suitably 0.5 - 2%

In one embodiment, there is a seal coat included between the core and the enteric coating. A seal coat is a coating material which can be used to protect the enteric casing from possible chemical attack by any alkaline ingredients in the core. The seal coat can also provide a smoother surface, thereby allowing easier attachment of the enteric casing. A person skilled in the art would be aware of suitable coatings. Suitably the seal coat is made of an Opadry coating, and particularly suitably it is Opadry White OY-S-28876.

The treatment of macular edema of any cause, e.g., due to RVO, inflammation, post-surgical, traction, and the like; AMD; uveitis; with LpPLA2 inhibitors as described in the appended claims, may also be administered locally, as a topical eye drop, a peri-ocular injection (e.g., sub-tenon) or via intravitreal injection. Sustained release of the drug may also be achieved by the use of technologies such as solid implants (which may or may not be bio-degradable) or bio-degradable polymeric matrices (e.g. micro-particles). These may be administered either peri-ocularly or intravitreally.

**REFERENCES**

Antonetti DA, Barber AJ, Bronson SK, Freeman WM, Gardner TW, Jefferson LS, Kester M, Kimball SR, Krady JK, LaNoue KF, Norbury CC, Quinn PG, Sandirasegarane L, Simpson IA. (2006) Diabetic retinopathy: seeing beyond glucose-induced microvascular disease. Diabetes; 55:2401-2411.

Curtis TM, Gardiner TA, Stitt AW. (2009) Microvascular lesions of diabetic retinopathy: clues towards understanding pathogenesis? Eye 23: 1496-1508

Chung EJ, Freeman WR, Azen SP, Lee H, Koh HJ. (2008)Comparison of combination posterior sub-tenon triamcinolone and modified grid laser treatment with intravitreal triamcinolone treatment in patients with diffuse diabetic macular edema. Yonsei Medical Journal 49:955-964.

Hammes HP, Alt A, Niwa T, Clausen JT, Bretzel RG, Brownlee M, Schleicher ED (1999) Differential accumulation of advanced glycation end products in the course of diabetic retinopathy. Diabetologia 42:728-736

Bucala R, Vlassara H (1995) Advanced glycosylation end products in diabetic renal and vascular disease. Am J Kidney Dis. 26:875-888

Makita Z, Bucala R, Rayfield EJ, Friedman EA, Kaufman AM, Korbet SM, Barth RH, Winston JA, Fuh H, Manogue KR (1994) Reactive glycosylation endproducts in diabetic uraemia and treatment of renal failure. Lancet 343:1519-1522

Murata T, Nagai R, Ishibashi T, Inomuta H, Ikeda K, Horiuchi S (1997) The relationship between accumulation of advanced glycation end products and expression of vascular endothelial growth factor in human diabetic retinas. Diabetologia 40:764-769

Stitt AW (2001) Advanced glycation: an important pathological event in diabetic and age related ocular disease. Br J Ophthalmol 85:746-753

Stitt AW, Li YM, Gardiner TA, Bucala R, Archer DB, Vlassara H (1997) Advanced glycation end products (AGEs) co-localize with AGE receptors in the retinal vasculature of diabetic and of AGE-infused rats. Am J Pathol 150:523-531

Stitt AW, Bhaduri T, McMullen CB, Gardiner TA, Archer DB (2000) Advanced glycation end products induce blood-retinal barrier dysfunction in normoglycemic rats. Mol Cell Biol Res Commun 3:380-388

Chakravarthy U, Hayes RG, Stitt AW, McAuley E, Archer DB (1998) Constitutive nitric oxide synthase expression in retinal vascular endothelial cells is suppressed by high glucose and advanced glycation end products. Diabetes 47:945-95

Giardino, D. Edelstein and M. Brownlee, (1994) Nonenzymatic glycosylation in vitro and in bovine endothelial cells alters basic fibroblast growth factor activity. A model for intracellular glycosylation in diabetes. J Clin Invest 94: pp. 489-496.

Y.H. Abdel-Wahab, F.P. O'Harte, H. Ratcliff, N.H. McClenaghan, C.R. Barnett and P.R. Flatt, (1996)Glycation of insulin in the islets of Langerhans of normal and diabetic animals. Diabetes 45: 1489-1496.

H. Vlassara, (2001)The AGE-receptor in the pathogenesis of diabetic complications. Diabetes Metab Res Rev 17: 436-443.

A.M. Schmidt, S.D. Yan, S.F. Yan and D.M. Stern, (2000) The biology of the receptor for advanced glycation end products and its ligands.Biochim Biophys Acta 1498:99-111.

P. Mene, C. Pascale, A. Teti, S. Bernardini, G.A. Cinotti and F. Pugliese, (1999) Effects of advanced glycation end products on cytosolic Ca2+ signaling of cultured human mesangial cells. J Am Soc Nephrol 10: 1478-1486.

V. Scivittaro, M.B. Ganz and M.F. Weiss, (2000) AGEs induce oxidative stress and activate protein kinase C-beta(II) in neonatal mesangial cells. Am J Physiol Renal Physiol 278: 676-683.

J.S. Huang, J.Y. Guh, W.C. Hung, M.L. Yang, Y.H. Lai, H.C. Chen et al., (1999)Role of the Janus kinase (JAK)/signal

transducters and activators of transcription (STAT) cascade in advanced glycation end-product-induced cellular mitogenesis in NRK-49F cells. Biochem J 342:231-238.

A.A. Deora, T. Win, B. Vanhaesebroeck and H.M. Lander, (1998) A redox-triggered ras-effector interaction. Recruitment of phosphatidylinositol 3'-kinase to Ras by redox stress. J Biol Chem 273:29923-29928.

H.M. Lander, J.M. Tauras, J.S. Ogiste, O. Hori, R.A. Moss and A.M. Schmidt, (1997) Activation of the receptor for advanced glycation end products triggers a p21 (ras)-dependent mitogen-activated protein kinase pathway regulated by oxidant stress. J Biol Chem 272:17810-17814.

A. Bierhaus, S. Schiekofer, M. Schwaninger, M. Andrassy, P.M. Humpert, J. Chen et al., (2001) Diabetes-associated sustained activation of the transcription factor nuclear factor-kappaB. Diabetes 50: 2792-2808.

H. Sano, R. Nagai, K. Matsumoto and S. Horiuchi, (1999) Receptors for proteins modified by advanced glycation endproducts (AGE)-their functional role in atherosclerosis. Mech Ageing Dev 107: 333-346.

Schmidt AM, Hori O, Brett J, Yan SD, Wautier JL, Stern D. (1994) Cellular receptors for glycation end products. Implications for induction of oxidant stress and cellular dysfunction in the pathogenesis of vascular lesions. Arterioscler Thromb 14:. 1521-1528.

Lu M, Kuroki M, Amano S, Tolentino M, Keough K, Kim I, Bucala R, Adamis AP (1998) Advanced glycation end products increase retinal vascular endothelial growth factor expression. J Clin Invest 101:1219-1224

Paget, M. Lecomte, D. Ruggiero, N. Wiernsperger and M. Lagarde, (1998) Modification of enzymatic antioxidants in retinal microvascular cells by glucose or advanced glycation end products. Free Radicals Biol Med 25: 121-129.

Gegg M, Harry R., Hankey D., Zambarakji H., Pryce G., Baker D, Adamson P., Calder V. And Greenwood J. (2005) Supression of autoimmune retinal disease by lovastatin does not require Th2 cytokine induction. J. Immunol 174: 2327-2235

Li J., Wang, J.J. Cgen D., Mott R., Qiang Yu., Ma J-X., Zhang S.X. (2010) Systemic administration of HMGCoA reductase inhibitor protects the blood-retinal barrier and ameliorates retinal inflammation in type2 diabetes. Exp. Eye Res. 89: 71-78.

Mohler ER, III, et al. (2008) Site-specific atherogenic gene expression correlates with subsequent variable lesion development in coronary and peripheral vasculature. Arterioscler. Thromb. Vasc. Biol. 28:850-855.

Wilensky RL, Shi Y, Mohler ER 3rd, Hamamdzic D, Burgert ME, Li J, Postle A, Fenning RS, Bollinger JG, Hoffman BE, Pelchovitz DJ, Yang J, Mirabile RC, Webb CL, Zhang L, Zhang P, Gelb MH, Walker MC, Zalewski A, Macphee CH. (2008) Inhibition of lipoprotein-associated phospholipase A2 reduces complex coronary atherosclerotic plaque development. Nat Med.14: 1059-66.

Pennathur, S. & Heinecke, J.W. (2007) Mechanisms for oxidative stress in diabetic cardiovascular disease. Antioxid. Redox Signal. 9, 955-969

Moreno, P.R. et al. (2000) Coronary composition and macrophage infiltration in atherectomy specimens from patients with diabetes mellitus. Circulation 102, 2180-2184.

Dave, G.S., and Kalia, K. (2007). Hyperglycemia induced oxidative stress in type-1 and type-2 diabetic patients with and without nephropathy. Cell. Mol. Biol., 53(5): 68-78.

Wilensky RL, Macphee CH (2009) Lipoprotein-associated phospholipase A(2) and atherosclerosis. Curr Opin Lipidol. 20: 415-20.

S. Lavi, J.P. McConnell, C.S. Rihal, A. Prasad, V. Mathew, L.O. Lerman and A. Lerman, (2007) Local production of lipoprotein-associated phospholipase A2 and lysophosphatidylcholine in the coronary circulation: association with early coronary atherosclerosis and endothelial dysfunction in humans. Circulation 115 2715-2721.

Clifford PM, Zarrabi S, Siu G, Kinsler KJ, Kosciuk MC, Venkataraman V, D'Andrea MR, Dinsmore S, Nagele RG. (2007) Abeta peptides can enter the brain through a defective blood-brain barrier and bind selectively to neurons. Brain Res. 1142: 223-36.

Lenzsér G, Kis B, Bari F, Busija DW. (2005) Diazoxide preconditioning attenuates global cerebral ischemia-induced blood-brain barrier permeability. Brain Res. 1051: 72-80.

Jones JM, Dowling TC, Park JJ, Phares DA, Park JY, Obisesan TO, Brown MD. (2007) Differential aerobic exercise-induced changes in plasma aldosterone between African Americans and Caucasians. Exp Physiol. 92:871-879.

Morrey JD, Olsen AL, Siddharthan V, Motter NE, Wang H, Taro BS, Chen D, Ruffner D, Hall JO. (2008) increased blood-brain barrier permeability is not a primary determinant for lethality of West Nile virus infection in rodents. J Gen Virol. 89: 467-473.

Xu Q., Qaum T and Adamsis A.P. (2001) Sensitive Blood-retinal barrier breakdown quantitation using Evans Blue. Invest. Ophthalmol. Vis. Sci. 42: 789-794

Bursell SE, Clermont AC, Kinsley BT, et al. (1996) Retinal blood flow changes in patients with insulin-dependent diabetes mellitus and no diabetic retinopathy. Invest Ophthalmol Vis Sci. 37:886-897

Ahlers C, Golbaz I, Einwallner E, Dunavölgyi R, Malamos P, Stock G, Pruente C, Schmidt-Erfurth U. (2009)Identification of optical density ratios in subretinal fluid as a clinically relevant biomarker in exudative macular disease. Invest Ophthalmol Vis Sci. ;50: 3417-3424.

Yan S, Chai H, Wang H, Yang H, Nan B, Yao Q, Chen C. (2005) Effects of lysophosphatidylcholine on monolayer cell permeability of human coronary artery endothelial cells. Surgery, 138:464-73
Tan M, Hao F, Xu X, Chisolm GM, Cui MZ. (2009) Lysophosphatidylcholine activates a novel PKD2-mediated signaling pathway that controls monocyte migration. Arterioscler Thromb Vasc Biol. 29:1376-82.
Sarker MH, Hu DE, Fraser PA. (2000) Acute effects of bradykinin on cerebral microvascular permeability in the anaesthetized rat. J Physiol. 528:177-87.
Umezu-Goto M, Kishi Y, Taira A, Hama K, Dohmae N, Takio K, Yamori T, Mills GB, Inoue K, Aoki J, Arai H. (2002) Autotaxin has lysophospholipase D activity leading to tumor cell growth and motility by lysophosphatidic acid production. J Cell Biol. 158:227-233.

## Claims

**1.** A compound selected from:

N-[2-(diethylamino)ethyl]-2-[[(4-fluorophenyl)methyl]thio]-4,5,6,7-tetrahydro-4-oxo-N-[[4'-(trifluoromethyl)[1,1'-biphenyl]-4-yl]methyl]-1H-cyclopentapyrimidine-1-acetamide, or a pharmaceutically acceptable salt thereof; and
N-[2-(diethylamino)ethyl]-2-{2-[2-(2,3-difluorophenyl)ethyl]-4-oxo-4,5,6,7-tetrahydro-1Hcyclopenta[d]pyrimidin-1-yl}-N-{[4'-(trifluoromethyl)-4-biphenylyl]methyl}acetamide bitartrate, or a pharmaceutically acceptable salt thereof; and
2-[[(2,3-difluorophenyl)methyl]thio]-N-[1-(2-methoxyethyl)-4-piperidinyl]-4-oxo-N-[[4'-(trifluoromethyl)[1,1'-biphenyl]-4-yl]methyl]-1(4H)-quinolineacetamide, or a pharmaceutically acceptable salt thereof; and
N-[2-(dimethylamino)ethyl]-2-[[(4-fluorophenyl)methyl]thio]-5-(1-methyl-1H-pyrazol-4-yl)methyl]-4-oxo-N-[[4'-(trifluoromethyl)[1,1'-biphenyl]-4-yl]methyl]-1(4H)-pyrimidineacetamide, or a pharmaceutically acceptable salt thereof,
for use in treating and/or preventing macular edema in a subject.

**2.** A pharmaceutical composition comprising a compound selected from:

N-[2-(diethylamino)ethyl]-2-[[(4-fluorophenyl)methyl]thio]-4,5,6,7-tetrahydro-4-oxo-N-[[4'-(trifluoromethyl)[1,1'-biphenyl]-4-yl]methyl]-1H-cyclopentapyrimidine-1-acetamide, or a pharmaceutically acceptable salt thereof; and
N-[2-(diethylamino)ethyl]-2-{2-[2-(2,3-difluorophenyl)ethyl]-4-oxo-4,5,6,7-tetrahydro-1Hcyclopenta[d]pyrimidin-1-yl}-N-{[4'-(trifluoromethyl)-4-biphenylyl]methyl}acetamide bitartrate, or a pharmaceutically acceptable salt thereof; and
2-[[(2,3-difluorophenyl)methyl]thio]-N-[1-(2-methoxyethyl)-4-piperidinyl]-4-oxo-N-[[4'-(trifluoromethyl)[1 ,1'-biphenyl]-4-yl]methyl]-1(4H)-quinolineacetamide, or a pharmaceutically acceptable salt thereof; and
N-[2-(dimethylamino)ethyl]-2-[[(4-fluorophenyl)methyl]thio]-5-(1-methyl-1H-pyrazol-4-yl)methyl]-4-oxo-N-[[4'-(trifluoromethyl)[1,1'-biphenyl]-4-yl]methyl]-1(4H)-pyrimidineacetamide, or a pharmaceutically acceptable salt thereof,
for use in treating and/or preventing macular edema in a subject.

**3.** The compound for use of claim 1 or the pharmaceutical composition for use of claim 2, wherein the macular edema is associated with a diabetic eye disease or disorder, or the macular edema is associated with retinal vein occlusion, inflammation, post-surgical, traction, or uveitis.

**4.** The compound for use or pharmaceutical composition for use of claim 3, wherein the macular edema is cystic macular edema.

## Patentansprüche

**1.** Eine Verbindung, ausgewählt aus:

N-[2-(Diethylamino)ethyl]-2-[[(4-fluorphenyl)methyl]thio]-4,5,6,7-tetrahydro-4-oxo-N-[[4'-(trifluormethyl)[1,1'-biphenyl]-4-yl]methyl]-1H-cyclopentapyrimidin-1-acetamid oder ein pharmazeutisch verträgliches Salz davon; und
N-[2-(Diethylamino)ethyl]-2-{2-[2-(2,3-difluorphenyl)ethyl]-4-oxo-4,5,6,7-tetrahydro-1H-cyclopenta[d]pyrimidin-1-yl}-N-{[4'-(trifluormethyl)-4-biphenylyl]methyl}acetamid-bitartrat oder ein pharmazeutisch verträgliches

Salz davon; und
2-[[(2,3-Difluorphenyl)methyl]thio]-N-[1-(2-methoxyethyl)-4-piperidinyl]-4-oxo-N-[[4'-(trifluormethyl) [1,1'-biphenyl] -4-yl] methyl]-1(4H)-chinolinacetamid oder ein pharmazeutisch verträgliches Salz davon; und
N-[2-(Dimethylamino)ethyl]-2-[[(4-fluorphenyl)methyl]thio]-5-(1-methyl-1H-pyrazo1-4-yl)methyl]-4-oxo-N-[[4'-(trifluormethyl) [1,1'-biphenyl] -4-yl] methyl]-1(4H)-pyrimidinacetamid oder ein pharmazeutisch verträgliches Salz davon,
zur Verwendung bei der Behandlung und/oder Vorbeugung eines Makulaödems bei einem Individuum.

2. Ein Arzneimittel, umfassend eine Verbindung, ausgewählt aus:

N-[2-(Diethylamino)ethyl]-2-[[(4-fluorphenyl)methyl]thio]-4,5,6,7-tetrahydro-4-oxo-N-[[4'-(trifluormethyl) [1,1'-biphenyl] -4-yl] methyl]-1H-cyclopentapyrimidin-1-acetamid oder ein pharmazeutisch verträgliches Salz davon; und
N-[2-(Diethylamino)ethyl]-2-{2-[2-(2,3-difluorphenyl)ethyl]-4-oxo-4,5,6,7-tetrahydro-1H-cyclopenta[dlpyrimidin-1-yl}-N-{[4'-(trifluormethyl)-4-biphenylyl]methyl}acetamid-bitartrat oder ein pharmazeutisch verträgliches Salz davon; und
2-[[(2,3-Difluorphenyl)methyl]thio]-N-[1-(2-methoxyethyl)-4-piperidinyl]-4-oxo-N-[[4'-(trifluormethyl) [1,1'-biphenyl] -4-yl] methyl]-1(4H)-chinolinacetamid oder ein pharmazeutisch verträgliches Salz davon; und
N-[2-(Dimethylamino)ethyl]-2-[[(4-fluorphenyl)methyl]thiol-5-(1-methyl-1H-pyrazol-4-yl)methyl]-4-oxo-N-[[4'-(trifluormethyl)]1,1'-biphenyl]-4-yl]methyl]-1(4H)-pyrimidinacetamid oder ein pharmazeutisch verträgliches Salz davon,
zur Verwendung bei der Behandlung und/oder Vorbeugung eines Makulaödems bei einem Individuum.

3. Die Verbindung zur Verwendung nach Anspruch 1 oder das Arzneimittel zur Verwendung nach Anspruch 2, wobei das Makulaödem mit einer diabetischen Augenerkrankung oder -störung in Verbindung steht, oder das Makulaödem mit Netzhautvenenverschluss, Entzündung, postoperativ, Traktion oder Uveitis in Verbindung steht.

4. Die Verbindung zur Verwendung oder das Arzneimittel zur Verwendung nach Anspruch 3, wobei das Makulaödem ein cystisches Makulaödem ist.

**Revendications**

1. Composé choisi parmi :

le N-[2-(diéthylamino)éthyl]-2-[[(4-fluorophényl)-méthyl]thio]-4,5,6,7-tétrahydro-4-oxo-N-[[4'-(trifluoro-méthyl)-[1,1-biphényl]-4-yl]méthyl]-1H-cyclopentapyrimidine-1-acétamide, ou un de ses sels pharmaceutiquement acceptables ; et
le bitartrate de N-[2-(diéthylamino)éthyl]-2-{2-[2-(2,3-difluorophényl)éthyl]-4-oxo-4,5,6,7-tétrahydro-1H-cyclopenta[d]pyrimidine-1-yl}-N-{[4'-(trifluorométhyl)-4-biphénylyl]méthyl}acétamide, ou un de ses sels pharmaceutiquement acceptables ; et
le 2-[[(2,3-difluorophényl)méthyllthiol-N-[1-(2-méthoxyéthyl)-4-pipéridinyl]-4-oxo-N-[[4'-(trifluorométhyl)-[1,1'-biphényl]-4-yl]méthyl]-1(4H)-quinoline-acétamide, ou un de ses sels pharmaceutiquement acceptables ; et
le N-[2-(diméthylamino)éthyl]-2-[[(4-fluorophényl)-méthyl]thio]-5-(1-méthyl-1H-pyrazole-4-yl)méthyl]-4-oxo-N-[[4'-(trifluorométhyl)[1,1'-biphényl]-4-yl]méthyl]-1(4H)-pyridine-acétamide, ou un de ses sels pharmaceutiquement acceptables,
pour une utilisation dans le traitement et/ou la prévention d'un œdème maculaire chez un sujet.

2. Composition pharmaceutique comprenant un composé choisi parmi :

le N-[2-(diéthylamino)éthyl]-2-[[(4-fluorophényl)-méthyl]thio]-4,5,6,7-tétrahydro-4-oxo-N-[[4'-(trifluoro-méthyl)[1,1'-biphényl]-4-yl]méthyl]-1H-cyclopentapyrimidine-1-acétamide, ou un de ses sels pharmaceutiquement acceptables ; et
le bitartrate de N-[2-(diéthylamino)éthyl]-2-{2-[2-(2,3-difluorophényl)éthyl]-4-oxo-4,5,6,7-tétrahydro-1H-cyclopenta[d]pyrimidine-1-yl}-N-{[4'-(trifluorométhyl)-4-biphénylyl]méthyl}acétamide, ou un de ses sels pharmaceutiquement acceptables ; et
le 2-[[(2,3-difluorophényl)méthyl]thio]-N-[1-(2-méthoxyéthyl)-4-pipéridinyl]-4-oxo-N-[[4'-(trifluorométhyl)-[1,1'-biphényl]-4-yl]méthyl]-1(4H)-quinoline-acétamide, ou un de ses sels pharmaceutiquement acceptables ; et

le N-[2-(diméthylamino)éthyl]-2-[[(4-fluorophényl)-méthyl]thio]-5-(1-méthyl-1H-pyrazole-4-yl)méthyl]-4-oxo-N-[[4'-(trifluorométhyl) [1,1'-biphényl]-4-yl]méthyl]-1(4H)-pyridine-acétamide, ou un de ses sels pharmaceutiquement acceptables,
pour une utilisation dans le traitement et/ou la prévention d'un œdème maculaire chez un sujet.

**3.** Composé pour une utilisation selon la revendication 1 ou composition pour une utilisation selon la revendication 2, où l'œdème maculaire est associé à une maladie ou un trouble oculaire diabétique, ou l'œdème maculaire est associé à une occlusion veineuse rétinienne, une inflammation, un état postopératoire, une traction, ou une uvéite.

**4.** Composé pour une utilisation ou composition pharmaceutique pour une utilisation selon la revendication 3, où l'œdème maculaire est un œdème maculaire kystique.

FIG. 1

# DMHC causes BBB breakdown, leading to plasma influx into the brain tissue and binding of IgG to neurons

***•IgG = brown***

Small leak at arteriolar bifurcation – neurons IgG positive

Several microvessels leaking IgG
Red dotted line shows IgG leak interface

Considerable IgG in interstium and bound to neurons in regions of leak

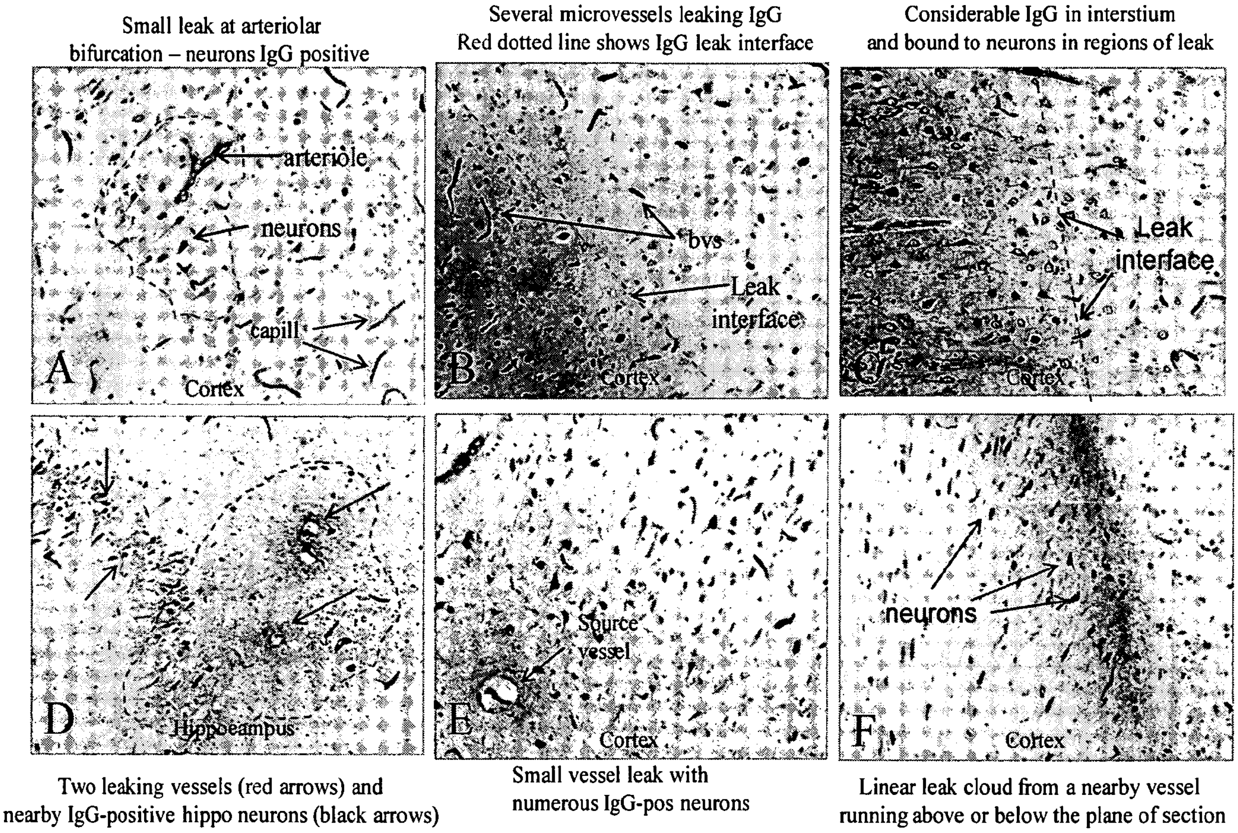


Two leaking vessels (red arrows) and nearby IgG-positive hippo neurons (black arrows)

Small vessel leak with numerous IgG-pos neurons

Linear leak cloud from a nearby vessel running above or below the plane of section

FIG. 2

Darapladib reduces the density of leaks from arterioles in the cortex

Density of Arteriolar Leaks in Cortex

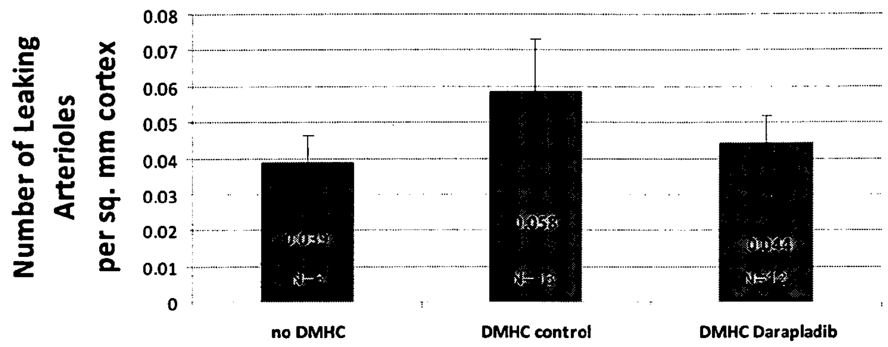


| | TTEST |
|---|---|
| DMHC control / no DMHC | 0.259 |
| DMHC control /DMHC Darapladib | 0.397 |
| DMHC Darapladib / no DMHC | 0.637 |

FIG. 2B

Darapladib reduces the amount of material that leaks from arterioles into the brain

Amount of material leaking from arterioles

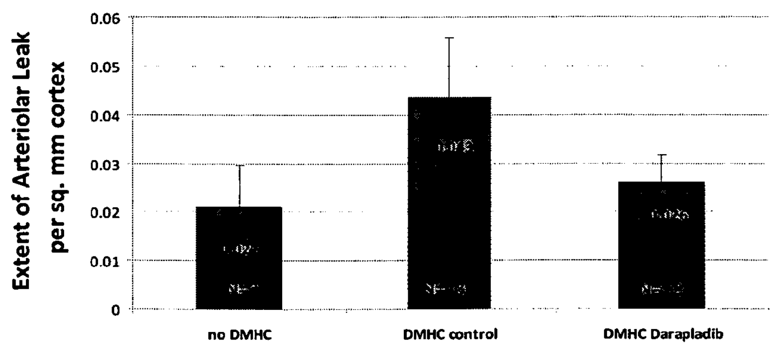


| | TTEST |
|---|---|
| DMHC control / no DMHC | 0.157 |
| DMHC control /DMHC Darapladib | 0.208 |
| DMHC Darapladib / no DMHC | 0.632 |

FIG. 2A

## FIG. 3

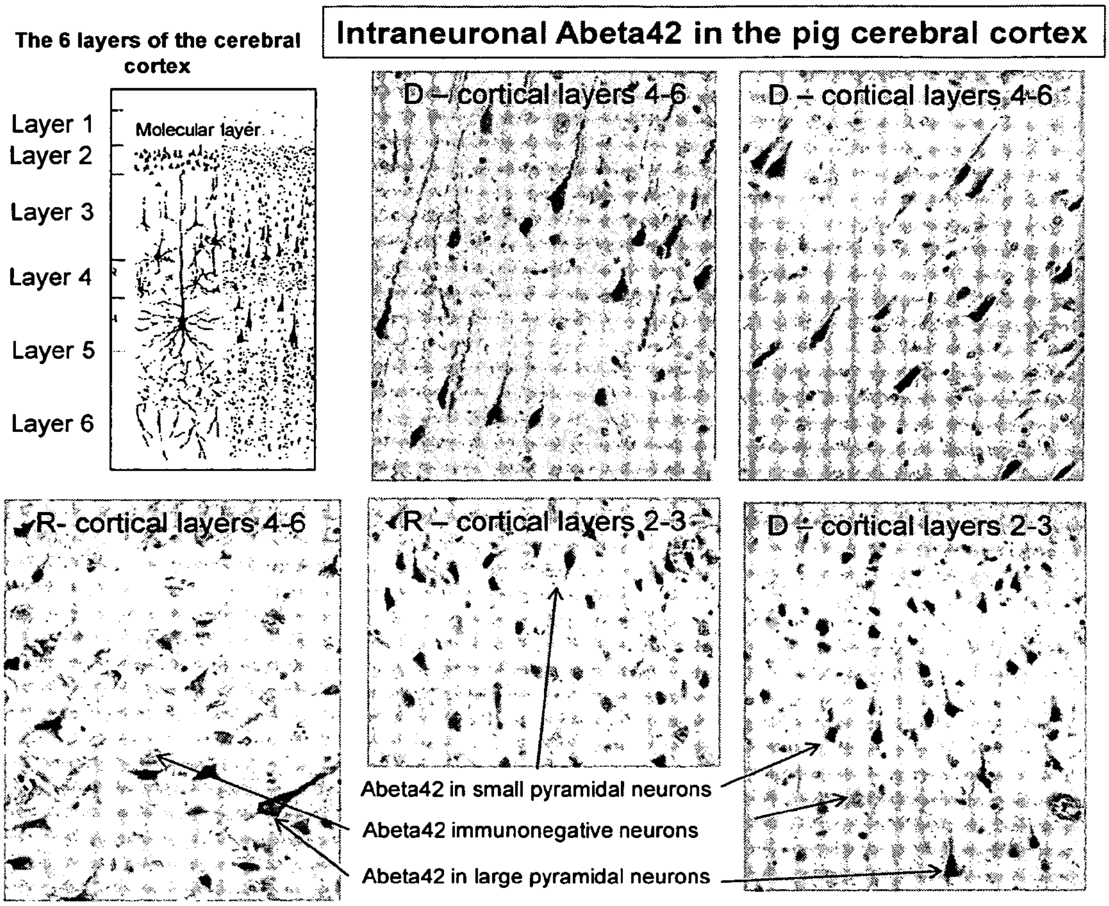
The 6 layers of the cerebral cortex
Layer 1
Layer 2
Layer 3
Layer 4
Layer 5
Layer 6
Molecular layer
Intraneuronal Abeta42 in the pig cerebral cortex
D – cortical layers 4-6
D – cortical layers 4-6
R- cortical layers 4-6
R – cortical layers 2-3
D – cortical layers 2-3
Abeta42 in small pyramidal neurons
Abeta42 immunonegative neurons
Abeta42 in large pyramidal neurons

FIG. 4

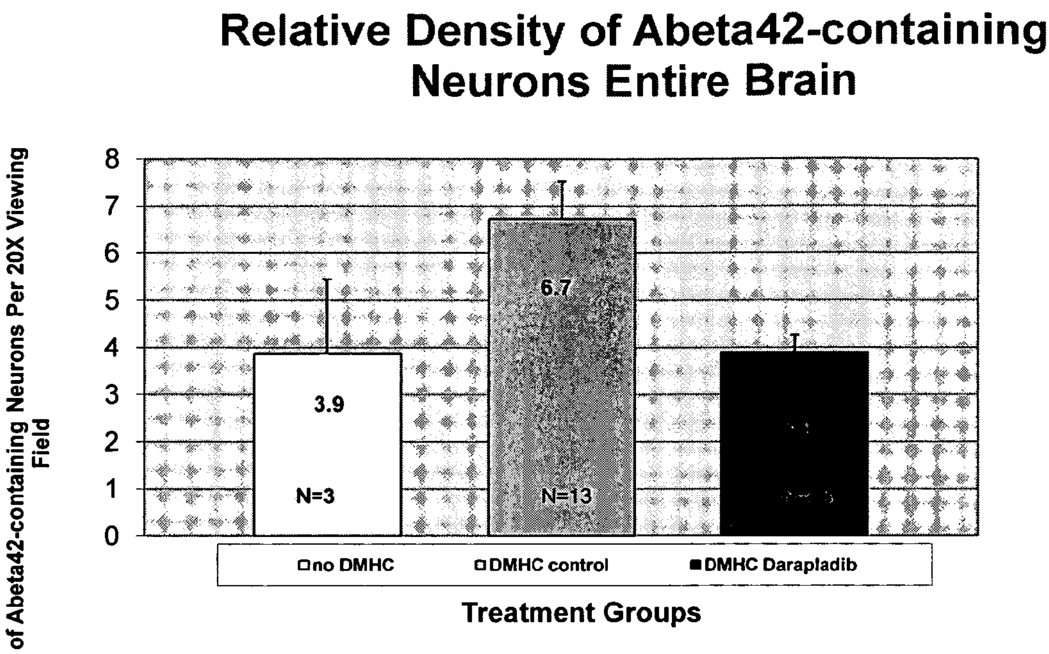


| | P-value |
|---|---|
| DMHC control / no DMHC | 0.201 |
| DMHC control / DMHC Darapladib | 0.005 |
| DMHC Darapladib / no DMHC | 0.986 |

FIG. 4A

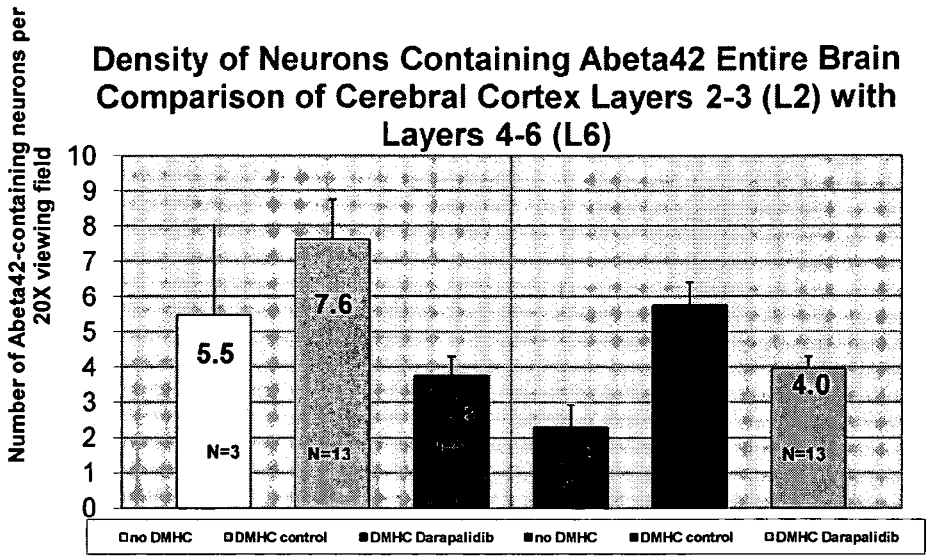


P-value

| | L2 | L6 |
|---|---|---|
| DMHC control / no DMHC | 0.503 | 0.006 |
| DMHC control / DMHC Darapladib | 0.007 | 0.024 |
| DMHC Darapladib / no DMHC | 0.577 | 0.096 |

FIG. 4B

FIG. 5

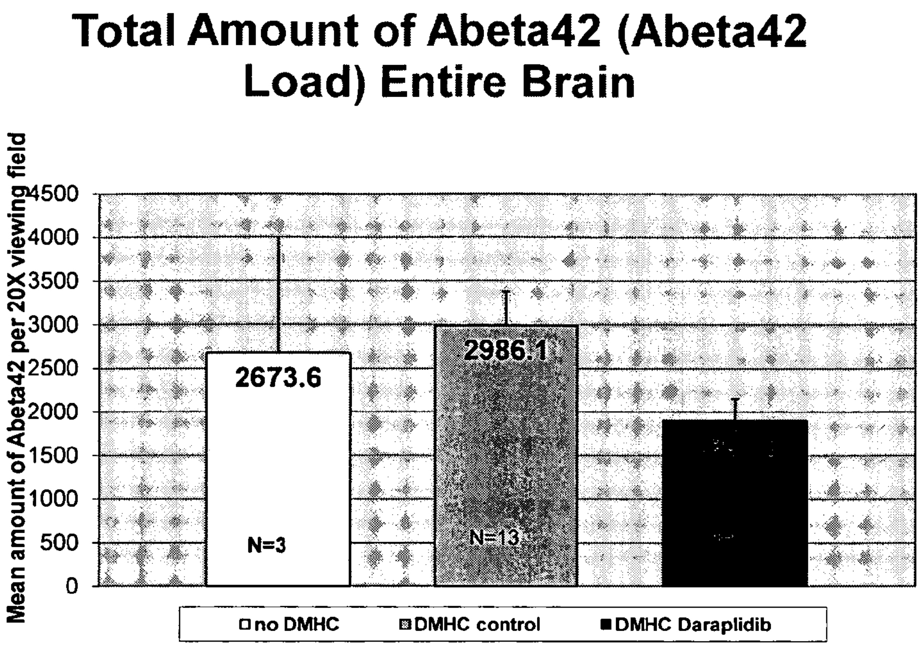


| | P-value |
|---|---|
| DMHC control / no DMHC | 0.839 |
| DMHC control / DMHC Darapladib | 0.029 |
| DMHC Darapladib / no DMHC | 0.622 |

FIG. 5A

## Amount of Abeta42 per Abeta42-containing Neuron Entire Brain

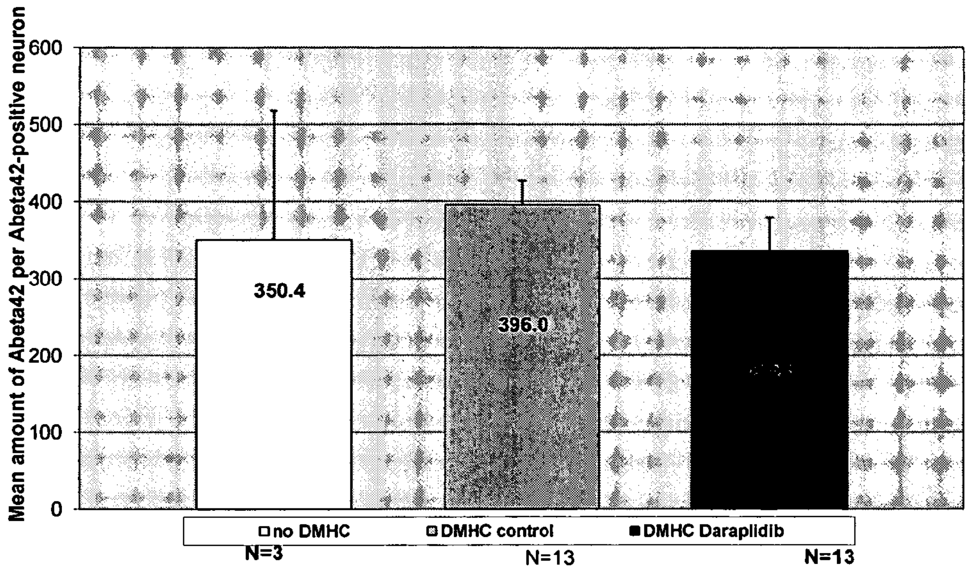


| | P-value |
|---|---|
| DMHC control / no DMHC | 0.813 |
| DMHC control / DMHC Darapladib | 0.266 |
| DMHC Darapladib / no DMHC | 0.938 |

FIG. 5B

FIG. 6

Lp-PLA2 Levels in Alzheimer Model Rabbits Over 10 Weeks

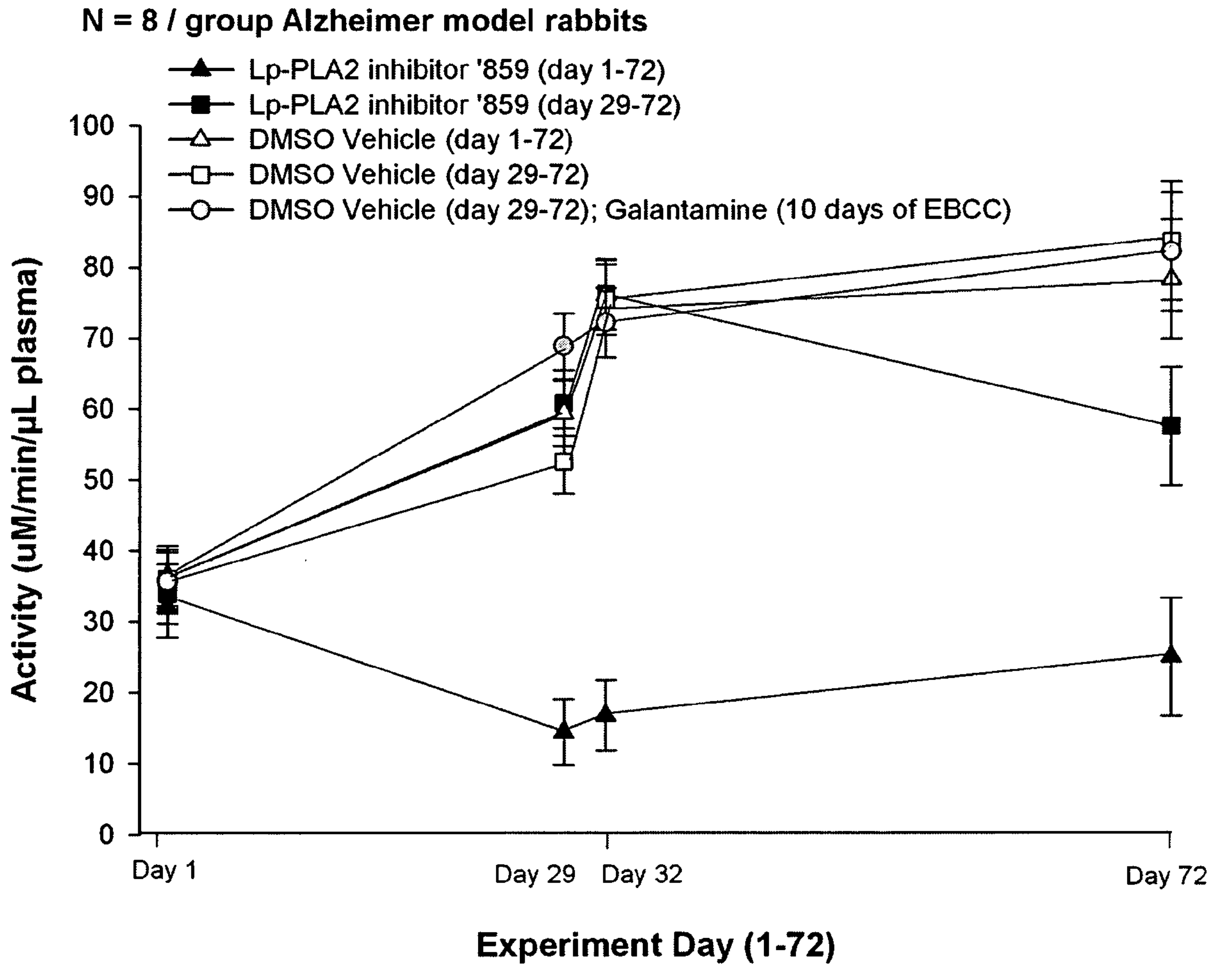

FIG. 7

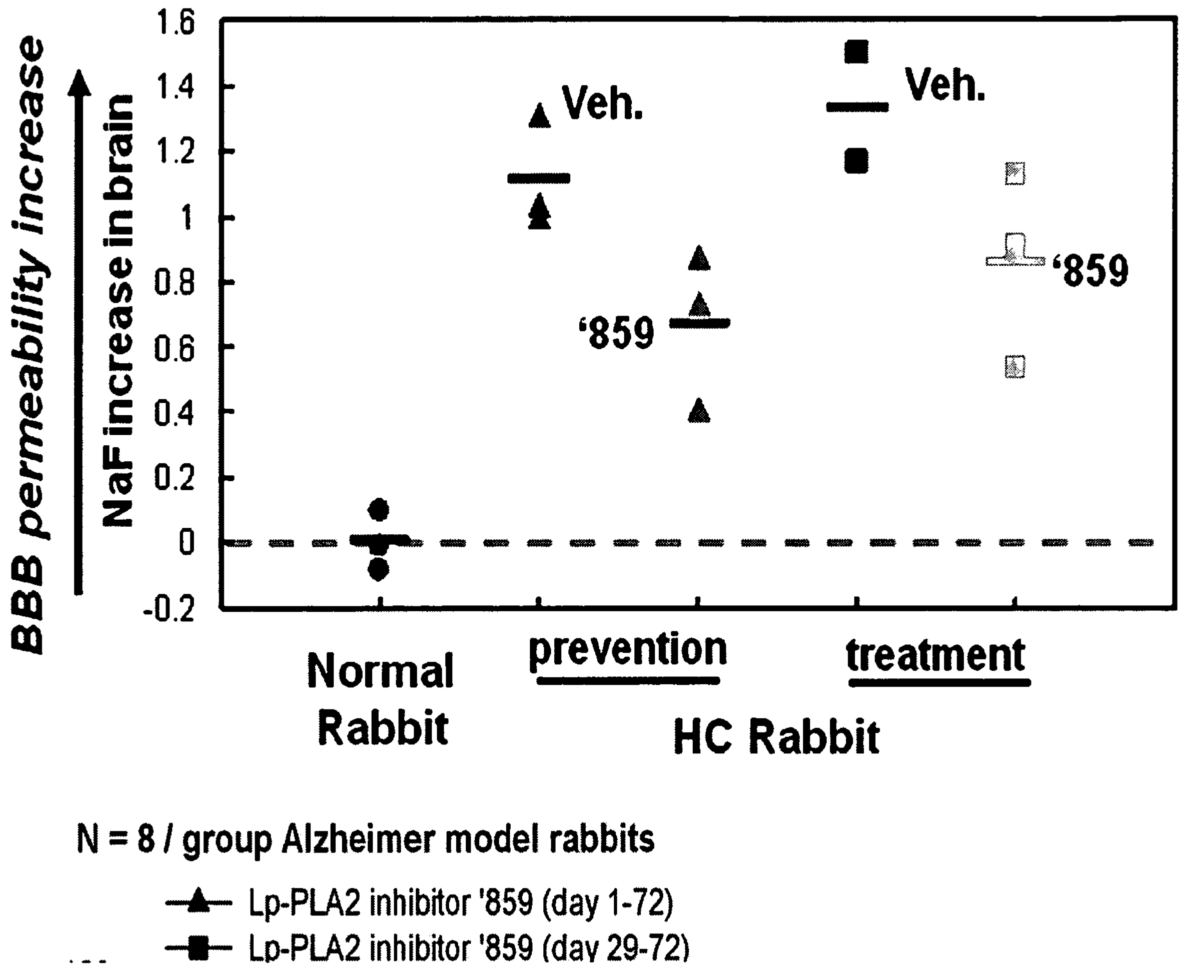
BBB permeability increase
NaF increase in brain
1.6
1.4
1.2
1
0.8
0.6
0.4
0.2
0
-0.2
Veh.
'859
Veh.
'859
Normal Rabbit
prevention
treatment
HC Rabbit
N = 8 / group Alzheimer model rabbits
Lp-PLA2 inhibitor '859 (day 1-72)
Lp-PLA2 inhibitor '859 (day 29-72)

FIG. 8

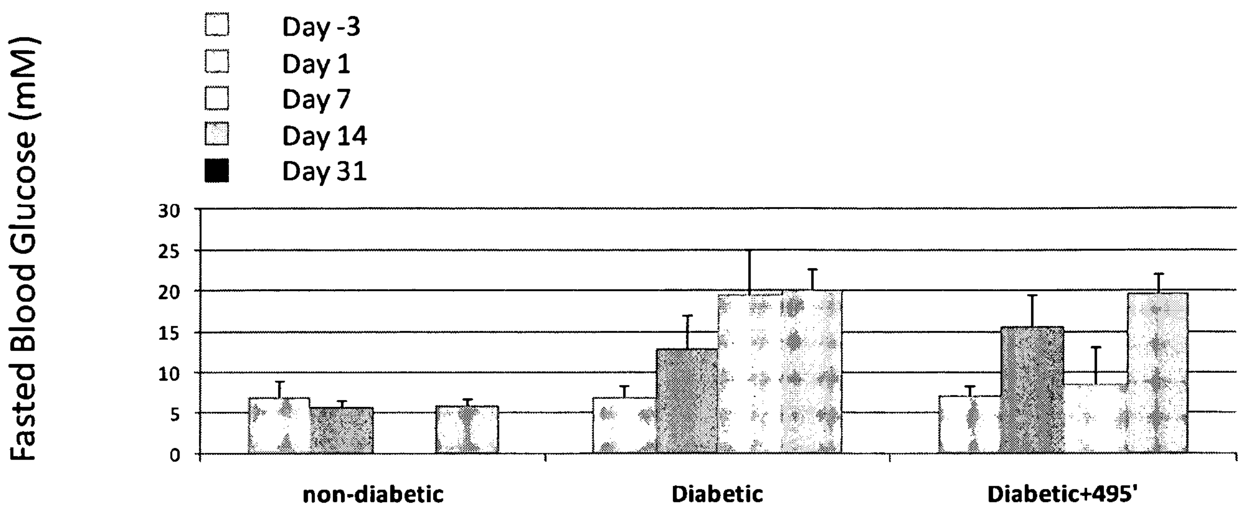
Day -3
Day 1
Day 7
Day 14
Day 31
Fasted Blood Glucose (mM)
30
25
20
15
10
5
0
non-diabetic
Diabetic
Diabetic+495'

FIG. 9

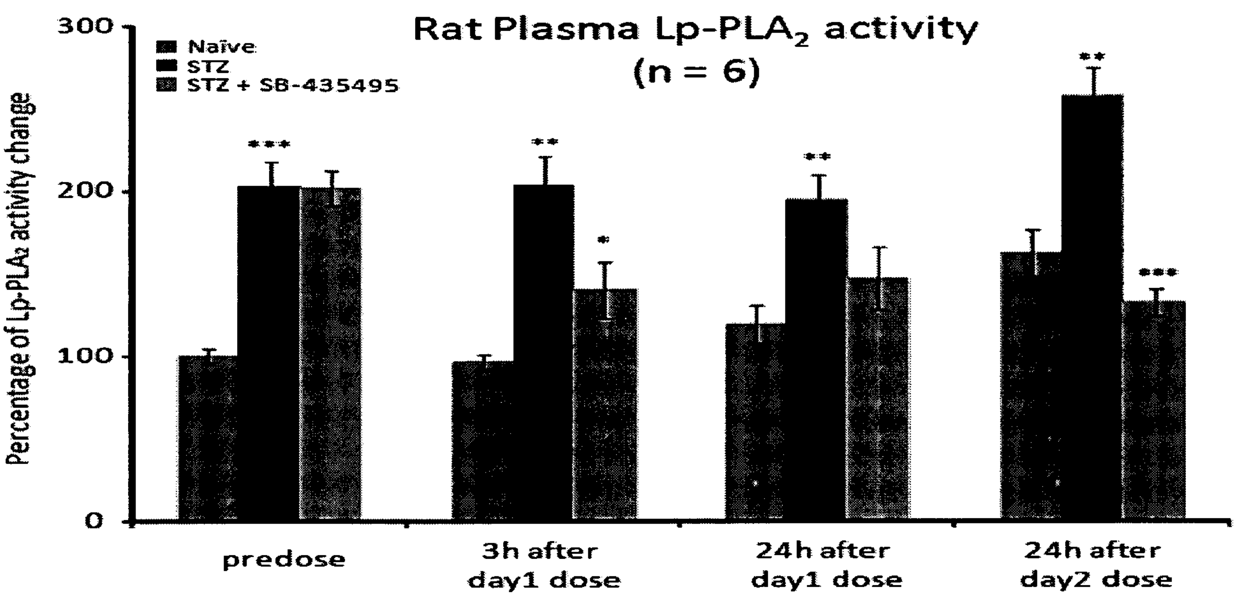
Rat Plasma Lp-PLA$_2$ activity
(n = 6)
Naïve
STZ
STZ + SB-435495
Percentage of Lp-PLA$_2$ activity change
300
200
100
0
***
**
*
**
**
***
predose
3h after day1 dose
24h after day1 dose
24h after day2 dose

FIG. 10

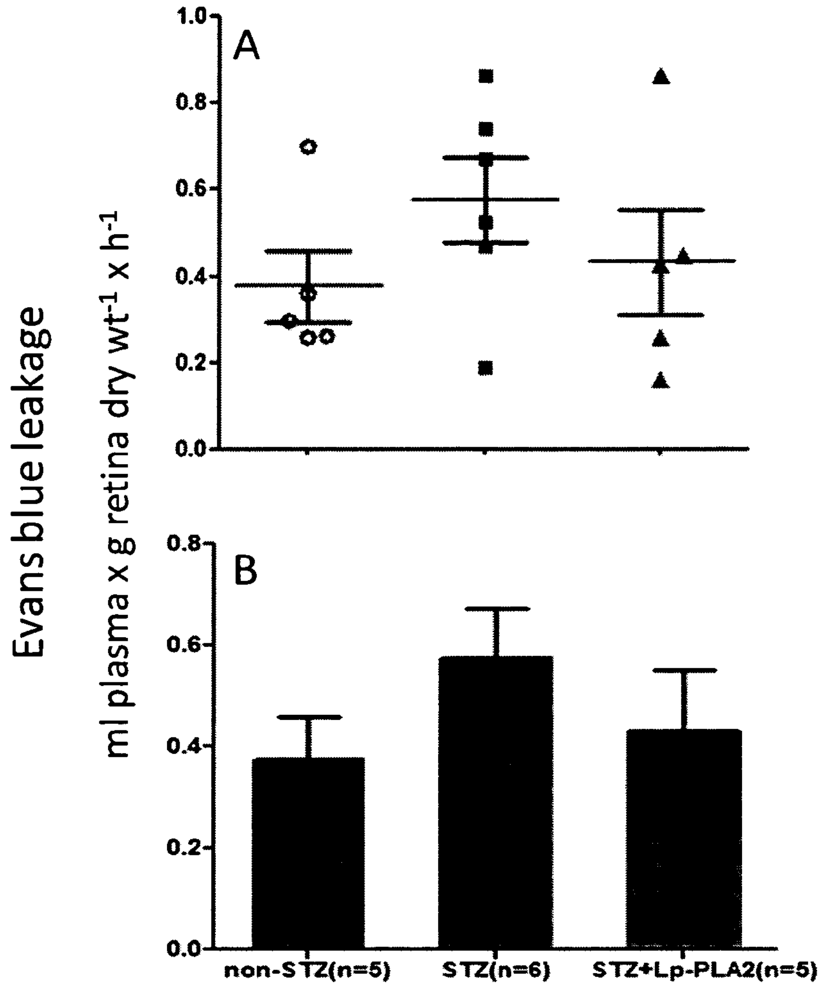
Evans blue leakage
ml plasma x g retina dry wt$^{-1}$ x h$^{-1}$
A
1.0
0.8
0.6
0.4
0.2
0.0
B
0.8
0.6
0.4
0.2
0.0
non-STZ(n=5)
STZ(n=6)
STZ+Lp-PLA2(n=5)

FIG. 11

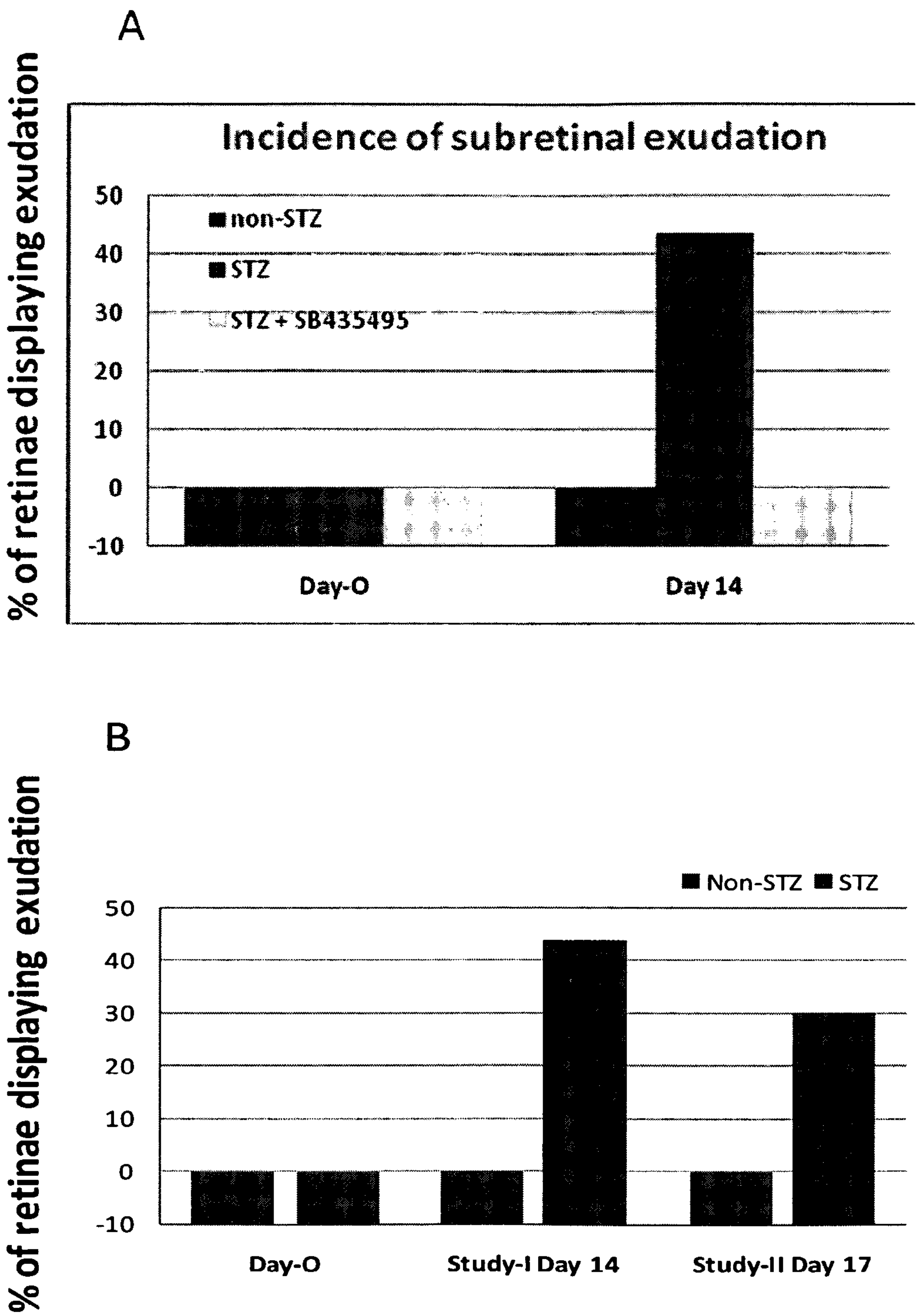
A
Incidence of subretinal exudation
% of retinae displaying exudation
non-STZ
STZ
STZ + SB435495
50
40
30
20
10
0
-10
Day-O
Day 14
B
% of retinae displaying exudation
Non-STZ
STZ
50
40
30
20
10
0
-10
Day-O
Study-I Day 14
Study-II Day 17

FIG. 12

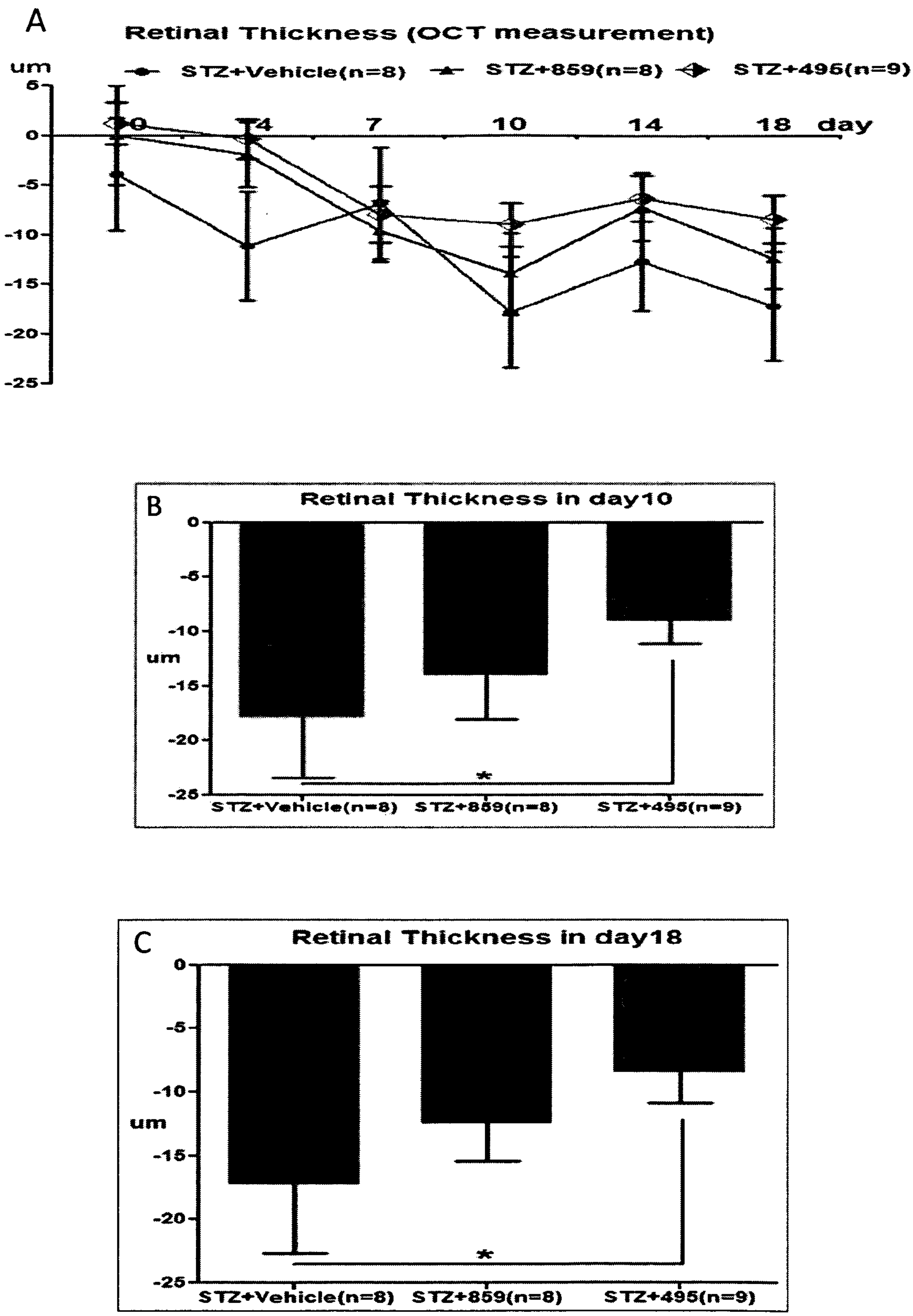
A
Retinal Thickness (OCT measurement)
um
STZ+Vehicle(n=8)
STZ+859(n=8)
STZ+495(n=9)
5
0
-5
-10
-15
-20
-25
0
4
7
10
14
18
day
B
Retinal Thickness in day10
0
-5
-10
um
-15
-20
-25
*
STZ+Vehicle(n=8)
STZ+859(n=8)
STZ+495(n=9)
C
Retinal Thickness in day18
0
-5
-10
um
-15
-20
-25
*
STZ+Vehicle(n=8)
STZ+859(n=8)
STZ+495(n=9)

FIG. 13

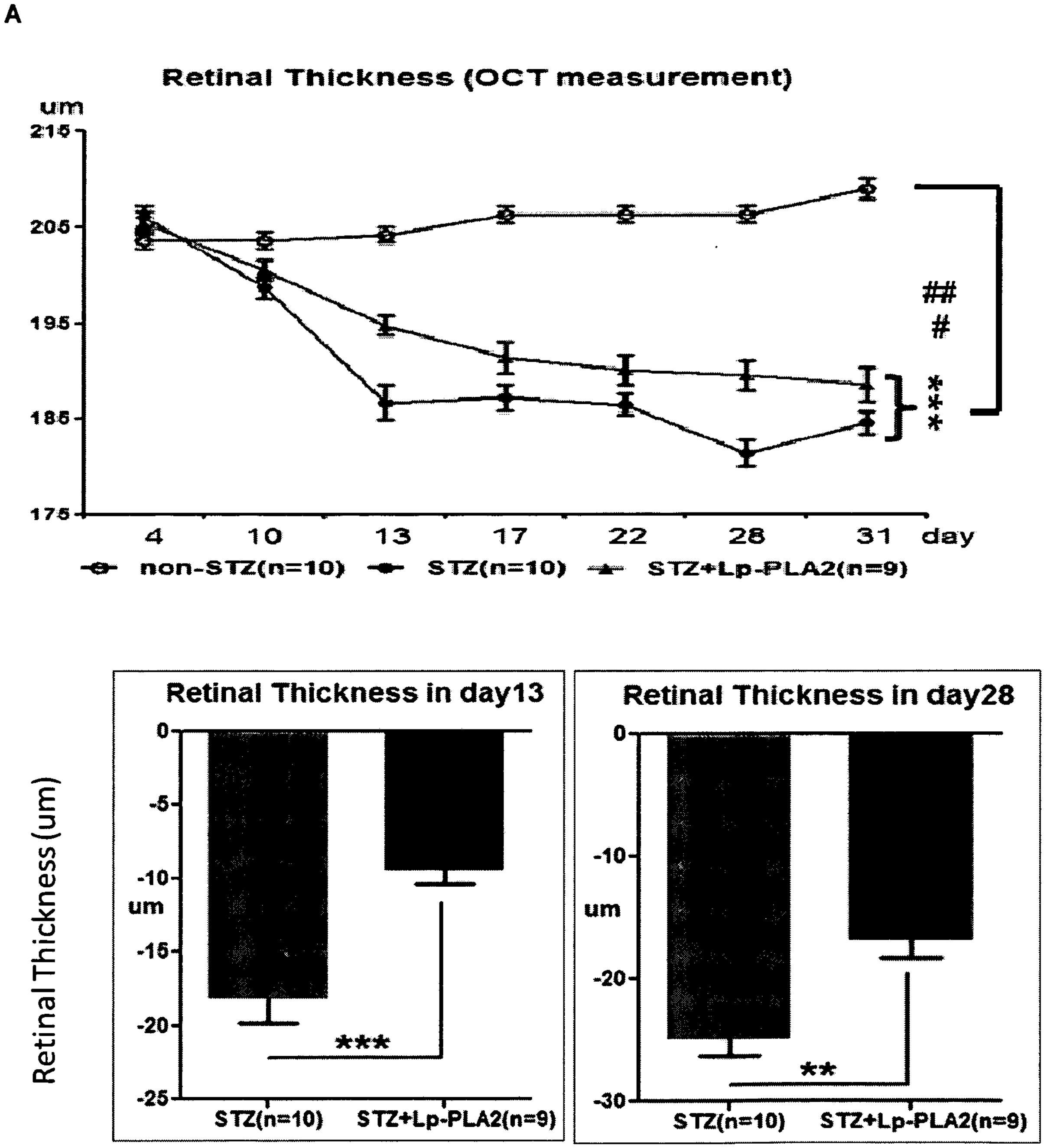
A
Retinal Thickness (OCT measurement)
um
215
205
195
185
175
4
10
13
17
22
28
31
day
##
#
***
non-STZ(n=10)
STZ(n=10)
STZ+Lp-PLA2(n=9)
Retinal Thickness (um)
Retinal Thickness in day13
0
-5
-10
um
-15
-20
-25
***
STZ(n=10)
STZ+Lp-PLA2(n=9)
Retinal Thickness in day28
0
-10
um
-20
-30
**
STZ(n=10)
STZ+Lp-PLA2(n=9)

FIG. 14

A

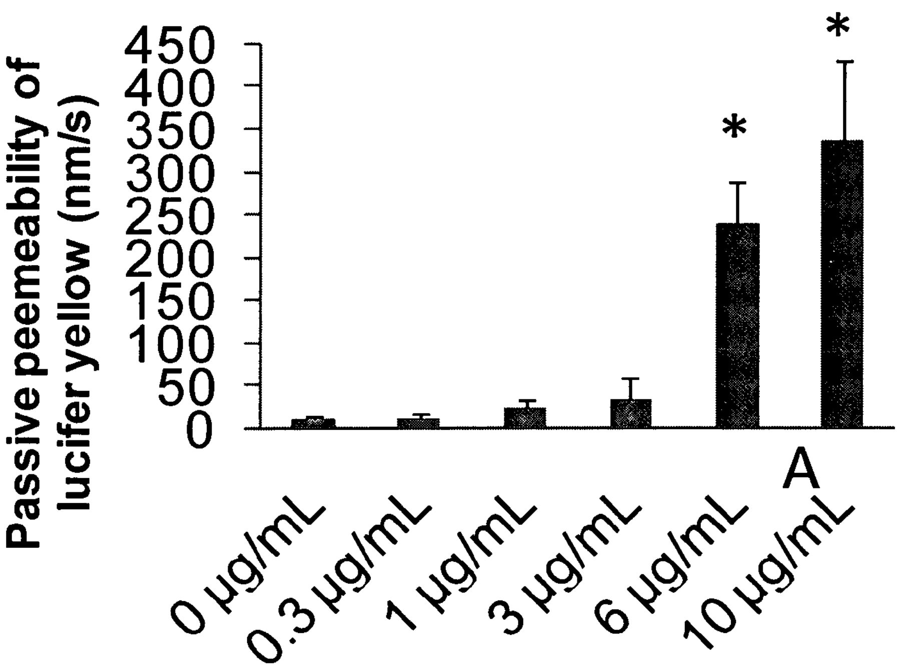
Passive peemeability of lucifer yellow (nm/s)
450
400
350
300
250
200
150
100
50
0
*
*
A
0 µg/mL
0.3 µg/mL
1 µg/mL
3 µg/mL
6 µg/mL
10 µg/mL

B

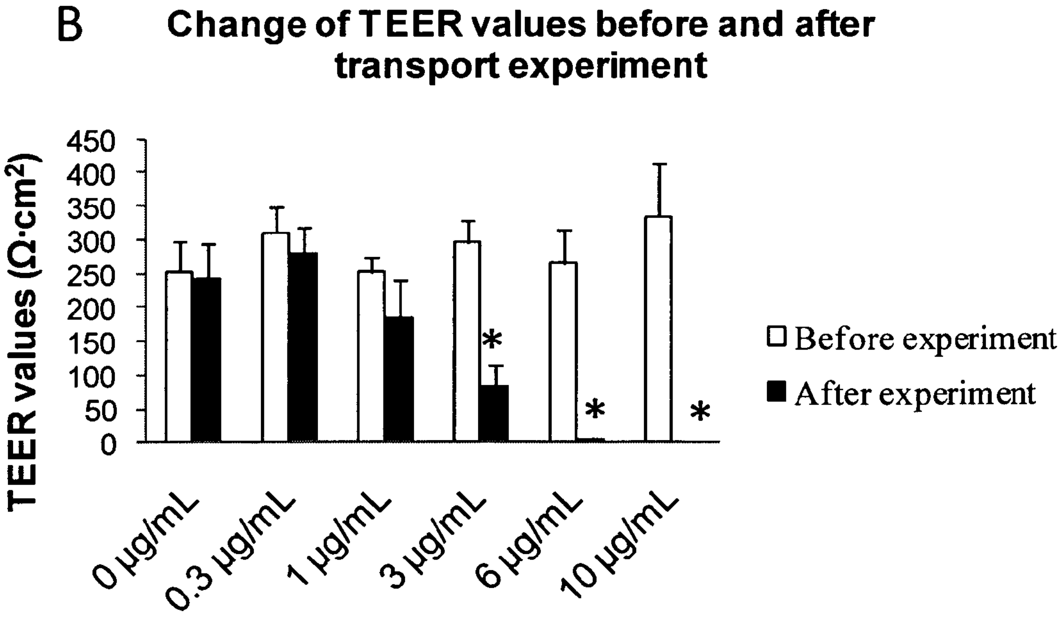
Change of TEER values before and after transport experiment
TEER values (Ω·cm²)
450
400
350
300
250
200
150
100
50
0
*
*
*
0 µg/mL
0.3 µg/mL
1 µg/mL
3 µg/mL
6 µg/mL
10 µg/mL
Before experiment
After experiment

FIG. 15

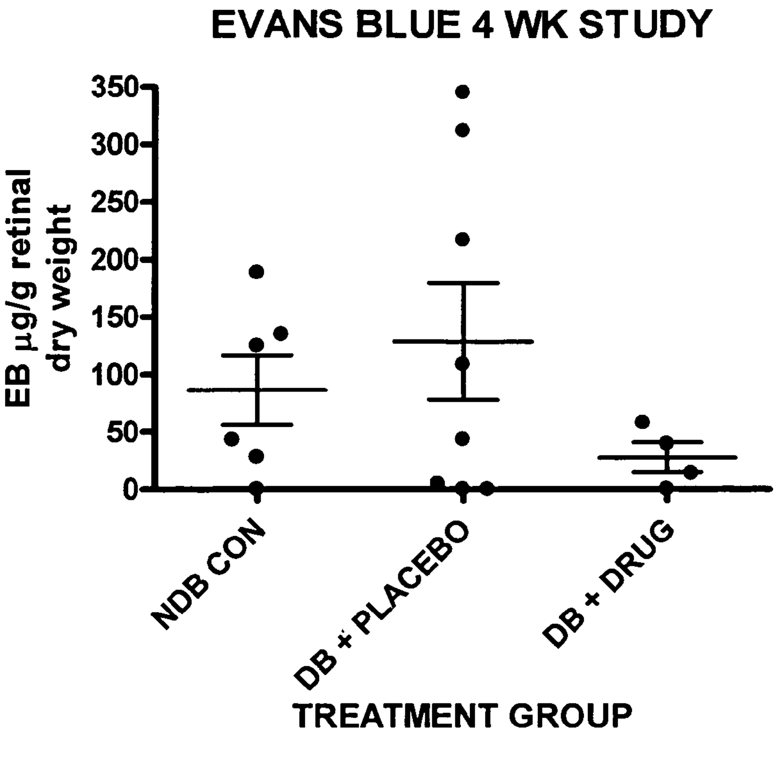
EVANS BLUE 4 WK STUDY
EB µg/g retinal dry weight
350
300
250
200
150
100
50
0
NDB CON
DB + PLACEBO
DB + DRUG
TREATMENT GROUP

FIG.16

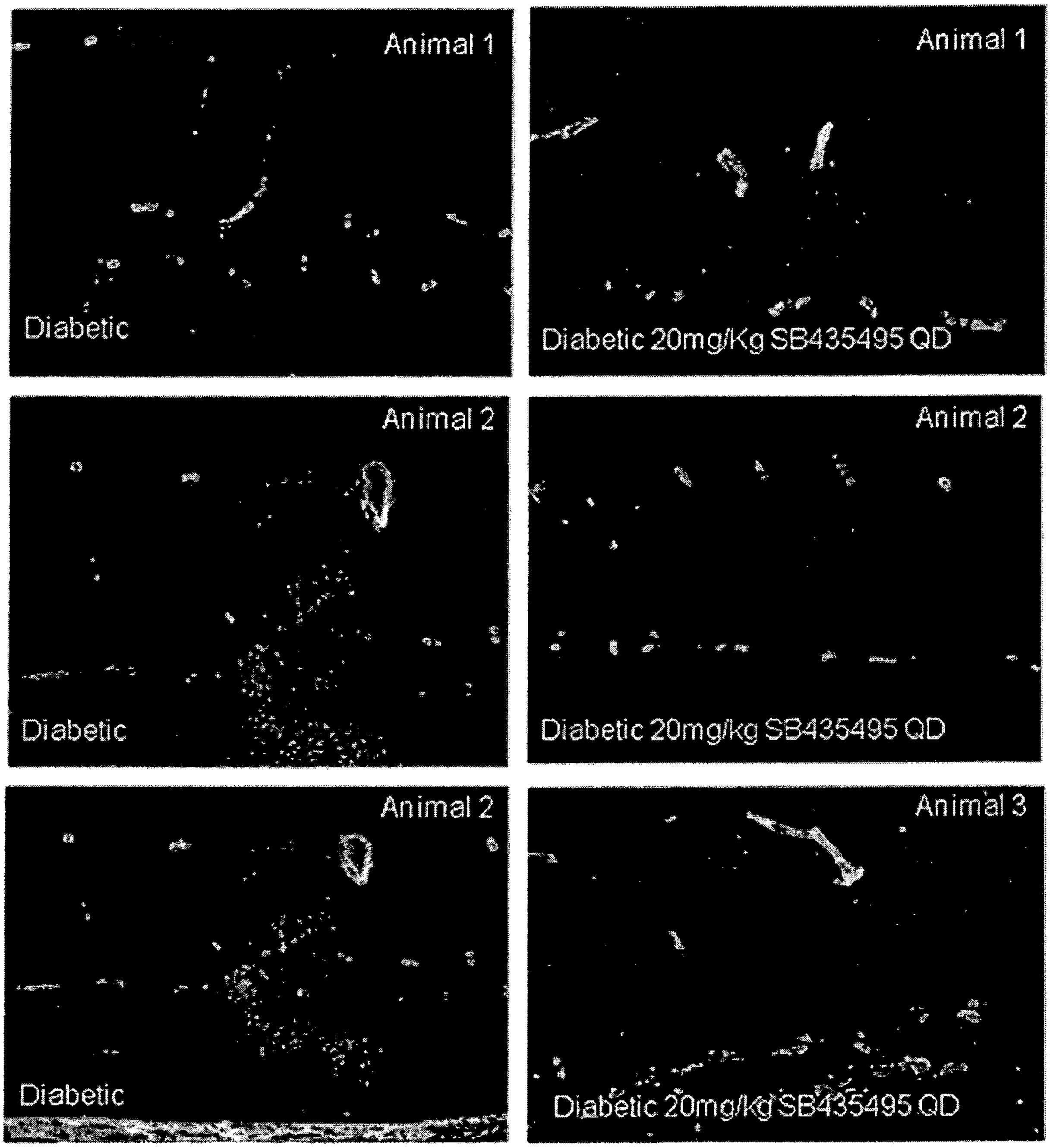
Animal 1
Diabetic
Animal 1
Diabetic 20mg/Kg SB435495 QD
Animal 2
Diabetic
Animal 2
Diabetic 20mg/kg SB435495 QD
Animal 2
Diabetic
Animal 3
Diabetic 20mg/kg SB435495 QD

FIG. 17

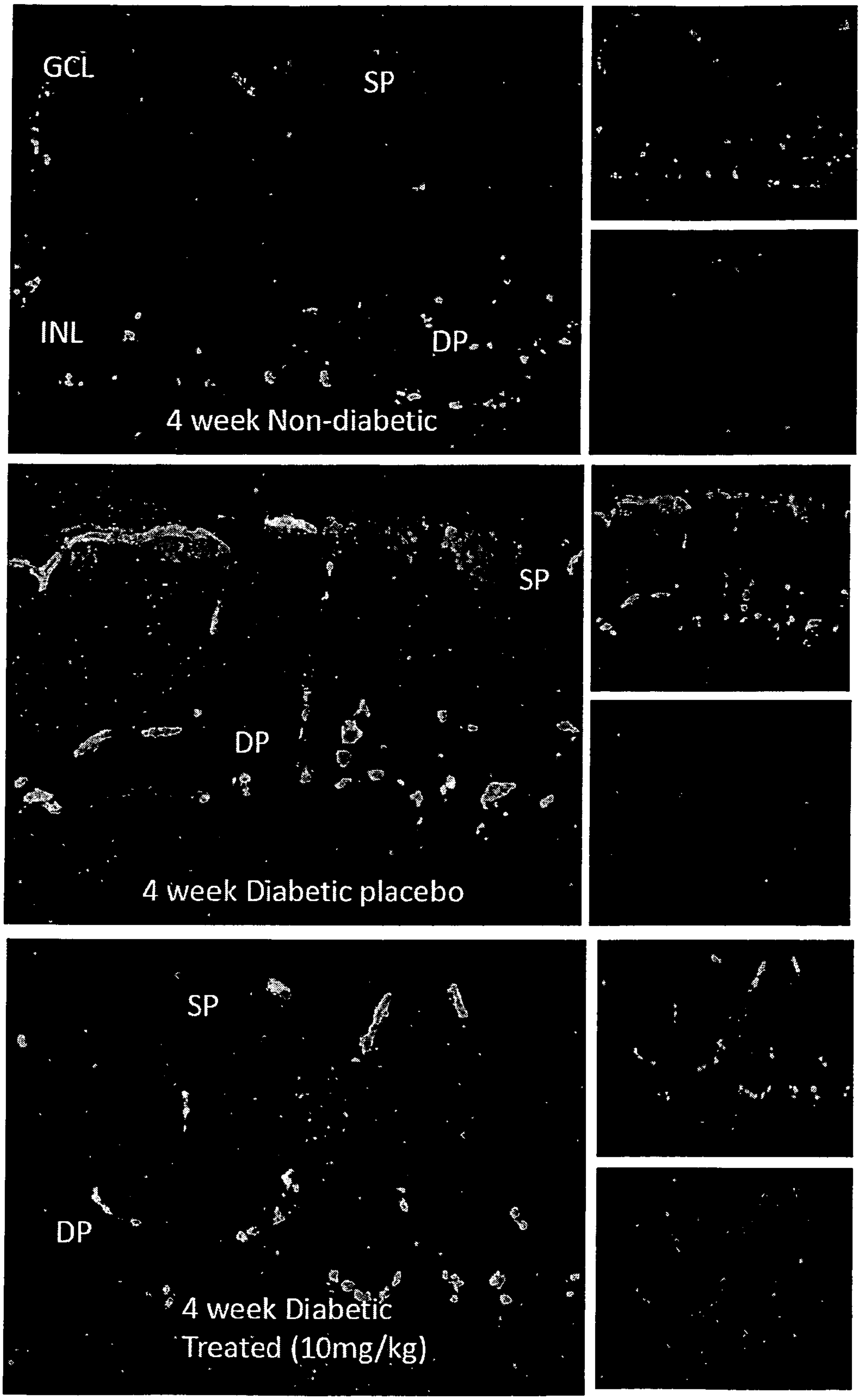
GCL
SP
INL
DP
4 week Non-diabetic
SP
DP
4 week Diabetic placebo
SP
DP
4 week Diabetic
Treated (10mg/kg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008140449 A1
- US 6649619 B
- US 7235566 B
- US 6953803 B
- US 5981252 A
- WO 9500649 A, SmithKline Beecham plc
- WO 9509921 A
- WO 2008140449 A
- US 20080279846 A
- WO 9613484 A
- WO 9619451 A
- WO 9702242 A
- WO 9712963 A
- WO 9721675 A
- WO 9721676 A
- WO 9741098 A
- WO 9741099 A, SmithKline Beecham plc
- WO 9924420 A
- WO 0010980 A
- WO 0066566 A
- WO 0066567 A
- WO 0068208 A
- WO 0160805 A
- WO 0230904 A
- WO 0230911 A
- WO 03015786 A
- WO 03016287 A
- WO 03041712 A
- WO 03042179 A
- WO 03042206 A
- WO 03042218 A
- WO 03086400 A
- WO 03087088 A
- WO 05003118 A
- WO 05021002 A
- WO 08048866 A
- WO 08140449 A
- WO 08141176 A
- WO 08048867 A
- US 20080280829 A
- US 20080103156 A
- US 20080090851 A
- US 20080090852 A
- CN 2010077154 W
- WO 2006063791 A1
- WO 2006063811 A1
- WO 2006063812 A1
- WO 2006063813 A1
- US 2006106017 A1
- US 7153861 B
- US 7169924 B
- US 20050033052 A1
- WO 0387088 A
- US 7232902 B
- US 20080090853 A
- WO 9748699 A
- US 4704295 A
- US 4556552 A
- US 4309404 A
- US 4309406 A
- WO 2005021002 A

### Non-patent literature cited in the description

- **TEW D et al.** *Arterioscler Thromb Vas Biol,* 1996, vol. 16, 591-9
- **TJOELKER et al.** *Nature,* 06 April 1995, vol. 374, 549
- **TEW et al.** *Biochemistry,* 1998, vol. 37, 10087
- **ANTONETTI DA ; BARBER AJ ; BRONSON SK ; FREEMAN WM ; GARDNER TW ; JEFFERSON LS ; KESTER M ; KIMBALL SR ; KRADY JK ; LANOUE KF.** Diabetic retinopathy: seeing beyond glucose-induced microvascular disease. *Diabetes,* 2006, vol. 55, 2401-2411
- **CURTIS TM ; GARDINER TA ; STITT AW.** Microvascular lesions of diabetic retinopathy: clues towards understanding pathogenesis?. *Eye,* 2009, vol. 23, 1496-1508
- **CHUNG EJ ; FREEMAN WR ; AZEN SP ; LEE H ; KOH HJ.** Comparison of combination posterior sub-tenon triamcinolone and modified grid laser treatment with intravitreal triamcinolone treatment in patients with diffuse diabetic macular edema. *Yonsei Medical Journal,* 2008, vol. 49, 955-964

- **HAMMES HP ; ALT A ; NIWA T ; CLAUSEN JT ; BRETZEL RG ; BROWNLEE M ; SCHLEICHER ED.** Differential accumulation of advanced glycation end products in the course of diabetic retinopathy. *Diabetologia,* 1999, vol. 42, 728-736
- **BUCALA R ; VLASSARA H.** Advanced glycosylation end products in diabetic renal and vascular disease. *Am J Kidney Dis.,* 1995, vol. 26, 875-888
- **MAKITA Z ; BUCALA R ; RAYFIELD EJ ; FRIEDMAN EA ; KAUFMAN AM ; KORBET SM ; BARTH RH ; WINSTON JA ; FUH H ; MANOGUE KR.** Reactive glycosylation endproducts in diabetic uraemia and treatment of renal failure. *Lancet,* 1994, vol. 343, 1519-1522
- **MURATA T ; NAGAI R ; ISHIBASHI T ; INOMUTA H ; IKEDA K ; HORIUCHI S.** The relationship between accumulation of advanced glycation end products and expression of vascular endothelial growth factor in human diabetic retinas. *Diabetologia,* 1997, vol. 40, 764-769
- **STITT AW.** Advanced glycation: an important pathological event in diabetic and age related ocular disease. *Br J Ophthalmol,* 2001, vol. 85, 746-753
- **STITT AW ; LI YM ; GARDINER TA ; BUCALA R ; ARCHER DB ; VLASSARA H.** Advanced glycation end products (AGEs) co-localize with AGE receptors in the retinal vasculature of diabetic and of AGE-infused rats. *Am J Pathol,* 1997, vol. 150, 523-531
- **STITT AW ; BHADURI T ; MCMULLEN CB ; GARDINER TA ; ARCHER DB.** Advanced glycation end products induce blood-retinal barrier dysfunction in normoglycemic rats. *Mol Cell Biol Res Commun,* 2000, vol. 3, 380-388
- **CHAKRAVARTHY U ; HAYES RG ; STITT AW ; MCAULEY E ; ARCHER DB.** Constitutive nitric oxide synthase expression in retinal vascular endothelial cells is suppressed by high glucose and advanced glycation end products. *Diabetes,* 1998, vol. 47, 945-95
- **GIARDINO, D. EDELSTEIN ; M. BROWNLEE.** Nonenzymatic glycosylation in vitro and in bovine endothelial cells alters basic fibroblast growth factor activity. A model for intracellular glycosylation in diabetes. *J Clin Invest,* 1994, vol. 94, 489-496
- **Y.H. ABDEL-WAHAB ; F.P. O'HARTE ; H. RATCLIFF ; N.H. MCCLENAGHAN ; C.R. BARNETT ; P.R. FLATT.** Glycation of insulin in the islets of Langerhans of normal and diabetic animals. *Diabetes,* 1996, vol. 45, 1489-1496
- **H. VLASSARA.** The AGE-receptor in the pathogenesis of diabetic complications. *Diabetes Metab Res Rev,* 2001, vol. 17, 436-443
- **A.M. SCHMIDT ; S.D. YAN ; S.F. YAN ; D.M. STERN.** The biology of the receptor for advanced glycation end products and its ligands. *Biochim Biophys Acta,* 2000, vol. 1498, 99-111
- **P. MENE ; C. PASCALE ; A. TETI ; S. BERNARDINI ; G.A. CINOTTI ; F. PUGLIESE.** Effects of advanced glycation end products on cytosolic Ca2+ signaling of cultured human mesangial cells. *J Am Soc Nephrol,* 1999, vol. 10, 1478-1486
- **V. SCIVITTARO ; M.B. GANZ ; M.F. WEISS.** AGEs induce oxidative stress and activate protein kinase C-beta(II) in neonatal mesangial cells. *Am J Physiol Renal Physiol,* 2000, vol. 278, 676-683
- **J.S. HUANG ; J.Y. GUH ; W.C. HUNG ; M.L. YANG ; Y.H. LAI ; H.C. CHEN et al.** Role of the Janus kinase (JAK)/signal transducters and activators of transcription (STAT) cascade in advanced glycation end-product-induced cellular mitogenesis in NRK-49F cells. *Biochem J,* 1999, vol. 342, 231-238
- **A.A. DEORA ; T. WIN ; B. VANHAESEBROECK ; H.M. LANDER.** A redox-triggered ras-effector interaction. Recruitment of phosphatidylinositol 3'-kinase to Ras by redox stress. *J Biol Chem,* 1998, vol. 273, 29923-29928
- **H.M. LANDER ; J.M. TAURAS ; J.S. OGISTE ; O. HORI ; R.A. MOSS ; A.M. SCHMIDT.** Activation of the receptor for advanced glycation end products triggers a p21 (ras)-dependent mitogen-activated protein kinase pathway regulated by oxidant stress. *J Biol Chem,* 1997, vol. 272, 17810-17814
- **A. BIERHAUS ; S. SCHIEKOFER ; M. SCHWANINGER ; M. ANDRASSY ; P.M. HUMPERT ; J. CHEN et al.** Diabetes-associated sustained activation of the transcription factor nuclear factor-kappaB. *Diabetes,* 2001, vol. 50, 2792-2808
- **H. SANO ; R. NAGAI ; K. MATSUMOTO ; S. HORIUCHI.** Receptors for proteins modified by advanced glycation endproducts (AGE)-their functional role in atherosclerosis. *Mech Ageing Dev,* 1999, vol. 107, 333-346
- **SCHMIDT AM ; HORI O ; BRETT J ; YAN SD ; WAUTIER JL ; STERN D.** Cellular receptors for glycation end products. Implications for induction of oxidant stress and cellular dysfunction in the pathogenesis of vascular lesions. *Arterioscler Thromb,* 1994, vol. 14, 1521-1528
- **LU M ; KUROKI M ; AMANO S ; TOLENTINO M ; KEOUGH K ; KIM I ; BUCALA R ; ADAMIS AP.** Advanced glycation end products increase retinal vascular endothelial growth factor expression. *J Clin Invest,* 1998, vol. 101, 1219-1224
- **PAGET, M. LECOMTE ; D. RUGGIERO ; N. WIERNSPERGER ; M. LAGARDE.** Modification of enzymatic antioxidants in retinal microvascular cells by glucose or advanced glycation end products. *Free Radicals Biol Med,* 1998, vol. 25, 121-129

- **GEGG M ; HARRY R. ; HANKEY D. ; ZAMBARAKJI H. ; PRYCE G. ; BAKER D ; ADAMSON P. ; CALDER V. ; GREENWOOD J.** Supression of autoimmune retinal disease by lovastatin does not require Th2 cytokine induction. *J. Immunol,* 2005, vol. 174, 2327-2235
- **LI J. ; WANG, J.J. CGEN D. ; MOTT R. ; QIANG YU. ; MA J-X. ; ZHANG S.X.** Systemic administration of HMGCoA reductase inhibitor protects the blood-retinal barrier and ameliorates retinal inflammation in type2 diabetes. *Exp. Eye Res.,* 2010, vol. 89, 71-78
- **MOHLER ER, III et al.** Site-specific atherogenic gene expression correlates with subsequent variable lesion development in coronary and peripheral vasculature. Arterioscler. *Thromb. Vasc. Biol.,* 2008, vol. 28, 850-855
- **WILENSKY RL ; SHI Y ; MOHLER ER 3RD ; HAMAMDZIC D ; BURGERT ME ; LI J ; POSTLE A ; FENNING RS ; BOLLINGER JG ; HOFFMAN BE.** Inhibition of lipoprotein-associated phospholipase A2 reduces complex coronary atherosclerotic plaque development. *Nat Med.,* 2008, vol. 14, 1059-66
- **PENNATHUR, S. ; HEINECKE, J.W.** Mechanisms for oxidative stress in diabetic cardiovascular disease. *Antioxid. Redox Signal.,* 2007, vol. 9, 955-969
- **MORENO, P.R. et al.** Coronary composition and macrophage infiltration in atherectomy specimens from patients with diabetes mellitus. *Circulation,* 2000, vol. 102, 2180-2184
- **DAVE, G.S. ; KALIA, K.** Hyperglycemia induced oxidative stress in type-1 and type-2 diabetic patients with and without nephropathy. *Cell. Mol. Biol.,* 2007, vol. 53 (5), 68-78
- **WILENSKY RL ; MACPHEE CH.** Lipoprotein-associated phospholipase A(2) and atherosclerosis. *Curr Opin Lipidol.,* 2009, vol. 20, 415-20
- **S. LAVI ; J.P. MCCONNELL ; C.S. RIHAL ; A. PRASAD ; V. MATHEW ; L.O. LERMAN ; A. LERMAN.** Local production of lipoprotein-associated phospholipase A2 and lysophosphatidylcholine in the coronary circulation: association with early coronary atherosclerosis and endothelial dysfunction in humans. *Circulation,* 2007, vol. 115, 2715-2721
- **CLIFFORD PM ; ZARRABI S ; SIU G ; KINSLER KJ ; KOSCIUK MC ; VENKATARAMAN V ; D'ANDREA MR ; DINSMORE S ; NAGELE RG.** Abeta peptides can enter the brain through a defective blood-brain barrier and bind selectively to neurons. *Brain Res.,* 2007, vol. 1142, 223-36
- **LENZSÉR G ; KIS B ; BARI F ; BUSIJA DW.** Diazoxide preconditioning attenuates global cerebral ischemia-induced blood-brain barrier permeability. *Brain Res.,* 2005, vol. 1051, 72-80
- **JONES JM ; DOWLING TC ; PARK JJ ; PHARES DA ; PARK JY ; OBISESAN TO ; BROWN MD.** Differential aerobic exercise-induced changes in plasma aldosterone between African Americans and Caucasians. *Exp Physiol.,* 2007, vol. 92, 871-879
- **MORREY JD ; OLSEN AL ; SIDDHARTHAN V ; MOTTER NE ; WANG H ; TARO BS ; CHEN D ; RUFFNER D ; HALL JO.** increased blood-brain barrier permeability is not a primary determinant for lethality of West Nile virus infection in rodents. *J Gen Virol.,* 2008, vol. 89, 467-473
- **XU Q. ; QAUM T ; ADAMSIS A.P.** Sensitive Blood-retinal barrier breakdown quantitation using Evans Blue. *Invest. Ophthalmol. Vis. Sci.,* 2001, vol. 42, 789-794
- **BURSELL SE ; CLERMONT AC ; KINSLEY BT et al.** Retinal blood flow changes in patients with insulin-dependent diabetes mellitus and no diabetic retinopathy. *Invest Ophthalmol Vis Sci.,* 1996, vol. 37, 886-897
- **AHLERS C ; GOLBAZ I ; EINWALLNER E ; DUNAVÖLGYI R ; MALAMOS P ; STOCK G ; PRUENTE C ; SCHMIDT-ERFURTH U.** Identification of optical density ratios in subretinal fluid as a clinically relevant biomarker in exudative macular disease. *Invest Ophthalmol Vis Sci.,* 2009, vol. 50, 3417-3424
- **YAN S ; CHAI H ; WANG H ; YANG H ; NAN B ; YAO Q ; CHEN C.** Effects of lysophosphatidylcholine on monolayer cell permeability of human coronary artery endothelial cells. *Surgery,* 2005, vol. 138, 464-73
- **TAN M ; HAO F ; XU X ; CHISOLM GM ; CUI MZ.** Lysophosphatidylcholine activates a novel PKD2-mediated signaling pathway that controls monocyte migration. *Arterioscler Thromb Vasc Biol.,* 2009, vol. 29, 1376-82
- **SARKER MH ; HU DE ; FRASER PA.** Acute effects of bradykinin on cerebral microvascular permeability in the anaesthetized rat. *J Physiol.,* 2000, vol. 528, 177-87
- **UMEZU-GOTO M ; KISHI Y ; TAIRA A ; HAMA K ; DOHMAE N ; TAKIO K ; YAMORI T ; MILLS GB ; INOUE K ; AOKI J.** Autotaxin has lysophospholipase D activity leading to tumor cell growth and motility by lysophosphatidic acid production. *J Cell Biol.,* 2002, vol. 158, 227-233